# EUROPEAN PATENT APPLICATION

(11) **EP 4 001 292 A1**
(43) Date of publication of application: **25.05.2022**
(21) Application number: 20840891.4
(22) Date of filing: 17.07.2020
(51) Int. Cl.: C07K 5/083, C07K 19/00

(54) **SEPARATED PEPTIDE**

(30) Priority: 18.07.2019 JP 2019132490
(71) Applicant: National University Corporation Tokai National Higher Education and Research System, Nagoya-shi, Aichi 464-8601 (JP)
(72) Inventor: HORI, Katsutoshi, Nagoya-shi, Aichi 464-8601 (JP)
(74) Representative: Engelhard, Markus
(86) International application number: PCT/JP2020/027922
(87) International publication number: WO 2021/010482

(57) **Abstract**

The present invention provides a peptide that disrupts the self-aggregation of an AtaA polypeptide or separates a bond between the AtaA polypeptide and another subject. A peptide found by the present inventors has the properties of disrupting the self-aggregation of an AtaA polypeptide or separating a bond between the AtaA polypeptide and another subject. A peptide according to the present disclosure includes amino acid sequences AVL, SVL, ATL, or functional equivalent sequences thereof. In one embodiment, a peptide according to the present disclosure has the ability to separate a bond between an AtaA and other molecules (for example, streptavidin and neutral avidin).

SEQUENCE LISTING

<110>  National University Corporation Tokai National Higher
       Education and Research System

<120>  Separation Peptide

<130>  NU006PCT

<150>  JP 2019-132490
<151>  2019-07-18

<160>  6

<170>  PatentIn version 3.5

<210>  1
<211>  3518
<212>  PRT
<213>  artificial sequence

<220>
<223>  Fragment of AtaA

<400>  1

Met Ala Thr Val Gln Val Gly Gly Gly Thr Asn Ser Gly Thr Thr Ala
1               5                   10                  15

Thr Ala Ser Thr Asn Cys Ala Asp Leu Tyr Asn Tyr Gln Asn Pro Glu
            20                  25                  30

Asn Ser Gly Ser Gly Ala Ala Gly Asn Tyr Asn Ala Gly Asn Pro Ser
        35                  40                  45

Val Cys Ser Ile Ala Ile Gly Glu Asn Ala Gln Gly Gly Thr Ser Gly
    50                  55                  60

Thr Gly Gly Ser Pro Gly Ile Ala Ile Gly Gly Asn Ser Lys Ala Thr
65                  70                  75                  80

Gly Gly Leu Ser Val Ala Ile Gly Gly Tyr Ala Gln Ala Thr Asn Val
                85                  90                  95

Gly Ser Ile Ala Leu Gly Thr Ala Ala Leu Ser Ser Gly Phe Asn Ser
            100                 105                 110

Leu Ala Ile Ser Arg Gln Ala Ala Ala Thr Asn Asn Tyr Ser Ile Ala
        115                 120                 125

Ile Gly Thr Thr Ser Val Ser Lys Gly Val Gly Ser Ile Ala Met Gly
    130                 135                 140

His Ser Thr Asn Ala Ser Gly Asp Gln Ser Ile Ala Ile Gly Ser Ser

145                 150                 155                 160

Asp Ala Val Asn Ser Ala Thr Ala Thr Thr Thr Tyr Asp Gly Thr Thr
                165                 170                 175

Asn Thr Gln Ala Ser Gly Ser Lys Ser Ile Ala Ile Gly Ala Ser Ala
            180                 185                 190

Lys Ala Ser Thr Asn Asn Ser Ile Ala Leu Gly Ala Gly Ser Val Thr
        195                 200                 205

Ser Ala Gln Ser Gly Asn Ser Tyr Leu Thr Gly Val Gly Ala Ser Ala
    210                 215                 220

Thr Asn Gly Val Val Ser Val Gly Thr Ser Thr Ala Thr Arg Arg Ile
225                 230                 235                 240

Gln Asn Val Ala Asp Gly Ser Ala Ala Ser Asp Ala Val Thr Val Ala
                245                 250                 255

Gln Leu Asp Lys Ala Tyr Asp Asp Thr Asn Gly Arg Leu Ala Ala Ala
            260                 265                 270

Leu Gly Thr Gly Ser Gly Ala Ala Tyr Asn Ala Ala Asn Asn Thr Tyr
        275                 280                 285

Thr Ala Pro Thr Asn Ile Gly Gly Thr Gly Lys Asn Thr Ile Asp Asp
    290                 295                 300

Ala Ile Lys Ala Thr Gln Arg Ser Val Val Ala Gly Ser Asn Ile Val
305                 310                 315                 320

Val Thr Pro Thr Thr Ala Ser Asp Gly Ser Ile Ser Tyr Ser Val Ala
                325                 330                 335

Thr Ser Ala Thr Pro Thr Phe Thr Ser Ile Thr Val Asn Asn Ala Pro
            340                 345                 350

Thr Ala Gly Thr Asp Ala Thr Asn Lys Thr Tyr Val Asp Ser Lys Ala
        355                 360                 365

Ala Ala Ser Arg Thr Glu Val Ala Ala Gly Ser Asn Val Ser Gly Val
    370                 375                 380

Val Lys Thr Thr Gly Ala Asn Gly Gln Asp Val Tyr Thr Val Asn Ala
385                 390                 395                 400

Asn Gly Thr Thr Ala Ser Ala Gly Ser Ser Ala Val Thr Val Thr Pro
                405                 410                 415

Gly Thr Lys Asp Ala Asn Asn Val Thr Asp Tyr Lys Val Asp Leu Ser
            420                 425                 430

Ala Thr Thr Lys Thr Asp Ile Gln Lys Gly Val Asp Ala Lys Asn Ala
        435                 440                 445

Val Asp Thr Ala Gly Leu Lys Phe Lys Gly Asp Thr Ala Thr Thr Ser
    450                 455                 460

Asn Thr Lys Lys Leu Gly Asp Thr Val Ser Ile Thr Gly Asp Thr Asn
465                 470                 475                 480

Ile Ser Thr Val Ala Thr Thr Asp Gly Val Gln Val Lys Leu Asn Pro
                485                 490                 495

Asn Leu Asp Leu Gly Ala Thr Gly Ser Val Lys Thr Gly Asn Thr Thr
            500                 505                 510

Ile Asn Asn Ala Gly Val Thr Ala Asp Gln Val Thr Val Gly Gly Val
        515                 520                 525

Val Ile Asn Asn Thr Ser Gly Ile Asn Ala Gly Gly Lys Ala Ile Thr
    530                 535                 540

Asn Val Ala Ala Pro Thr Asn Asn Thr Asp Ala Ala Asn Lys Lys Tyr
545                 550                 555                 560

Val Asp Asp Ala Gly Thr Ala Leu Thr Asn Leu Gly Phe Gly Leu Lys
                565                 570                 575

Ala Gln Asp Gly Thr Thr Val Asn Lys Lys Leu Gly Glu Ala Val Asp
            580                 585                 590

Ile Val Gly Ser Asn Ser Asn Ile Ser Thr Lys Val Asn Ala Gly Lys
        595                 600                 605

Val Glu Val Ala Leu Ser Asn Thr Leu Asp Leu Gly Thr Thr Gly Ser
    610                 615                 620

Val Thr Thr Gly Ser Thr Val Ile Asn Asn Ala Gly Val Thr Ala Thr
625                 630                 635                 640

Gln Val Thr Ala Asn Lys Val Thr Ile Asn Asn Ala Pro Thr Ala Gly
                645                 650                 655

Thr Asp Ala Thr Asn Lys Thr Tyr Val Asp Ser Lys Ala Ala Ala Ser
        660                 665                 670

Arg Thr Glu Val Ala Ala Gly Ser Asn Val Ser Gly Val Val Lys Thr
    675                 680                 685

Thr Gly Ala Asn Gly Gln Asp Ile Tyr Ala Val Asn Ala Asn Gly Thr
690                 695                 700

Thr Ala Ser Ala Gly Ser Ser Ala Val Thr Val Thr Pro Gly Thr Lys
705                 710                 715                 720

Asp Ala Asn Asn Val Thr Asp Tyr Lys Val Asp Leu Ser Ala Thr Thr
                725                 730                 735

Lys Thr Asp Ile Gln Lys Gly Val Asp Ala Lys Asn Ala Val Asp Thr
            740                 745                 750

Ala Gly Leu Lys Phe Lys Gly Asp Thr Ala Thr Thr Ser Asn Thr Lys
        755                 760                 765

Lys Leu Gly Asp Thr Val Ser Ile Thr Gly Asp Thr Asn Ile Ser Thr
    770                 775                 780

Val Ala Thr Thr Asp Gly Val Gln Val Lys Leu Asn Pro Asn Leu Asp
785                 790                 795                 800

Leu Gly Ala Thr Gly Ser Val Lys Thr Gly Asn Thr Thr Ile Asn Asn
                805                 810                 815

Ala Gly Val Thr Ala Asp Gln Val Thr Val Gly Gly Val Val Ile Asn
            820                 825                 830

Asn Thr Ser Gly Ile Asn Ala Gly Gly Lys Ala Ile Thr Asn Val Ala
        835                 840                 845

Ala Pro Thr Asn Asn Thr Asp Ala Ala Asn Lys Lys Tyr Val Asp Asp
    850                 855                 860

Ala Gly Thr Ala Leu Thr Asn Leu Gly Phe Gly Leu Lys Ala Gln Asp
865                 870                 875                 880

Gly Thr Thr Val Asn Lys Lys Leu Gly Glu Ala Val Asp Ile Val Gly
                885                 890                 895

Ser Asn Ser Asn Ile Ser Thr Lys Val Asn Ala Gly Lys Val Glu Val
            900                 905                 910

Ala Leu Ser Asn Thr Leu Asp Leu Gly Thr Thr Gly Ser Val Thr Thr
        915                 920                 925

Gly Ser Thr Val Ile Asn Asn Ala Gly Val Thr Ala Thr Gln Val Thr
    930                 935                 940

Ala Asn Lys Val Thr Val Asn Asn Ala Pro Thr Ala Gly Thr Asp Ala
945                 950                 955                 960

Thr Asn Lys Thr Tyr Val Asp Ser Lys Ala Ala Ala Ser Arg Thr Glu
                965                 970                 975

Val Ala Ala Gly Ser Asn Val Ser Gly Val Val Lys Thr Thr Gly Ala
            980                 985                 990

Asn Gly Gln Asp Val Tyr Thr Val Asn Ala Asn Gly Thr Thr Ala Ser
        995                 1000                1005

Ala Gly Ser Ser Ala Val Thr Val Thr Pro Gly Thr Lys Asp Ala
    1010                1015                1020

Asn Asn Val Thr Asp Tyr Lys Val Asp Leu Ser Ala Thr Thr Lys
    1025                1030                1035

Thr Asp Ile Gln Lys Gly Val Asp Ala Lys Asn Ala Val Asp Thr
    1040                1045                1050

Ala Gly Leu Lys Phe Lys Gly Asp Thr Ala Thr Thr Ser Asn Thr
    1055                1060                1065

Lys Lys Leu Gly Asp Thr Val Ser Ile Thr Gly Asp Thr Asn Ile
    1070                1075                1080

Ser Thr Val Ala Thr Thr Asp Gly Val Gln Val Lys Leu Asn Pro
    1085                1090                1095

Asn Leu Asp Leu Gly Ala Thr Gly Ser Val Lys Thr Gly Asn Thr
    1100                1105                1110

Thr Ile Asn Asn Ala Gly Val Thr Ala Asp Gln Val Thr Val Gly
    1115                1120                1125

Gly Val Val Ile Asn Asn Thr Ser Gly Ile Asn Ala Gly Gly Lys
    1130                1135                1140

Ala Ile Thr Asn Val Ala Ala Pro Thr Asn Asn Thr Asp Ala Ala

1145                1150                1155

Asn Lys Lys Tyr Val Asp Asp Ala Gly Thr Ala Leu Thr Asn Leu
    1160                1165                1170

Gly Phe Gly Leu Lys Ala Gln Asp Gly Thr Thr Val Asn Lys Lys
    1175                1180                1185

Leu Gly Glu Ala Val Glu Val Val Gly Ala Asp Ser Asn Ile Thr
    1190                1195                1200

Thr Lys Val Ala Gly Gly Gln Val Ala Ile Glu Leu Asn Lys Asn
    1205                1210                1215

Leu Asn Asn Leu Thr Gly Ile Thr Val Asn Asp Gly Thr Asn Gly
    1220                1225                1230

Thr Asn Gly Ser Thr Val Ile Gly Lys Asp Gly Ile Ser Val Lys
    1235                1240                1245

Asp Gly Ser Gly Asn Thr Ile Ala Gly Val Asp Asn Thr Ala Leu
    1250                1255                1260

Thr Val Lys Asp Gly Ser Gly Asn Thr Glu Thr Ser Ile Asn Gln
    1265                1270                1275

Ala Ile Asn Thr Leu Asn Ala Ala Gln Gly Glu Thr Asp Lys Phe
    1280                1285                1290

Ala Val Lys Tyr Asp Lys Asn Ala Asp Gly Ser Val Asn Tyr Asn
    1295                1300                1305

Asn Ile Thr Leu Ala Gly Thr Thr Ala Ser Ser Thr Gln Asp Ala
    1310                1315                1320

Thr Thr Gly Lys Ile Thr Thr Thr Gly Gly Thr Ser Leu Asn Asn
    1325                1330                1335

Val Ala Ser Ala Gly Asp Tyr Lys Asp Val Ala Asn Ala Ser Lys
    1340                1345                1350

Gly Val Asn Ala Gly Asp Leu Asn Asn Ala Val Val Asp Ala Thr
    1355                1360                1365

Asn Ala Ala Thr Ser Lys Gly Phe Ala Leu Gln Ala Ala Asp Gly
    1370                1375                1380

Ala Lys Val Gln Lys Asn Leu Gly Glu Ala Val Glu Val Val Gly
    1385                1390                1395

Ala Asp Ser Asn Ile Thr Thr Lys Val Ala Gly Gly Gln Val Ala
    1400                1405                1410

Ile Glu Leu Asn Lys Asn Leu Asn Asn Leu Thr Gly Ile Thr Val
    1415                1420                1425

Asn Asp Gly Thr Asn Gly Thr Asn Gly Ser Thr Val Ile Gly Lys
    1430                1435                1440

Asp Gly Ile Ser Val Lys Asp Gly Ser Gly Asn Thr Ile Ala Gly
    1445                1450                1455

Val Asp Asn Thr Ala Leu Thr Val Lys Asp Gly Ser Gly Asn Thr
    1460                1465                1470

Glu Thr Ser Ile Asn Gln Ala Ile Asn Thr Leu Asn Ala Ala Gln
    1475                1480                1485

Gly Glu Thr Asp Lys Phe Ala Val Lys Tyr Asp Lys Asn Thr Asp
    1490                1495                1500

Gly Ser Thr Asn Tyr Asn Ser Ile Thr Ala Gly Asn Gly Asn Gly
    1505                1510                1515

Thr Ala Ala Thr Ile Gly Thr Asp Thr Ala Gly Asn Ser Val Val
    1520                1525                1530

Thr Ser Gly Gly Thr Lys Ile Ser Asn Val Ala Asn Gly Val Asn
    1535                1540                1545

Ala Ser Asp Ala Val Asn Lys Gly Gln Leu Asp Ser Leu Ser Thr
    1550                1555                1560

Gly Leu Thr Asn Thr Gly Phe Gly Leu Lys Ala Ala Asp Gly Asn
    1565                1570                1575

Thr Val Asn Lys Lys Leu Gly Glu Ala Val Asp Val Val Gly Ala
    1580                1585                1590

Asp Ser Asn Ile Thr Thr Lys Val Ala Gly Gly Gln Val Ala Ile
    1595                1600                1605

Glu Leu Asn Lys Asn Leu Asn Asn Leu Thr Gly Ile Thr Val Asn
    1610                1615                1620

Asp Gly Thr Asn Gly Thr Asn Gly Ser Thr Val Ile Gly Lys Asp
    1625                1630                1635

Gly Ile Ser Ile Lys Asp Gly Ser Gly Asn Thr Ile Ala Gly Val
    1640                1645                1650

Asp Asn Thr Ala Leu Thr Val Lys Asp Gly Ser Gly Asn Thr Glu
    1655                1660                1665

Thr Ser Ile Asn Gln Ala Ile Asn Thr Leu Asn Ala Ala Gln Gly
    1670                1675                1680

Glu Thr Asp Lys Phe Ala Val Lys Tyr Asp Lys Asn Ala Asp Gly
    1685                1690                1695

Ser Ala Asn Tyr Asn Asn Ile Thr Leu Ala Gly Thr Thr Ala Ser
    1700                1705                1710

Ser Thr Gln Asp Ala Thr Thr Gly Lys Ile Thr Thr Thr Gly Gly
    1715                1720                1725

Thr Ser Leu Asn Asn Val Ala Ser Ala Gly Asp Tyr Lys Asp Val
    1730                1735                1740

Ala Asn Ala Ser Lys Gly Val Asn Ala Gly Asp Leu Asn Asn Ala
    1745                1750                1755

Val Val Asp Ala Thr Asn Ala Ala Thr Ser Lys Gly Phe Ala Leu
    1760                1765                1770

Gln Ala Ala Asp Gly Ala Lys Val Gln Lys Asn Leu Gly Glu Ala
    1775                1780                1785

Val Glu Val Val Gly Ala Asp Ser Asn Ile Thr Thr Lys Val Val
    1790                1795                1800

Gly Gly Gln Val Ala Ile Glu Leu Asn Lys Asn Leu Asn Asn Leu
    1805                1810                1815

Thr Gly Ile Thr Val Asn Asp Gly Thr Asn Gly Thr Asn Gly Ser
    1820                1825                1830

Thr Val Ile Gly Lys Asp Gly Ile Ser Val Lys Asp Gly Ser Gly
    1835                1840                1845

Asn Thr Ile Ala Gly Val Asp Asn Thr Ala Leu Thr Val Lys Asp
    1850                1855                1860

Gly Ser Gly Asn Thr Glu Thr Ser Ile Asn Gln Ala Ile Asn Thr
    1865                1870                1875

Leu Asn Ala Ala Gln Gly Glu Thr Asp Lys Phe Ala Val Lys Tyr
    1880                1885                1890

Asp Lys Asn Ala Asp Gly Ser Val Asn Tyr Asn Asn Ile Thr Leu
    1895                1900                1905

Ala Gly Thr Thr Ala Ser Ser Thr Gln Asp Ala Thr Thr Gly Lys
    1910                1915                1920

Ile Thr Thr Thr Gly Gly Thr Ser Leu Asn Asn Val Ala Ser Ala
    1925                1930                1935

Gly Asp Tyr Lys Asp Val Ala Asn Ala Ser Lys Gly Val Asn Ala
    1940                1945                1950

Gly Asp Leu Asn Asn Ala Val Val Asp Ala Thr Asn Ala Ala Thr
    1955                1960                1965

Ser Lys Gly Phe Ala Leu Gln Ala Ala Asp Gly Ala Lys Val Gln
    1970                1975                1980

Lys Asn Leu Gly Glu Ala Val Glu Val Val Gly Ala Asp Ser Asn
    1985                1990                1995

Ile Thr Thr Lys Val Ala Gly Gly Gln Val Ala Ile Glu Leu Asn
    2000                2005                2010

Lys Asn Leu Asn Asn Leu Thr Gly Ile Thr Val Asn Asp Gly Thr
    2015                2020                2025

Asn Gly Thr Asn Gly Ser Thr Val Ile Gly Lys Asp Gly Ile Ser
    2030                2035                2040

Val Lys Asp Gly Ser Gly Asn Thr Ile Ala Gly Val Asp Asn Thr
    2045                2050                2055

Ala Leu Thr Val Lys Asp Gly Ser Gly Asn Thr Glu Thr Ser Ile
    2060                2065                2070

Asn Gln Ala Ile Asn Thr Leu Asn Ala Ala Gln Gly Glu Thr Asp
    2075                2080                2085

Lys Phe Ala Val Lys Tyr Asp Lys Asn Ala Asp Gly Ser Val Asn

2090                2095                2100

Tyr Asn Asn Ile Thr Leu Ala Gly Thr Thr Ala Ser Ser Thr Gln
    2105                2110                2115

Asp Ala Thr Thr Gly Lys Ile Thr Thr Thr Gly Gly Thr Ser Leu
    2120                2125                2130

Asn Asn Val Ala Ser Ala Gly Asp Tyr Lys Asp Val Ala Asn Ala
    2135                2140                2145

Ser Lys Gly Val Asn Ala Gly Asp Leu Asn Asn Ala Val Val Asp
    2150                2155                2160

Ala Thr Asn Ala Ala Thr Ser Lys Gly Phe Ala Leu Gln Ala Ala
    2165                2170                2175

Asp Gly Ala Lys Val Gln Lys Asn Leu Gly Glu Ala Val Glu Val
    2180                2185                2190

Val Gly Ala Asp Ser Asn Ile Thr Thr Lys Val Ala Gly Gly Gln
    2195                2200                2205

Val Ala Ile Glu Leu Asn Lys Asn Leu Asn Asn Leu Thr Gly Ile
    2210                2215                2220

Thr Val Asn Asp Gly Thr Asn Gly Thr Asn Gly Ser Thr Val Ile
    2225                2230                2235

Gly Lys Asp Gly Ile Ser Val Lys Asp Gly Ser Gly Asn Thr Ile
    2240                2245                2250

Ala Gly Val Asp Asn Thr Ala Leu Thr Val Lys Asp Gly Ser Gly
    2255                2260                2265

Asn Thr Glu Thr Ser Ile Asn Gln Ala Ile Asn Thr Leu Asn Ala
    2270                2275                2280

Ala Gln Gly Glu Thr Asp Lys Phe Ala Val Lys Tyr Asp Lys Asn
    2285                2290                2295

Ala Asp Gly Ser Ala Asn Tyr Asn Asn Val Thr Leu Ala Gly Thr
    2300                2305                2310

Asn Gly Thr Ile Ile Ser Asn Val Lys Ala Gly Ala Val Thr Ser
    2315                2320                2325

Thr Ser Thr Asp Ala Ile Asn Gly Ser Gln Leu Tyr Gly Val Ala
    2330                2335                2340

Asn Ser Val Lys Asn Ala Ile Gly Gly Ser Thr Thr Ile Asp Ala
    2345                2350                2355

Thr Thr Gly Ala Ile Thr Thr Thr Asn Ile Gly Gly Thr Gly Ser
    2360                2365                2370

Asn Thr Ile Asp Gly Ala Ile Ser Ser Ile Lys Asp Ser Ala Thr
    2375                2380                2385

Lys Ala Lys Thr Thr Val Ser Ala Gly Asp Asn Val Val Val Thr
    2390                2395                2400

Ser Gly Thr Asn Ala Asp Gly Ser Thr Asn Tyr Glu Val Ala Thr
    2405                2410                2415

Ala Lys Asp Val Asn Phe Asp Lys Val Thr Val Gly Ser Val Val
    2420                2425                2430

Val Asp Lys Ser Ser Asn Thr Ile Lys Gly Leu Ser Asn Thr Thr
    2435                2440                2445

Trp Asn Gly Thr Ala Val Ser Gly Gln Ala Ala Thr Glu Asp Gln
    2450                2455                2460

Leu Lys Thr Val Ser Asp Ala Gln Gly Glu Thr Asp Lys Phe Ala
    2465                2470                2475

Val Lys Tyr Asp Lys Asn Ala Asp Gly Ser Ala Asn Tyr Asn Ser
    2480                2485                2490

Ile Thr Ala Gly Asn Gly Asn Gly Thr Ala Ala Thr Ile Gly Thr
    2495                2500                2505

Asp Thr Ala Gly Asn Ser Val Val Thr Ser Gly Gly Thr Lys Ile
    2510                2515                2520

Ser Asn Val Ala Asn Gly Val Asn Ala Ser Asp Ala Val Asn Lys
    2525                2530                2535

Gly Gln Leu Asp Ser Leu Ser Thr Gly Leu Thr Asn Thr Gly Phe
    2540                2545                2550

Gly Leu Lys Ala Ala Asp Gly Asn Thr Val Asn Lys Lys Leu Gly
    2555                2560                2565

Glu Ala Val Asp Val Val Gly Ala Asp Ser Asn Ile Thr Thr Lys
    2570                2575                2580

Val Ala Gly Gly Gln Val Ala Ile Glu Leu Asn Lys Asn Leu Asn
    2585                2590                2595

Asn Leu Thr Gly Ile Thr Val Asn Asp Gly Thr Asn Gly Thr Asn
    2600                2605                2610

Gly Ser Thr Val Ile Gly Lys Asp Gly Ile Ser Ile Lys Asp Gly
    2615                2620                2625

Ser Gly Asn Thr Ile Ala Gly Val Asp Asn Thr Ala Leu Thr Val
    2630                2635                2640

Lys Asp Ser Ser Gly Asn Thr Glu Thr Ser Ile Asn Gln Ala Ile
    2645                2650                2655

Asn Thr Leu Asn Ala Ala Gln Gly Glu Thr Asp Lys Phe Ala Val
    2660                2665                2670

Lys Tyr Asp Lys Asn Ala Asp Gly Ser Val Asn Tyr Asn Asn Val
    2675                2680                2685

Thr Leu Ala Gly Thr Asn Gly Thr Ile Ile Arg Asn Val Lys Ala
    2690                2695                2700

Gly Ala Val Thr Ser Thr Ser Thr Asp Ala Ile Asn Gly Ser Gln
    2705                2710                2715

Leu Tyr Asp Ile Ala Asn Ser Val Lys Asn Ala Ile Gly Gly Ser
    2720                2725                2730

Thr Thr Arg Asp Val Thr Thr Gly Ala Ile Thr Thr Thr Asn Ile
    2735                2740                2745

Gly Gly Thr Gly Ser Asn Thr Ile Asp Gly Ala Ile Ser Ser Ile
    2750                2755                2760

Lys Asp Ser Ala Thr Lys Ala Lys Thr Thr Ile Ser Ala Gly Asp
    2765                2770                2775

Asn Val Val Val Thr Ser Gly Thr Asn Ala Asp Gly Ser Thr Asn
    2780                2785                2790

Tyr Glu Val Ala Thr Ala Lys Asp Val Asn Phe Asp Lys Val Thr
    2795                2800                2805

Val Gly Asn Val Val Val Asp Lys Ala Asn Asp Thr Ile Gln Gly
    2810                2815                2820

Leu Ser Asn Lys Asp Leu Asn Ser Thr Asp Phe Ala Thr Lys Gly
    2825                2830                2835

Arg Ala Ala Thr Glu Glu Gln Leu Lys Ala Val Ile Thr Ser Asn
    2840                2845                2850

Ile Thr Glu Val Val Asp Gly Asn Gly Asn Lys Val Asn Ile Ile
    2855                2860                2865

Asp Gln Val Val Asn Thr Lys Pro Asp Asn Lys Asn Gln Asp Ser
    2870                2875                2880

Leu Phe Leu Thr Tyr Asp Lys Gln Gly Gln Glu Thr Thr Asp Arg
    2885                2890                2895

Leu Thr Ile Gly Gln Thr Val Gln Lys Met Asn Thr Asp Gly Ile
    2900                2905                2910

Lys Phe Phe His Thr Asn Ala Asp Thr Ser Lys Gly Asp Leu Gly
    2915                2920                2925

Thr Thr Asn Asp Ser Ser Ala Gly Gly Leu Asn Ser Thr Ala Ile
    2930                2935                2940

Gly Val Asn Ala Ile Val Ala Asn Gly Ala Asp Ser Ser Val Ala
    2945                2950                2955

Leu Gly His Asn Thr Lys Val Asn Gly Lys Gln Ser Ile Ala Ile
    2960                2965                2970

Gly Ser Gly Ala Glu Ala Leu Gly Asn Gln Ser Ile Ser Ile Gly
    2975                2980                2985

Thr Gly Asn Lys Val Thr Gly Asp His Ser Gly Ala Ile Gly Asp
    2990                2995                3000

Pro Thr Ile Val Asn Gly Ala Asn Ser Tyr Ser Val Gly Asn Asn
    3005                3010                3015

Asn Gln Val Leu Thr Asp Asp Thr Phe Val Leu Gly Asn Asn Val
    3020                3025                3030

Thr Lys Thr Ile Ala Gly Ser Val Val Leu Gly Asn Gly Ser Ala

3035                3040                3045

Ala Thr Thr Gly Ala Gly Glu Ala Gly Tyr Ala Leu Ser Val Ala
    3050                3055                3060

Thr Asn Ala Asp Lys Ala Ala Ile Thr Lys Thr Thr Ser Ser Thr
    3065                3070                3075

Gly Ala Val Ala Val Gly Asp Ala Ser Ser Gly Ile Tyr Arg Gln
    3080                3085                3090

Ile Thr Gly Val Ala Ala Gly Ser Val Asp Ser Asp Ala Val Asn
    3095                3100                3105

Val Ala Gln Leu Lys Ala Val Gly Asn Gln Val Val Thr Thr Gln
    3110                3115                3120

Thr Thr Leu Val Asn Ser Leu Gly Gly Asn Ala Lys Val Asn Ala
    3125                3130                3135

Asp Gly Thr Ile Thr Gly Pro Thr Tyr Asn Val Ala Gln Gly Asn
    3140                3145                3150

Gln Thr Asn Val Gly Asp Ala Leu Thr Ala Leu Asp Asn Ala Ile
    3155                3160                3165

Asn Thr Ala Ala Thr Thr Ser Lys Ser Thr Val Ser Asn Gly Gln
    3170                3175                3180

Asn Ile Val Val Ser Lys Ser Lys Asn Ala Asp Gly Ser Asp Asn
    3185                3190                3195

Tyr Glu Val Ser Thr Ala Lys Asp Leu Thr Val Asp Ser Val Lys
    3200                3205                3210

Ala Gly Asp Thr Val Leu Asn Asn Ala Gly Ile Thr Ile Gly Asn
    3215                3220                3225

Asn Ala Val Val Leu Asn Asn Thr Gly Leu Thr Ile Ser Gly Gly
    3230                3235                3240

Pro Ser Val Thr Leu Ala Gly Ile Asp Ala Gly Asn Lys Thr Ile
    3245                3250                3255

Gln Asn Val Ala Asn Ala Val Asn Ala Thr Asp Ala Val Asn Lys
    3260                3265                3270

Gly Gln Leu Asp Ser Ala Ile Asn Asn Val Asn Asn Asn Val Asn
    3275                3280                3285

Glu Leu Ala Asn Asn Ala Val Lys Tyr Asp Asp Ala Ser Lys Asp
    3290                3295                3300

Lys Ile Thr Leu Gly Gly Gly Ala Thr Gly Thr Thr Ile Thr Asn
    3305                3310                3315

Val Lys Asp Gly Thr Val Ala Gln Gly Ser Lys Asp Ala Val Asn
    3320                3325                3330

Gly Gly Gln Leu Trp Asn Val Gln Gln Gln Val Asp Gln Asn Thr
    3335                3340                3345

Thr Asp Ile Ser Asn Ile Lys Asn Asp Ile Asn Asn Gly Thr Val
    3350                3355                3360

Gly Leu Val Gln Gln Ala Gly Lys Asp Ala Pro Val Thr Val Ala
    3365                3370                3375

Lys Asp Thr Gly Gly Thr Thr Val Asn Val Ala Gly Thr Asp Gly
    3380                3385                3390

Asn Arg Val Val Thr Gly Val Lys Glu Gly Ala Val Asn Ala Thr
    3395                3400                3405

Ser Lys Asp Ala Val Asn Gly Ser Gln Leu Asn Thr Thr Asn Gln
    3410                3415                3420

Ala Val Val Asn Tyr Leu Gly Gly Gly Ala Gly Tyr Asp Asn Ile
    3425                3430                3435

Thr Gly Ser Phe Thr Ala Pro Ser Tyr Thr Val Gly Asp Ser Lys
    3440                3445                3450

Tyr Asn Asn Val Gly Gly Ala Ile Asp Ala Leu Asn Gln Ala Asp
    3455                3460                3465

Gln Ala Leu Asn Ser Lys Ile Asp Asn Val Ser Asn Lys Leu Asp
    3470                3475                3480

Asn Ala Phe Arg Ile Thr Asn Asn Arg Ile Asp Asp Val Glu Lys
    3485                3490                3495

Lys Ala Asn Ala Gly Ile Ala Ala Ala Met Ala Leu Glu Ser Ala
    3500                3505                3510

Pro Tyr Val Pro Gly
    3515

<210> 2
<211> 10554
<212> DNA
<213> artificial sequence

<220>
<223> Fragment of AtaA

<400> 2
atggctactg ttcaagttgg agggggaact aattctggaa caactgctac agcttctacg 60

aattgtgcag acttatataa ttatcaaaat cctgagaact caggctctgg agcggctggg 120

aattataatg caggaaatcc aagtgtgtgt tcgatcgcta taggtgaaaa cgcacaaggt 180

ggtacttctg gaactggagg gtcgccaggg atagcgatag gtggaaattc taaagctacg 240

ggtggtttat ctgttgctat aggcggatat gctcaagcga caaatgttgg aagtattgct 300

ttaggcacag cagctttatc aagtggtttt aacagtttag caatatccag acaagctgct 360

gcaacgaata actattcaat agctataggt acaacttcag tttcgaaagg agttggatcg 420

attgctatgg ggcattcaac gaatgcttct ggagatcaat cgatagcaat tggtagctcg 480

gatgctgtta attcagcaac agcaacaaca acatacgatg gtacaacaaa tactcaagca 540

tcaggtagta aatcgattgc tataggtgca agcgcaaagg catcaaccaa taacagcatt 600

gcactaggtg caggatcggt aacttctgca caatctggta attcttatct tactggtgta 660

ggtgcatcag ctacaaatgg tgttgtatct gttggaactt caactgcaac acgtcgtatc 720

caaaatgtag cagatggttc agccgcttca gatgctgtga cagttgctca gttggataaa 780

gcttatgatg atacaaatgg tcgtttagct gctgctttag gtacaggtag tggtgctgcc 840

tataatgcag caaacaatac atataccgct ccaacgaata ttgggggaac aggtaaaaat 900

acgattgatg atgcaattaa agcaactcaa cgaagtgtag tcgctggatc aaatattgtc 960

gttaccccga cgacagcttc tgatggttca atatcgtatt cggttgctac aagcgcaaca 1020

ccgacgttta caagtataac tgtaaacaat gcaccaacgg caggtacaga tgcgaccaac 1080

aagacttatg tagactcaaa agcagcagca tcgagaacag aagtagcagc tggaagcaat 1140

gtatctggtg tagtaaaaac gacaggcgca aacggtcaag acgtttatac agtaaatgcc 1200

aatggtacga ctgcatcagc aggttcttca gcagttaccg taacaccagg cacgaaagat 1260

gcaaataatg tcactgacta taaagtagac ttatcagcga ctacaaaaac cgatatccaa 1320

aaaggtgtag atgcaaaaaa tgctgtagat accgcaggtc taaaatttaa aggtgataca 1380

gcaaccacaa gcaataccaa gaaattaggt gacaccgttt cgattacggg tgatacgaac 1440

attagtacag ttgcgacaac agatggtgta caggttaagt taaatccaaa cttggattta 1500

ggagcaactg gtagcgttaa aacgggtaat accacgatta acaatgcagg tgtaacagct 1560

gatcaagtta cggttggtgg tgttgttatt aacaacacat caggtattaa tgctggtggt 1620

aaagcgatta ctaatgtagc agcaccaaca aataacacag atgctgctaa caagaagtat 1680

gtagatgatg caggtacagc attaaccaat ttgggctttg gattaaaagc acaagatggt 1740

acgactgtga acaagaaatt aggtgaagca gttgatattg ttggttcaaa cagcaacatc 1800

agtacaaaag taaatgcagg caaagtagaa gttgcactat ccaatacatt ggacttaggt 1860

actacaggta gcgttactac gggttcaact gtaattaaca atgctggtgt tacggcaact 1920

caagttaccg caaacaaagt cacaataaac aatgcaccaa cagcaggtac agatgcgacc 1980

aacaagactt atgtagactc aaaagcagca gcatcaagaa cagaagtcgc agctggaagc 2040

aatgtatctg gtgtagtaaa aacgacaggc gcaaacggtc aagatattta tgcagtaaat 2100

gccaatggta cgactgcatc agcaggttct tcagcagtta ccgtaacacc aggcacgaaa 2160

gatgcaaata atgtcactga ctataaagta gacttgtcag cgactacaaa aaccgatatt 2220

caaaaaggtg tagatgcaaa aaatgctgta gatactgcag gtctaaaatt taaaggtgat 2280

acagcaacca caagcaatac caagaaatta ggtgacaccg tttcgattac gggtgatacg 2340

aacattagta cagttgcaac aactgatggt gtacaggtta agttaaatcc aaacttagat 2400

ttaggagcaa ctggtagcgt taaaacgggt aataccacga ttaacaatgc aggtgtaaca 2460

gctgaccaag ttacggttgg tggtgttgtt attaacaaca catcaggtat taatgctggt 2520

ggtaaagcga ttaccaatgt agcagcacca acaaataaca cagatgctgc taacaagaag 2580

tatgtagatg acgcaggtac agcattaacc aatttgggct ttggattaaa agcgcaagat 2640

ggtacgactg tgaacaagaa attaggtgaa gcagttgata ttgttggttc aaacagcaac 2700

atcagtacaa aagtaaatgc aggcaaagta gaagttgcac tatccaatac attggactta 2760

ggtactacag gtagcgttac tacgggttca actgtaatta acaatgctgg tgttacggca 2820

actcaagtta ccgcaaacaa agtcacagtt aataatgcac caacagcagg tacagatgcg 2880

accaataaaa cttatgtaga ctcaaaagca gcggcatcaa gaacagaagt cgcagctgga 2940

agcaatgtat ctggcgtagt aaaaacgaca ggtgcaaacg gtcaagacgt ttatacagta 3000

aatgccaatg gtacgactgc atcagcaggt tcttcagcag ttaccgtaac accaggcacg 3060

aaagatgcaa ataatgtcac tgactataaa gtagacttgt cagcgactac aaaaaccgat 3120

attcaaaaag gtgtagatgc aaaaaatgct gtagataccg caggtctaaa atttaaaggt 3180

gatacagcaa ccacaagcaa taccaagaaa ttaggtgaca ccgtttcgat tacgggtgat 3240

acgaacatta gtacagttgc gacaactgat ggtgtacagg ttaagctaaa tccaaacttg 3300

gatttaggag caactggtag cgttaaaacg ggtaatacca cgattaacaa tgcaggtgta 3360

acagctgatc aagttacagt tggtggtgtt gttattaaca acacatcagg tattaatgct 3420

ggtggtaaag cgattaccaa tgtagcagca ccaacaaata acacagatgc tgctaacaag 3480

aagtatgtag atgatgcagg tacagcatta accaatttgg gctttggatt aaaagcgcaa 3540

gatggtacga ctgtgaacaa gaaattaggc gaagcagttg aagttgttgg tgcggacagt 3600

aacatcacca cgaaagttgc aggcggtcag gttgcaattg agttaaataa aaacctcaac 3660

aacttaactg gcattaccgt gaacgatgga accaatggca ccaatggttc aactgtgatt 3720

ggtaaagatg gtatttcggt taaagatggt tcaggcaata ccattgcagg tgtagataac 3780

acagcgttga cagttaaaga tggcagtggc aacacagaaa ccagcattaa ccaagcgatc 3840

aacacgttaa atgcagcgca aggtgaaact gataagtttg cagtgaagta cgacaaaaat 3900

gctgatggca gtgtgaacta caacaacatc acattggcag gtacgactgc aagcagtaca 3960

caagatgcaa ctacaggcaa gatcaccaca acaggtggaa caagcttgaa caatgttgca 4020

agtgcgggtg actacaaaga tgttgccaat gcaagcaaag gtgtaaacgc aggtgactta 4080

aacaatgcag ttgttgatgc aaccaatgca gcaaccagca aaggctttgc attacaagca 4140

gcagatggcg ctaaagttca gaagaaccta ggcgaagcag ttgaagttgt cggtgccgac 4200

agcaacatca ccacaaaagt tgcaggcggt caggttgcaa ttgagttaaa taaaaacctc 4260

aacaacttaa ctggcattac cgtgaacgat ggaaccaatg gcaccaatgg ttcaactgtg 4320

attggtaaag atggtatttc agttaaagac ggttcaggca ataccattgc aggtgtagat 4380

aacacagcgt tgacagttaa agatggcagt ggcaacacag aaaccagcat taaccaagcg 4440

atcaacacgt taaatgcagc gcaaggtgaa actgataagt ttgcagtgaa gtacgacaaa 4500

aatacggatg gtagtaccaa ctacaacagt attactgcag gcaatggtaa cggtactgca 4560

gcaacgatcg gaactgacac agcaggtaat agtgttgtga ccagtggcgg aactaaaatt 4620

agtaatgttg cgaatggtgt caatgcaagt gatgcagtaa acaaaggtca attggatagc 4680

ttaagtacag gtcttaccaa tacaggcttt ggtttaaaag cagcagatgg caacaccgtt 4740

aacaaaaaat taggcgaagc agtagacgtt gtcggtgctg acagcaacat caccacgaaa 4800

gttgcaggcg gtcaggttgc gattgagtta aataaaaacc tcaacaactt aactggcatt 4860

accgtgaacg atggaaccaa tggcaccaat ggttcaactg tgattggtaa agatggtatt 4920

tcgattaaag atggttcagg caataccatt gcaggtgtag ataacacagc gttgacagtt 4980

aaagatggca gtggcaacac agaaaccagc attaaccaag cgatcaacac gttaaatgca 5040

gcgcaaggtg aaactgacaa gtttgcagtg aagtacgaca agaatgctga tggcagtgca 5100

aactacaaca acatcacatt ggcaggtacg actgcaagta gcacgcaaga tgcaacaaca 5160

ggcaagatca ccacaacagg tggaacaagc ttgaacaacg ttgcaagtgc aggtgactac 5220

aaagatgttg ccaatgcaag caaaggtgta aacgcaggtg acttgaacaa tgcagttgtt 5280

gatgcaacca atgcagcaac cagcaaaggc tttgcattac aagcagcaga tggcgctaaa 5340

gttcagaaga acctaggcga agcagttgaa gttgtcggtg cggacagcaa catcaccaca 5400

aaagtagtgg gtggacaagt tgcgattgag ttaaataaaa acctcaacaa cttaactggc 5460

attaccgtga acgatggaac caatggcaca aatggttcaa ctgtgattgg taaagatggt 5520

atttcggtta aagatggttc aggtaatacc attgcaggtg tagataacac agcgttgaca 5580

gttaaagatg gcagtggcaa cacagaaacc agcattaacc aagcgatcaa cacgttaaat 5640

gcagcgcaag gtgaaactga taagtttgca gtgaagtacg acaaaaatgc tgatggcagt 5700

gtgaactaca acaacatcac attggcaggt acgactgcaa gcagtacaca agatgcaact 5760

acaggcaaga tcaccacaac aggtggaaca agcttgaaca atgttgcaag tgcgggtgac 5820

tacaaagatg ttgccaatgc aagcaaaggt gtaaacgcag gtgacttaaa caatgcagtt 5880

gttgatgcaa ccaatgcagc aaccagcaaa ggctttgcat tacaagcagc agatggcgct 5940

aaagttcaga agaacctagg cgaagcagtt gaagttgtcg gtgccgacag caacatcacc 6000

acaaaagttg caggcggtca ggttgcaatt gagttaaata aaaacctcaa caacttaact 6060

ggcattaccg tgaacgatgg aaccaatggc accaatggtt caactgtgat tggtaaagat 6120

ggtatttcag ttaaagacgg ttcaggcaat accattgcag gtgtagataa cacagcgttg 6180

acagttaaag atggcagtgg caacacagaa accagcatta accaagcgat caacacgtta 6240

aatgcagcgc aaggtgaaac tgataagttt gcagtgaagt acgacaaaaa tgctgatggc 6300

agtgtgaact acaacaacat cacattggca ggtacgactg caagcagtac acaagatgca 6360

actacaggca agatcaccac aacaggtggt acaagcttga acaatgttgc aagtgcgggt 6420

gactacaaag atgttgccaa tgcaagcaaa ggtgtaaacg caggtgactt gaacaatgca 6480

gttgttgatg caaccaatgc agcgaccagc aaaggctttg cattacaagc agcagatggc 6540

gctaaagttc agaagaacct aggcgaagca gttgaagttg ttggtgcgga cagtaacatc 6600

accacgaaag ttgcaggcgg tcaggttgca attgagttaa ataaaaacct caacaactta 6660

actggcatta ccgtgaacga tggaaccaat ggcaccaatg gttcaactgt gattggtaaa 6720

gatggtattt cggttaaaga tggttcaggc aataccattg caggtgtaga taacacagcg 6780

ttgacagtta aagatggcag tggcaacaca gaaaccagca ttaaccaagc gatcaacacg 6840

ttaaatgcag cgcaaggtga aactgataag tttgcagtga agtacgacaa aaatgctgat 6900

ggcagtgcaa actataacaa tgtcacttta gctggtacaa atggcacaat aatcagcaat 6960

gttaaagcgg gtgctgtgac ctcaacatct actgatgcga tcaatggtag ccaattatat 7020

ggtgttgcaa acagcgtgaa gaatgcaatt ggtggttcaa ccacaattga tgcaacgact 7080

ggtgcaatca cgacgaccaa tattggtggt acaggttcaa atacgattga tggtgcaatc 7140

agcagtatta aagattcagc gactaaagcg aaaaccacgg taagtgctgg ggataatgtt 7200

gtcgttacat cgggtaccaa tgcagatggc tcaacaaact atgaagttgc gacagcgaaa 7260

gacgttaact ttgacaaagt gactgtaggt agtgttgttg tagataaatc aagcaataca 7320

atcaaaggat taagtaatac cacttggaac ggaacagcag tatcaggtca agcggcgaca 7380

gaagaccagt taaaaacggt cagcgatgcg caaggtgaaa ctgataagtt tgcagtgaag 7440

tacgacaaaa atgctgatgg cagtgcgaac tacaacagta ttactgcagg caatggtaac 7500

ggtactgcag caacgatcgg aactgacaca gcaggtaata gtgttgtgac cagtggcgga 7560

actaaaatta gtaatgttgc gaatggtgtc aatgcaagtg atgcagtaaa caaaggtcaa 7620

ttggatagct taagtacagg tcttaccaat acaggctttg gtttaaaagc agcagatggc 7680

aacaccgtta acaaaaaatt aggcgaagca gtagacgttg tcggtgctga cagcaacatc 7740

accacgaaag ttgcaggcgg tcaggttgcg attgagttaa ataaaaacct caacaactta 7800

actggcatta ccgtgaacga tggaaccaat ggcaccaatg gttcaactgt gattggtaaa 7860

gatggtattt cgattaaaga tggttcaggc aataccattg caggtgtaga taacacagcg 7920

ttgacggtta aagatagcag tggcaacaca gaaaccagca ttaaccaagc gatcaacacg 7980

ttaaatgcag cgcaaggtga aactgataag tttgcagtga agtacgataa gaatgctgat 8040

ggcagtgtga actataacaa tgtcacttta gcaggtacaa atggcacaat aatcagaaat 8100

gttaaagcgg gtgctgtgac ctcaacatct actgatgcga tcaatggtag ccaattatac 8160

gatattgcaa acagcgtgaa gaatgcaatt ggtggttcaa ccacaagaga tgtaacgact 8220

ggtgcaatca caacgaccaa tattggtggt acaggttcaa acacgattga tggtgcaatc 8280

agcagtatta aagattcagc gactaaagcg aaaaccacga taagtgctgg ggataatgtt 8340

gtcgttacat cgggtaccaa tgcagatggc tcaacaaact atgaagttgc gacagcgaaa 8400

gacgttaact ttgacaaagt aactgtaggt aatgttgttg ttgataaggc aaatgacacg 8460

atccaaggtt tgagcaataa agatctaaat tcaactgatt ttgcgaccaa aggtagagct 8520

gcgactgaag aacagttaaa agcagtgatt accagtaata tcacggaagt tgtggatggt 8580

aatggcaaca aggtgaatat tattgaccaa gttgtaaata ccaaacctga caataagaac 8640

caagattcat tgttcttaac gtatgacaaa caaggtcaag aaaccacaga tcgcctaacg 8700

attggtcaaa cggtacagaa gatgaatact gatggtatta aattcttcca taccaatgcc 8760

gatacatcaa aaggtgattt gggtacaaca aatgactcaa gtgcaggtgg tttaaactct 8820

acagcaattg gtgtaaatgc gattgttgcg aatggtgcag atagttcagt tgctttaggt 8880

cataacacca aagtcaatgg taaacaatca attgcaattg gttctggtgc agaagcttta 8940

ggcaatcaat cgatcagtat tggtacaggc aataaagtca ctggtgatca ttcgggtgcg 9000

attggtgatc caactattgt aaatggtgca aacagctact ctgtgggtaa taacaaccaa 9060

gtacttacag atgacacttt cgtacttgga aacaatgtca ccaaaactat tgctggttca 9120

gtagtattgg gtaacggttc agctgcaacg acaggtgctg gtgaggcagg ctatgcctta 9180

tctgtagcaa caaatgcaga taaagccgcg atcactaaaa ctacgtcaag cactggtgct 9240

gttgcagttg gtgatgcgtc gagcggtatt tatcgtcaaa ttaccggtgt tgctgcgggt 9300

agcgtagatt cagatgctgt gaacgttgca cagttaaaag cggtgggtaa ccaagttgta 9360

acgactcaaa ctacattggt gaacagtttg ggtggtaacg ctaaagtaaa tgcagacggt 9420

acgattacag gaccaactta taatgttgct caaggtaatc agaccaatgt tggtgatgca 9480

ttaactgcgc ttgataacgc aattaatact gcggcaacaa catctaaatc gactgtttct 9540

aatggtcaga atattgttgt cagcaagagc aaaaatgcag atggttcaga caactatgaa 9600

gtatcaacag caaaagactt gacagttgat tctgtcaaag cgggtgatac ggttctgaat 9660

aatgcaggta ttacaattgg caataacgca gttgtattga acaacactgg attaaccatt 9720

agtggtggac caagtgttac cttggcaggc atcgatgcag gcaataaaac cattcaaaat 9780

gttgcgaatg cagtaaatgc aacagatgca gtcaacaaag ggcaattgga cagcgcaatt 9840

aacaatgtga ataacaatgt aaatgagctt gccaacaacg ctgttaaata tgacgatgca 9900

tcaaaagata agatcacact tggtggtggg gcaactggta caacaatcac caatgtgaaa 9960

gatggtactg ttgcgcaagg ttctaaagat gctgtgaatg gcggtcaatt gtggaatgtt 10020

caacaacaag ttgatcagaa cacaactgat attagcaata tcaaaaatga tattaacaac 10080

ggtactgttg gtttggttca acaagcaggt aaagatgcac cagtgacggt tgcaaaagat 10140

actggcggta caacggtgaa tgtcgctgga acagatggca accgagtagt gacaggtgtt 10200

aaggaaggtg cagtgaatgc aacatctaaa gatgctgtca atggtagtca attgaataca 10260

accaaccaag cggtagtcaa ttatcttggt ggtggggcag gttatgacaa cattacaggt 10320

agcttcacag cgccaagtta tacggtaggt gactcgaaat acaacaatgt tggtggcgca 10380

attgatgcat tgaatcaagc agatcaagca ttgaatagca aaattgacaa tgtcagtaac 10440

aagttggata acgcattccg tattaccaac aaccgtattg atgatgtaga gaaaaaagcc 10500

aatgctggta ttgccgctgc gatggctctg gaatcagcac catatgtccc aggt 10554

<210> 3
<211> 10893
<212> DNA
<213> Acinetobacter sp

<220>
<221> CDS
<222> (1)..(10893)

<400> 3

atg aat aaa atc tac aaa gtg att tgg aat gcg act ttg ttg gca tgg 48
Met Asn Lys Ile Tyr Lys Val Ile Trp Asn Ala Thr Leu Leu Ala Trp
1               5                   10                  15

gtt gca gta tct gaa ttg gca aaa ggg aaa acc aaa tct acg aca tca 96
Val Ala Val Ser Glu Leu Ala Lys Gly Lys Thr Lys Ser Thr Thr Ser
            20                  25                  30

aaa tcc aaa gct aaa tca tta tct tca tct gta ata gtt ggt ggg ata 144
Lys Ser Lys Ala Lys Ser Leu Ser Ser Ser Val Ile Val Gly Gly Ile
        35                  40                  45

ata tta aca aca cct tta tct tta ata gca gct act gtt caa gtt gga 192
Ile Leu Thr Thr Pro Leu Ser Leu Ile Ala Ala Thr Val Gln Val Gly
    50                  55                  60

ggg gga act aat tct gga aca act gct aca gct tct acg aat tgt gca 240
Gly Gly Thr Asn Ser Gly Thr Thr Ala Thr Ala Ser Thr Asn Cys Ala
65                  70                  75                  80

gac tta tat aat tat caa aat cct gag aac tca ggc tct gga gcg gct 288
Asp Leu Tyr Asn Tyr Gln Asn Pro Glu Asn Ser Gly Ser Gly Ala Ala
                85                  90                  95

ggg aat tat aat gca gga aat cca agt gtg tgt tcg atc gct ata ggt 336
Gly Asn Tyr Asn Ala Gly Asn Pro Ser Val Cys Ser Ile Ala Ile Gly
            100                 105                 110

gaa aac gca caa ggt ggt act tct gga act gga ggg tcg cca ggg ata 384
Glu Asn Ala Gln Gly Gly Thr Ser Gly Thr Gly Gly Ser Pro Gly Ile
        115                 120                 125

gcg ata ggt gga aat tct aaa gct acg ggt ggt tta tct gtt gct ata 432
Ala Ile Gly Gly Asn Ser Lys Ala Thr Gly Gly Leu Ser Val Ala Ile
    130                 135                 140

ggc gga tat gct caa gcg aca aat gtt gga agt att gct tta ggc aca 480
Gly Gly Tyr Ala Gln Ala Thr Asn Val Gly Ser Ile Ala Leu Gly Thr
145                 150                 155                 160

gca gct tta tca agt ggt ttt aac agt tta gca ata tcc aga caa gct 528
Ala Ala Leu Ser Ser Gly Phe Asn Ser Leu Ala Ile Ser Arg Gln Ala
                165                 170                 175

gct gca acg aat aac tat tca ata gct ata ggt aca act tca gtt tcg 576
Ala Ala Thr Asn Asn Tyr Ser Ile Ala Ile Gly Thr Thr Ser Val Ser
            180                 185                 190

aaa gga gtt gga tcg att gct atg ggg cat tca acg aat gct tct gga 624
Lys Gly Val Gly Ser Ile Ala Met Gly His Ser Thr Asn Ala Ser Gly
        195                 200                 205

gat caa tcg ata gca att ggt agc tcg gat gct gtt aat tca gca aca 672
Asp Gln Ser Ile Ala Ile Gly Ser Ser Asp Ala Val Asn Ser Ala Thr
    210                 215                 220

gca aca aca aca tac gat ggt aca aca aat act caa gca tca ggt agt 720
Ala Thr Thr Thr Tyr Asp Gly Thr Thr Asn Thr Gln Ala Ser Gly Ser
225                 230                 235                 240

aaa tcg att gct ata ggt gca agc gca aag gca tca acc aat aac agc 768
Lys Ser Ile Ala Ile Gly Ala Ser Ala Lys Ala Ser Thr Asn Asn Ser
                245                 250                 255

att gca cta ggt gca gga tcg gta act tct gca caa tct ggt aat tct     816
Ile Ala Leu Gly Ala Gly Ser Val Thr Ser Ala Gln Ser Gly Asn Ser
            260                 265                 270

tat ctt act ggt gta ggt gca tca gct aca aat ggt gtt gta tct gtt     864
Tyr Leu Thr Gly Val Gly Ala Ser Ala Thr Asn Gly Val Val Ser Val
        275                 280                 285

gga act tca act gca aca cgt cgt atc caa aat gta gca gat ggt tca     912
Gly Thr Ser Thr Ala Thr Arg Arg Ile Gln Asn Val Ala Asp Gly Ser
    290                 295                 300

gcc gct tca gat gct gtg aca gtt gct cag ttg gat aaa gct tat gat     960
Ala Ala Ser Asp Ala Val Thr Val Ala Gln Leu Asp Lys Ala Tyr Asp
305                 310                 315                 320

gat aca aat ggt cgt tta gct gct gct tta ggt aca ggt agt ggt gct     1008
Asp Thr Asn Gly Arg Leu Ala Ala Ala Leu Gly Thr Gly Ser Gly Ala
                325                 330                 335

gcc tat aat gca gca aac aat aca tat acc gct cca acg aat att ggg     1056
Ala Tyr Asn Ala Ala Asn Asn Thr Tyr Thr Ala Pro Thr Asn Ile Gly
            340                 345                 350

gga aca ggt aaa aat acg att gat gat gca att aaa gca act caa cga     1104
Gly Thr Gly Lys Asn Thr Ile Asp Asp Ala Ile Lys Ala Thr Gln Arg
        355                 360                 365

agt gta gtc gct gga tca aat att gtc gtt acc ccg acg aca gct tct     1152
Ser Val Val Ala Gly Ser Asn Ile Val Val Thr Pro Thr Thr Ala Ser
    370                 375                 380

gat ggt tca ata tcg tat tcg gtt gct aca agc gca aca ccg acg ttt     1200
Asp Gly Ser Ile Ser Tyr Ser Val Ala Thr Ser Ala Thr Pro Thr Phe
385                 390                 395                 400

aca agt ata act gta aac aat gca cca acg gca ggt aca gat gcg acc     1248
Thr Ser Ile Thr Val Asn Asn Ala Pro Thr Ala Gly Thr Asp Ala Thr
                405                 410                 415

aac aag act tat gta gac tca aaa gca gca gca tcg aga aca gaa gta     1296
Asn Lys Thr Tyr Val Asp Ser Lys Ala Ala Ala Ser Arg Thr Glu Val
            420                 425                 430

gca gct gga agc aat gta tct ggt gta gta aaa acg aca ggc gca aac     1344
Ala Ala Gly Ser Asn Val Ser Gly Val Val Lys Thr Thr Gly Ala Asn
        435                 440                 445

ggt caa gac gtt tat aca gta aat gcc aat ggt acg act gca tca gca     1392
Gly Gln Asp Val Tyr Thr Val Asn Ala Asn Gly Thr Thr Ala Ser Ala
    450                 455                 460

ggt tct tca gca gtt acc gta aca cca ggc acg aaa gat gca aat aat     1440
Gly Ser Ser Ala Val Thr Val Thr Pro Gly Thr Lys Asp Ala Asn Asn
465                 470                 475                 480

gtc act gac tat aaa gta gac tta tca gcg act aca aaa acc gat atc     1488
Val Thr Asp Tyr Lys Val Asp Leu Ser Ala Thr Thr Lys Thr Asp Ile
                485                 490                 495

caa aaa ggt gta gat gca aaa aat gct gta gat acc gca ggt cta aaa     1536
Gln Lys Gly Val Asp Ala Lys Asn Ala Val Asp Thr Ala Gly Leu Lys

500                 505                 510

ttt aaa ggt gat aca gca acc aca agc aat acc aag aaa tta ggt gac        1584
Phe Lys Gly Asp Thr Ala Thr Thr Ser Asn Thr Lys Lys Leu Gly Asp
        515                 520                 525

acc gtt tcg att acg ggt gat acg aac att agt aca gtt gcg aca aca        1632
Thr Val Ser Ile Thr Gly Asp Thr Asn Ile Ser Thr Val Ala Thr Thr
    530                 535                 540

gat ggt gta cag gtt aag tta aat cca aac ttg gat tta gga gca act        1680
Asp Gly Val Gln Val Lys Leu Asn Pro Asn Leu Asp Leu Gly Ala Thr
545                 550                 555                 560

ggt agc gtt aaa acg ggt aat acc acg att aac aat gca ggt gta aca        1728
Gly Ser Val Lys Thr Gly Asn Thr Thr Ile Asn Asn Ala Gly Val Thr
                565                 570                 575

gct gat caa gtt acg gtt ggt ggt gtt gtt att aac aac aca tca ggt        1776
Ala Asp Gln Val Thr Val Gly Gly Val Val Ile Asn Asn Thr Ser Gly
            580                 585                 590

att aat gct ggt ggt aaa gcg att act aat gta gca gca cca aca aat        1824
Ile Asn Ala Gly Gly Lys Ala Ile Thr Asn Val Ala Ala Pro Thr Asn
        595                 600                 605

aac aca gat gct gct aac aag aag tat gta gat gat gca ggt aca gca        1872
Asn Thr Asp Ala Ala Asn Lys Lys Tyr Val Asp Asp Ala Gly Thr Ala
    610                 615                 620

tta acc aat ttg ggc ttt gga tta aaa gca caa gat ggt acg act gtg        1920
Leu Thr Asn Leu Gly Phe Gly Leu Lys Ala Gln Asp Gly Thr Thr Val
625                 630                 635                 640

aac aag aaa tta ggt gaa gca gtt gat att gtt ggt tca aac agc aac        1968
Asn Lys Lys Leu Gly Glu Ala Val Asp Ile Val Gly Ser Asn Ser Asn
                645                 650                 655

atc agt aca aaa gta aat gca ggc aaa gta gaa gtt gca cta tcc aat        2016
Ile Ser Thr Lys Val Asn Ala Gly Lys Val Glu Val Ala Leu Ser Asn
            660                 665                 670

aca ttg gac tta ggt act aca ggt agc gtt act acg ggt tca act gta        2064
Thr Leu Asp Leu Gly Thr Thr Gly Ser Val Thr Thr Gly Ser Thr Val
        675                 680                 685

att aac aat gct ggt gtt acg gca act caa gtt acc gca aac aaa gtc        2112
Ile Asn Asn Ala Gly Val Thr Ala Thr Gln Val Thr Ala Asn Lys Val
    690                 695                 700

aca ata aac aat gca cca aca gca ggt aca gat gcg acc aac aag act        2160
Thr Ile Asn Asn Ala Pro Thr Ala Gly Thr Asp Ala Thr Asn Lys Thr
705                 710                 715                 720

tat gta gac tca aaa gca gca gca tca aga aca gaa gtc gca gct gga        2208
Tyr Val Asp Ser Lys Ala Ala Ala Ser Arg Thr Glu Val Ala Ala Gly
                725                 730                 735

agc aat gta tct ggt gta gta aaa acg aca ggc gca aac ggt caa gat        2256
Ser Asn Val Ser Gly Val Val Lys Thr Thr Gly Ala Asn Gly Gln Asp
            740                 745                 750

att tat gca gta aat gcc aat ggt acg act gca tca gca ggt tct tca        2304

Ile Tyr Ala Val Asn Ala Asn Gly Thr Thr Ala Ser Ala Gly Ser Ser
        755                 760                 765

gca gtt acc gta aca cca ggc acg aaa gat gca aat aat gtc act gac        2352
Ala Val Thr Val Thr Pro Gly Thr Lys Asp Ala Asn Asn Val Thr Asp
    770                 775                 780

tat aaa gta gac ttg tca gcg act aca aaa acc gat att caa aaa ggt        2400
Tyr Lys Val Asp Leu Ser Ala Thr Thr Lys Thr Asp Ile Gln Lys Gly
785                 790                 795                 800

gta gat gca aaa aat gct gta gat act gca ggt cta aaa ttt aaa ggt        2448
Val Asp Ala Lys Asn Ala Val Asp Thr Ala Gly Leu Lys Phe Lys Gly
                805                 810                 815

gat aca gca acc aca agc aat acc aag aaa tta ggt gac acc gtt tcg        2496
Asp Thr Ala Thr Thr Ser Asn Thr Lys Lys Leu Gly Asp Thr Val Ser
            820                 825                 830

att acg ggt gat acg aac att agt aca gtt gca aca act gat ggt gta        2544
Ile Thr Gly Asp Thr Asn Ile Ser Thr Val Ala Thr Thr Asp Gly Val
        835                 840                 845

cag gtt aag tta aat cca aac tta gat tta gga gca act ggt agc gtt        2592
Gln Val Lys Leu Asn Pro Asn Leu Asp Leu Gly Ala Thr Gly Ser Val
    850                 855                 860

aaa acg ggt aat acc acg att aac aat gca ggt gta aca gct gac caa        2640
Lys Thr Gly Asn Thr Thr Ile Asn Asn Ala Gly Val Thr Ala Asp Gln
865                 870                 875                 880

gtt acg gtt ggt ggt gtt gtt att aac aac aca tca ggt att aat gct        2688
Val Thr Val Gly Gly Val Val Ile Asn Asn Thr Ser Gly Ile Asn Ala
                885                 890                 895

ggt ggt aaa gcg att acc aat gta gca gca cca aca aat aac aca gat        2736
Gly Gly Lys Ala Ile Thr Asn Val Ala Ala Pro Thr Asn Asn Thr Asp
            900                 905                 910

gct gct aac aag aag tat gta gat gac gca ggt aca gca tta acc aat        2784
Ala Ala Asn Lys Lys Tyr Val Asp Asp Ala Gly Thr Ala Leu Thr Asn
        915                 920                 925

ttg ggc ttt gga tta aaa gcg caa gat ggt acg act gtg aac aag aaa        2832
Leu Gly Phe Gly Leu Lys Ala Gln Asp Gly Thr Thr Val Asn Lys Lys
    930                 935                 940

tta ggt gaa gca gtt gat att gtt ggt tca aac agc aac atc agt aca        2880
Leu Gly Glu Ala Val Asp Ile Val Gly Ser Asn Ser Asn Ile Ser Thr
945                 950                 955                 960

aaa gta aat gca ggc aaa gta gaa gtt gca cta tcc aat aca ttg gac        2928
Lys Val Asn Ala Gly Lys Val Glu Val Ala Leu Ser Asn Thr Leu Asp
                965                 970                 975

tta ggt act aca ggt agc gtt act acg ggt tca act gta att aac aat        2976
Leu Gly Thr Thr Gly Ser Val Thr Thr Gly Ser Thr Val Ile Asn Asn
            980                 985                 990

gct ggt gtt acg gca act caa gtt  acc gca aac aaa gtc  aca gtt aat        3024
Ala Gly Val Thr Ala Thr Gln Val  Thr Ala Asn Lys Val  Thr Val Asn
        995                 1000                1005

aat gca cca aca gca ggt aca gat gcg acc aat aaa act tat gta 3069
Asn Ala Pro Thr Ala Gly Thr Asp Ala Thr Asn Lys Thr Tyr Val
    1010                1015                1020

gac tca aaa gca gcg gca tca aga aca gaa gtc gca gct gga agc 3114
Asp Ser Lys Ala Ala Ala Ser Arg Thr Glu Val Ala Ala Gly Ser
    1025                1030                1035

aat gta tct ggc gta gta aaa acg aca ggt gca aac ggt caa gac 3159
Asn Val Ser Gly Val Val Lys Thr Thr Gly Ala Asn Gly Gln Asp
    1040                1045                1050

gtt tat aca gta aat gcc aat ggt acg act gca tca gca ggt tct 3204
Val Tyr Thr Val Asn Ala Asn Gly Thr Thr Ala Ser Ala Gly Ser
    1055                1060                1065

tca gca gtt acc gta aca cca ggc acg aaa gat gca aat aat gtc 3249
Ser Ala Val Thr Val Thr Pro Gly Thr Lys Asp Ala Asn Asn Val
    1070                1075                1080

act gac tat aaa gta gac ttg tca gcg act aca aaa acc gat att 3294
Thr Asp Tyr Lys Val Asp Leu Ser Ala Thr Thr Lys Thr Asp Ile
    1085                1090                1095

caa aaa ggt gta gat gca aaa aat gct gta gat acc gca ggt cta 3339
Gln Lys Gly Val Asp Ala Lys Asn Ala Val Asp Thr Ala Gly Leu
    1100                1105                1110

aaa ttt aaa ggt gat aca gca acc aca agc aat acc aag aaa tta 3384
Lys Phe Lys Gly Asp Thr Ala Thr Thr Ser Asn Thr Lys Lys Leu
    1115                1120                1125

ggt gac acc gtt tcg att acg ggt gat acg aac att agt aca gtt 3429
Gly Asp Thr Val Ser Ile Thr Gly Asp Thr Asn Ile Ser Thr Val
    1130                1135                1140

gcg aca act gat ggt gta cag gtt aag cta aat cca aac ttg gat 3474
Ala Thr Thr Asp Gly Val Gln Val Lys Leu Asn Pro Asn Leu Asp
    1145                1150                1155

tta gga gca act ggt agc gtt aaa acg ggt aat acc acg att aac 3519
Leu Gly Ala Thr Gly Ser Val Lys Thr Gly Asn Thr Thr Ile Asn
    1160                1165                1170

aat gca ggt gta aca gct gat caa gtt aca gtt ggt ggt gtt gtt 3564
Asn Ala Gly Val Thr Ala Asp Gln Val Thr Val Gly Gly Val Val
    1175                1180                1185

att aac aac aca tca ggt att aat gct ggt ggt aaa gcg att acc 3609
Ile Asn Asn Thr Ser Gly Ile Asn Ala Gly Gly Lys Ala Ile Thr
    1190                1195                1200

aat gta gca gca cca aca aat aac aca gat gct gct aac aag aag 3654
Asn Val Ala Ala Pro Thr Asn Asn Thr Asp Ala Ala Asn Lys Lys
    1205                1210                1215

tat gta gat gat gca ggt aca gca tta acc aat ttg ggc ttt gga 3699
Tyr Val Asp Asp Ala Gly Thr Ala Leu Thr Asn Leu Gly Phe Gly
    1220                1225                1230

tta aaa gcg caa gat ggt acg act gtg aac aag aaa tta ggc gaa 3744
Leu Lys Ala Gln Asp Gly Thr Thr Val Asn Lys Lys Leu Gly Glu
    1235                1240                1245

gca gtt gaa gtt gtt ggt gcg gac agt aac atc acc acg aaa gtt 3789
Ala Val Glu Val Val Gly Ala Asp Ser Asn Ile Thr Thr Lys Val
    1250                1255                1260

gca ggc ggt cag gtt gca att gag tta aat aaa aac ctc aac aac 3834
Ala Gly Gly Gln Val Ala Ile Glu Leu Asn Lys Asn Leu Asn Asn
    1265                1270                1275

tta act ggc att acc gtg aac gat gga acc aat ggc acc aat ggt 3879
Leu Thr Gly Ile Thr Val Asn Asp Gly Thr Asn Gly Thr Asn Gly
    1280                1285                1290

tca act gtg att ggt aaa gat ggt att tcg gtt aaa gat ggt tca 3924
Ser Thr Val Ile Gly Lys Asp Gly Ile Ser Val Lys Asp Gly Ser
    1295                1300                1305

ggc aat acc att gca ggt gta gat aac aca gcg ttg aca gtt aaa 3969
Gly Asn Thr Ile Ala Gly Val Asp Asn Thr Ala Leu Thr Val Lys
    1310                1315                1320

gat ggc agt ggc aac aca gaa acc agc att aac caa gcg atc aac 4014
Asp Gly Ser Gly Asn Thr Glu Thr Ser Ile Asn Gln Ala Ile Asn
    1325                1330                1335

acg tta aat gca gcg caa ggt gaa act gat aag ttt gca gtg aag 4059
Thr Leu Asn Ala Ala Gln Gly Glu Thr Asp Lys Phe Ala Val Lys
    1340                1345                1350

tac gac aaa aat gct gat ggc agt gtg aac tac aac aac atc aca 4104
Tyr Asp Lys Asn Ala Asp Gly Ser Val Asn Tyr Asn Asn Ile Thr
    1355                1360                1365

ttg gca ggt acg act gca agc agt aca caa gat gca act aca ggc 4149
Leu Ala Gly Thr Thr Ala Ser Ser Thr Gln Asp Ala Thr Thr Gly
    1370                1375                1380

aag atc acc aca aca ggt gga aca agc ttg aac aat gtt gca agt 4194
Lys Ile Thr Thr Thr Gly Gly Thr Ser Leu Asn Asn Val Ala Ser
    1385                1390                1395

gcg ggt gac tac aaa gat gtt gcc aat gca agc aaa ggt gta aac 4239
Ala Gly Asp Tyr Lys Asp Val Ala Asn Ala Ser Lys Gly Val Asn
    1400                1405                1410

gca ggt gac tta aac aat gca gtt gtt gat gca acc aat gca gca 4284
Ala Gly Asp Leu Asn Asn Ala Val Val Asp Ala Thr Asn Ala Ala
    1415                1420                1425

acc agc aaa ggc ttt gca tta caa gca gca gat ggc gct aaa gtt 4329
Thr Ser Lys Gly Phe Ala Leu Gln Ala Ala Asp Gly Ala Lys Val
    1430                1435                1440

cag aag aac cta ggc gaa gca gtt gaa gtt gtc ggt gcc gac agc 4374
Gln Lys Asn Leu Gly Glu Ala Val Glu Val Val Gly Ala Asp Ser
    1445                1450                1455

aac atc acc aca aaa gtt gca ggc ggt cag gtt gca att gag tta 4419
Asn Ile Thr Thr Lys Val Ala Gly Gly Gln Val Ala Ile Glu Leu
    1460                1465                1470

aat aaa aac ctc aac aac tta act ggc att acc gtg aac gat gga 4464
Asn Lys Asn Leu Asn Asn Leu Thr Gly Ile Thr Val Asn Asp Gly

1475                1480                1485

acc aat ggc acc aat ggt tca act gtg att ggt aaa gat ggt att      4509
Thr Asn Gly Thr Asn Gly Ser Thr Val Ile Gly Lys Asp Gly Ile
    1490                1495                1500

tca gtt aaa gac ggt tca ggc aat acc att gca ggt gta gat aac      4554
Ser Val Lys Asp Gly Ser Gly Asn Thr Ile Ala Gly Val Asp Asn
    1505                1510                1515

aca gcg ttg aca gtt aaa gat ggc agt ggc aac aca gaa acc agc      4599
Thr Ala Leu Thr Val Lys Asp Gly Ser Gly Asn Thr Glu Thr Ser
    1520                1525                1530

att aac caa gcg atc aac acg tta aat gca gcg caa ggt gaa act      4644
Ile Asn Gln Ala Ile Asn Thr Leu Asn Ala Ala Gln Gly Glu Thr
    1535                1540                1545

gat aag ttt gca gtg aag tac gac aaa aat acg gat ggt agt acc      4689
Asp Lys Phe Ala Val Lys Tyr Asp Lys Asn Thr Asp Gly Ser Thr
    1550                1555                1560

aac tac aac agt att act gca ggc aat ggt aac ggt act gca gca      4734
Asn Tyr Asn Ser Ile Thr Ala Gly Asn Gly Asn Gly Thr Ala Ala
    1565                1570                1575

acg atc gga act gac aca gca ggt aat agt gtt gtg acc agt ggc      4779
Thr Ile Gly Thr Asp Thr Ala Gly Asn Ser Val Val Thr Ser Gly
    1580                1585                1590

gga act aaa att agt aat gtt gcg aat ggt gtc aat gca agt gat      4824
Gly Thr Lys Ile Ser Asn Val Ala Asn Gly Val Asn Ala Ser Asp
    1595                1600                1605

gca gta aac aaa ggt caa ttg gat agc tta agt aca ggt ctt acc      4869
Ala Val Asn Lys Gly Gln Leu Asp Ser Leu Ser Thr Gly Leu Thr
    1610                1615                1620

aat aca ggc ttt ggt tta aaa gca gca gat ggc aac acc gtt aac      4914
Asn Thr Gly Phe Gly Leu Lys Ala Ala Asp Gly Asn Thr Val Asn
    1625                1630                1635

aaa aaa tta ggc gaa gca gta gac gtt gtc ggt gct gac agc aac      4959
Lys Lys Leu Gly Glu Ala Val Asp Val Val Gly Ala Asp Ser Asn
    1640                1645                1650

atc acc acg aaa gtt gca ggc ggt cag gtt gcg att gag tta aat      5004
Ile Thr Thr Lys Val Ala Gly Gly Gln Val Ala Ile Glu Leu Asn
    1655                1660                1665

aaa aac ctc aac aac tta act ggc att acc gtg aac gat gga acc      5049
Lys Asn Leu Asn Asn Leu Thr Gly Ile Thr Val Asn Asp Gly Thr
    1670                1675                1680

aat ggc acc aat ggt tca act gtg att ggt aaa gat ggt att tcg      5094
Asn Gly Thr Asn Gly Ser Thr Val Ile Gly Lys Asp Gly Ile Ser
    1685                1690                1695

att aaa gat ggt tca ggc aat acc att gca ggt gta gat aac aca      5139
Ile Lys Asp Gly Ser Gly Asn Thr Ile Ala Gly Val Asp Asn Thr
    1700                1705                1710

gcg ttg aca gtt aaa gat ggc agt ggc aac aca gaa acc agc att      5184

Ala Leu Thr Val Lys Asp Gly Ser Gly Asn Thr Glu Thr Ser Ile
    1715                1720                1725

aac caa gcg atc aac acg tta aat gca gcg caa ggt gaa act gac 5229
Asn Gln Ala Ile Asn Thr Leu Asn Ala Ala Gln Gly Glu Thr Asp
    1730                1735                1740

aag ttt gca gtg aag tac gac aag aat gct gat ggc agt gca aac 5274
Lys Phe Ala Val Lys Tyr Asp Lys Asn Ala Asp Gly Ser Ala Asn
    1745                1750                1755

tac aac aac atc aca ttg gca ggt acg act gca agt agc acg caa 5319
Tyr Asn Asn Ile Thr Leu Ala Gly Thr Thr Ala Ser Ser Thr Gln
    1760                1765                1770

gat gca aca aca ggc aag atc acc aca aca ggt gga aca agc ttg 5364
Asp Ala Thr Thr Gly Lys Ile Thr Thr Thr Gly Gly Thr Ser Leu
    1775                1780                1785

aac aac gtt gca agt gca ggt gac tac aaa gat gtt gcc aat gca 5409
Asn Asn Val Ala Ser Ala Gly Asp Tyr Lys Asp Val Ala Asn Ala
    1790                1795                1800

agc aaa ggt gta aac gca ggt gac ttg aac aat gca gtt gtt gat 5454
Ser Lys Gly Val Asn Ala Gly Asp Leu Asn Asn Ala Val Val Asp
    1805                1810                1815

gca acc aat gca gca acc agc aaa ggc ttt gca tta caa gca gca 5499
Ala Thr Asn Ala Ala Thr Ser Lys Gly Phe Ala Leu Gln Ala Ala
    1820                1825                1830

gat ggc gct aaa gtt cag aag aac cta ggc gaa gca gtt gaa gtt 5544
Asp Gly Ala Lys Val Gln Lys Asn Leu Gly Glu Ala Val Glu Val
    1835                1840                1845

gtc ggt gcg gac agc aac atc acc aca aaa gta gtg ggt gga caa 5589
Val Gly Ala Asp Ser Asn Ile Thr Thr Lys Val Val Gly Gly Gln
    1850                1855                1860

gtt gcg att gag tta aat aaa aac ctc aac aac tta act ggc att 5634
Val Ala Ile Glu Leu Asn Lys Asn Leu Asn Asn Leu Thr Gly Ile
    1865                1870                1875

acc gtg aac gat gga acc aat ggc aca aat ggt tca act gtg att 5679
Thr Val Asn Asp Gly Thr Asn Gly Thr Asn Gly Ser Thr Val Ile
    1880                1885                1890

ggt aaa gat ggt att tcg gtt aaa gat ggt tca ggt aat acc att 5724
Gly Lys Asp Gly Ile Ser Val Lys Asp Gly Ser Gly Asn Thr Ile
    1895                1900                1905

gca ggt gta gat aac aca gcg ttg aca gtt aaa gat ggc agt ggc 5769
Ala Gly Val Asp Asn Thr Ala Leu Thr Val Lys Asp Gly Ser Gly
    1910                1915                1920

aac aca gaa acc agc att aac caa gcg atc aac acg tta aat gca 5814
Asn Thr Glu Thr Ser Ile Asn Gln Ala Ile Asn Thr Leu Asn Ala
    1925                1930                1935

gcg caa ggt gaa act gat aag ttt gca gtg aag tac gac aaa aat 5859
Ala Gln Gly Glu Thr Asp Lys Phe Ala Val Lys Tyr Asp Lys Asn
    1940                1945                1950

gct gat ggc agt gtg aac tac aac aac atc aca ttg gca ggt acg 5904
Ala Asp Gly Ser Val Asn Tyr Asn Asn Ile Thr Leu Ala Gly Thr
    1955                1960                1965

act gca agc agt aca caa gat gca act aca ggc aag atc acc aca 5949
Thr Ala Ser Ser Thr Gln Asp Ala Thr Thr Gly Lys Ile Thr Thr
    1970                1975                1980

aca ggt gga aca agc ttg aac aat gtt gca agt gcg ggt gac tac 5994
Thr Gly Gly Thr Ser Leu Asn Asn Val Ala Ser Ala Gly Asp Tyr
    1985                1990                1995

aaa gat gtt gcc aat gca agc aaa ggt gta aac gca ggt gac tta 6039
Lys Asp Val Ala Asn Ala Ser Lys Gly Val Asn Ala Gly Asp Leu
    2000                2005                2010

aac aat gca gtt gtt gat gca acc aat gca gca acc agc aaa ggc 6084
Asn Asn Ala Val Val Asp Ala Thr Asn Ala Ala Thr Ser Lys Gly
    2015                2020                2025

ttt gca tta caa gca gca gat ggc gct aaa gtt cag aag aac cta 6129
Phe Ala Leu Gln Ala Ala Asp Gly Ala Lys Val Gln Lys Asn Leu
    2030                2035                2040

ggc gaa gca gtt gaa gtt gtc ggt gcc gac agc aac atc acc aca 6174
Gly Glu Ala Val Glu Val Val Gly Ala Asp Ser Asn Ile Thr Thr
    2045                2050                2055

aaa gtt gca ggc ggt cag gtt gca att gag tta aat aaa aac ctc 6219
Lys Val Ala Gly Gly Gln Val Ala Ile Glu Leu Asn Lys Asn Leu
    2060                2065                2070

aac aac tta act ggc att acc gtg aac gat gga acc aat ggc acc 6264
Asn Asn Leu Thr Gly Ile Thr Val Asn Asp Gly Thr Asn Gly Thr
    2075                2080                2085

aat ggt tca act gtg att ggt aaa gat ggt att tca gtt aaa gac 6309
Asn Gly Ser Thr Val Ile Gly Lys Asp Gly Ile Ser Val Lys Asp
    2090                2095                2100

ggt tca ggc aat acc att gca ggt gta gat aac aca gcg ttg aca 6354
Gly Ser Gly Asn Thr Ile Ala Gly Val Asp Asn Thr Ala Leu Thr
    2105                2110                2115

gtt aaa gat ggc agt ggc aac aca gaa acc agc att aac caa gcg 6399
Val Lys Asp Gly Ser Gly Asn Thr Glu Thr Ser Ile Asn Gln Ala
    2120                2125                2130

atc aac acg tta aat gca gcg caa ggt gaa act gat aag ttt gca 6444
Ile Asn Thr Leu Asn Ala Ala Gln Gly Glu Thr Asp Lys Phe Ala
    2135                2140                2145

gtg aag tac gac aaa aat gct gat ggc agt gtg aac tac aac aac 6489
Val Lys Tyr Asp Lys Asn Ala Asp Gly Ser Val Asn Tyr Asn Asn
    2150                2155                2160

atc aca ttg gca ggt acg act gca agc agt aca caa gat gca act 6534
Ile Thr Leu Ala Gly Thr Thr Ala Ser Ser Thr Gln Asp Ala Thr
    2165                2170                2175

aca ggc aag atc acc aca aca ggt ggt aca agc ttg aac aat gtt 6579
Thr Gly Lys Ile Thr Thr Thr Gly Gly Thr Ser Leu Asn Asn Val
    2180                2185                2190

gca agt gcg ggt gac tac aaa gat gtt gcc aat gca agc aaa ggt 6624
Ala Ser Ala Gly Asp Tyr Lys Asp Val Ala Asn Ala Ser Lys Gly
    2195                2200                2205

gta aac gca ggt gac ttg aac aat gca gtt gtt gat gca acc aat 6669
Val Asn Ala Gly Asp Leu Asn Asn Ala Val Val Asp Ala Thr Asn
    2210                2215                2220

gca gcg acc agc aaa ggc ttt gca tta caa gca gca gat ggc gct 6714
Ala Ala Thr Ser Lys Gly Phe Ala Leu Gln Ala Ala Asp Gly Ala
    2225                2230                2235

aaa gtt cag aag aac cta ggc gaa gca gtt gaa gtt gtt ggt gcg 6759
Lys Val Gln Lys Asn Leu Gly Glu Ala Val Glu Val Val Gly Ala
    2240                2245                2250

gac agt aac atc acc acg aaa gtt gca ggc ggt cag gtt gca att 6804
Asp Ser Asn Ile Thr Thr Lys Val Ala Gly Gly Gln Val Ala Ile
    2255                2260                2265

gag tta aat aaa aac ctc aac aac tta act ggc att acc gtg aac 6849
Glu Leu Asn Lys Asn Leu Asn Asn Leu Thr Gly Ile Thr Val Asn
    2270                2275                2280

gat gga acc aat ggc acc aat ggt tca act gtg att ggt aaa gat 6894
Asp Gly Thr Asn Gly Thr Asn Gly Ser Thr Val Ile Gly Lys Asp
    2285                2290                2295

ggt att tcg gtt aaa gat ggt tca ggc aat acc att gca ggt gta 6939
Gly Ile Ser Val Lys Asp Gly Ser Gly Asn Thr Ile Ala Gly Val
    2300                2305                2310

gat aac aca gcg ttg aca gtt aaa gat ggc agt ggc aac aca gaa 6984
Asp Asn Thr Ala Leu Thr Val Lys Asp Gly Ser Gly Asn Thr Glu
    2315                2320                2325

acc agc att aac caa gcg atc aac acg tta aat gca gcg caa ggt 7029
Thr Ser Ile Asn Gln Ala Ile Asn Thr Leu Asn Ala Ala Gln Gly
    2330                2335                2340

gaa act gat aag ttt gca gtg aag tac gac aaa aat gct gat ggc 7074
Glu Thr Asp Lys Phe Ala Val Lys Tyr Asp Lys Asn Ala Asp Gly
    2345                2350                2355

agt gca aac tat aac aat gtc act tta gct ggt aca aat ggc aca 7119
Ser Ala Asn Tyr Asn Asn Val Thr Leu Ala Gly Thr Asn Gly Thr
    2360                2365                2370

ata atc agc aat gtt aaa gcg ggt gct gtg acc tca aca tct act 7164
Ile Ile Ser Asn Val Lys Ala Gly Ala Val Thr Ser Thr Ser Thr
    2375                2380                2385

gat gcg atc aat ggt agc caa tta tat ggt gtt gca aac agc gtg 7209
Asp Ala Ile Asn Gly Ser Gln Leu Tyr Gly Val Ala Asn Ser Val
    2390                2395                2400

aag aat gca att ggt ggt tca acc aca att gat gca acg act ggt 7254
Lys Asn Ala Ile Gly Gly Ser Thr Thr Ile Asp Ala Thr Thr Gly
    2405                2410                2415

gca atc acg acg acc aat att ggt ggt aca ggt tca aat acg att 7299
Ala Ile Thr Thr Thr Asn Ile Gly Gly Thr Gly Ser Asn Thr Ile

2420                    2425                    2430

gat ggt gca atc agc agt att aaa gat tca gcg act aaa gcg aaa          7344
Asp Gly Ala Ile Ser Ser Ile Lys Asp Ser Ala Thr Lys Ala Lys
    2435                    2440                    2445

acc acg gta agt gct ggg gat aat gtt gtc gtt aca tcg ggt acc          7389
Thr Thr Val Ser Ala Gly Asp Asn Val Val Val Thr Ser Gly Thr
    2450                    2455                    2460

aat gca gat ggc tca aca aac tat gaa gtt gcg aca gcg aaa gac          7434
Asn Ala Asp Gly Ser Thr Asn Tyr Glu Val Ala Thr Ala Lys Asp
    2465                    2470                    2475

gtt aac ttt gac aaa gtg act gta ggt agt gtt gtt gta gat aaa          7479
Val Asn Phe Asp Lys Val Thr Val Gly Ser Val Val Val Asp Lys
    2480                    2485                    2490

tca agc aat aca atc aaa gga tta agt aat acc act tgg aac gga          7524
Ser Ser Asn Thr Ile Lys Gly Leu Ser Asn Thr Thr Trp Asn Gly
    2495                    2500                    2505

aca gca gta tca ggt caa gcg gcg aca gaa gac cag tta aaa acg          7569
Thr Ala Val Ser Gly Gln Ala Ala Thr Glu Asp Gln Leu Lys Thr
    2510                    2515                    2520

gtc agc gat gcg caa ggt gaa act gat aag ttt gca gtg aag tac          7614
Val Ser Asp Ala Gln Gly Glu Thr Asp Lys Phe Ala Val Lys Tyr
    2525                    2530                    2535

gac aaa aat gct gat ggc agt gcg aac tac aac agt att act gca          7659
Asp Lys Asn Ala Asp Gly Ser Ala Asn Tyr Asn Ser Ile Thr Ala
    2540                    2545                    2550

ggc aat ggt aac ggt act gca gca acg atc gga act gac aca gca          7704
Gly Asn Gly Asn Gly Thr Ala Ala Thr Ile Gly Thr Asp Thr Ala
    2555                    2560                    2565

ggt aat agt gtt gtg acc agt ggc gga act aaa att agt aat gtt          7749
Gly Asn Ser Val Val Thr Ser Gly Gly Thr Lys Ile Ser Asn Val
    2570                    2575                    2580

gcg aat ggt gtc aat gca agt gat gca gta aac aaa ggt caa ttg          7794
Ala Asn Gly Val Asn Ala Ser Asp Ala Val Asn Lys Gly Gln Leu
    2585                    2590                    2595

gat agc tta agt aca ggt ctt acc aat aca ggc ttt ggt tta aaa          7839
Asp Ser Leu Ser Thr Gly Leu Thr Asn Thr Gly Phe Gly Leu Lys
    2600                    2605                    2610

gca gca gat ggc aac acc gtt aac aaa aaa tta ggc gaa gca gta          7884
Ala Ala Asp Gly Asn Thr Val Asn Lys Lys Leu Gly Glu Ala Val
    2615                    2620                    2625

gac gtt gtc ggt gct gac agc aac atc acc acg aaa gtt gca ggc          7929
Asp Val Val Gly Ala Asp Ser Asn Ile Thr Thr Lys Val Ala Gly
    2630                    2635                    2640

ggt cag gtt gcg att gag tta aat aaa aac ctc aac aac tta act          7974
Gly Gln Val Ala Ile Glu Leu Asn Lys Asn Leu Asn Asn Leu Thr
    2645                    2650                    2655

ggc att acc gtg aac gat gga acc aat ggc acc aat ggt tca act          8019

Gly Ile Thr Val Asn Asp Gly Thr Asn Gly Thr Asn Gly Ser Thr
    2660                2665                2670

gtg att ggt aaa gat ggt att tcg att aaa gat ggt tca ggc aat 8064
Val Ile Gly Lys Asp Gly Ile Ser Ile Lys Asp Gly Ser Gly Asn
    2675                2680                2685

acc att gca ggt gta gat aac aca gcg ttg acg gtt aaa gat agc 8109
Thr Ile Ala Gly Val Asp Asn Thr Ala Leu Thr Val Lys Asp Ser
    2690                2695                2700

agt ggc aac aca gaa acc agc att aac caa gcg atc aac acg tta 8154
Ser Gly Asn Thr Glu Thr Ser Ile Asn Gln Ala Ile Asn Thr Leu
    2705                2710                2715

aat gca gcg caa ggt gaa act gat aag ttt gca gtg aag tac gat 8199
Asn Ala Ala Gln Gly Glu Thr Asp Lys Phe Ala Val Lys Tyr Asp
    2720                2725                2730

aag aat gct gat ggc agt gtg aac tat aac aat gtc act tta gca 8244
Lys Asn Ala Asp Gly Ser Val Asn Tyr Asn Asn Val Thr Leu Ala
    2735                2740                2745

ggt aca aat ggc aca ata atc aga aat gtt aaa gcg ggt gct gtg 8289
Gly Thr Asn Gly Thr Ile Ile Arg Asn Val Lys Ala Gly Ala Val
    2750                2755                2760

acc tca aca tct act gat gcg atc aat ggt agc caa tta tac gat 8334
Thr Ser Thr Ser Thr Asp Ala Ile Asn Gly Ser Gln Leu Tyr Asp
    2765                2770                2775

att gca aac agc gtg aag aat gca att ggt ggt tca acc aca aga 8379
Ile Ala Asn Ser Val Lys Asn Ala Ile Gly Gly Ser Thr Thr Arg
    2780                2785                2790

gat gta acg act ggt gca atc aca acg acc aat att ggt ggt aca 8424
Asp Val Thr Thr Gly Ala Ile Thr Thr Thr Asn Ile Gly Gly Thr
    2795                2800                2805

ggt tca aac acg att gat ggt gca atc agc agt att aaa gat tca 8469
Gly Ser Asn Thr Ile Asp Gly Ala Ile Ser Ser Ile Lys Asp Ser
    2810                2815                2820

gcg act aaa gcg aaa acc acg ata agt gct ggg gat aat gtt gtc 8514
Ala Thr Lys Ala Lys Thr Thr Ile Ser Ala Gly Asp Asn Val Val
    2825                2830                2835

gtt aca tcg ggt acc aat gca gat ggc tca aca aac tat gaa gtt 8559
Val Thr Ser Gly Thr Asn Ala Asp Gly Ser Thr Asn Tyr Glu Val
    2840                2845                2850

gcg aca gcg aaa gac gtt aac ttt gac aaa gta act gta ggt aat 8604
Ala Thr Ala Lys Asp Val Asn Phe Asp Lys Val Thr Val Gly Asn
    2855                2860                2865

gtt gtt gtt gat aag gca aat gac acg atc caa ggt ttg agc aat 8649
Val Val Val Asp Lys Ala Asn Asp Thr Ile Gln Gly Leu Ser Asn
    2870                2875                2880

aaa gat cta aat tca act gat ttt gcg acc aaa ggt aga gct gcg 8694
Lys Asp Leu Asn Ser Thr Asp Phe Ala Thr Lys Gly Arg Ala Ala
    2885                2890                2895

act gaa gaa cag tta aaa gca gtg att acc agt aat atc acg gaa 8739
Thr Glu Glu Gln Leu Lys Ala Val Ile Thr Ser Asn Ile Thr Glu
    2900                2905                2910

gtt gtg gat ggt aat ggc aac aag gtg aat att att gac caa gtt 8784
Val Val Asp Gly Asn Gly Asn Lys Val Asn Ile Ile Asp Gln Val
    2915                2920                2925

gta aat acc aaa cct gac aat aag aac caa gat tca ttg ttc tta 8829
Val Asn Thr Lys Pro Asp Asn Lys Asn Gln Asp Ser Leu Phe Leu
    2930                2935                2940

acg tat gac aaa caa ggt caa gaa acc aca gat cgc cta acg att 8874
Thr Tyr Asp Lys Gln Gly Gln Glu Thr Thr Asp Arg Leu Thr Ile
    2945                2950                2955

ggt caa acg gta cag aag atg aat act gat ggt att aaa ttc ttc 8919
Gly Gln Thr Val Gln Lys Met Asn Thr Asp Gly Ile Lys Phe Phe
    2960                2965                2970

cat acc aat gcc gat aca tca aaa ggt gat ttg ggt aca aca aat 8964
His Thr Asn Ala Asp Thr Ser Lys Gly Asp Leu Gly Thr Thr Asn
    2975                2980                2985

gac tca agt gca ggt ggt tta aac tct aca gca att ggt gta aat 9009
Asp Ser Ser Ala Gly Gly Leu Asn Ser Thr Ala Ile Gly Val Asn
    2990                2995                3000

gcg att gtt gcg aat ggt gca gat agt tca gtt gct tta ggt cat 9054
Ala Ile Val Ala Asn Gly Ala Asp Ser Ser Val Ala Leu Gly His
    3005                3010                3015

aac acc aaa gtc aat ggt aaa caa tca att gca att ggt tct ggt 9099
Asn Thr Lys Val Asn Gly Lys Gln Ser Ile Ala Ile Gly Ser Gly
    3020                3025                3030

gca gaa gct tta ggc aat caa tcg atc agt att ggt aca ggc aat 9144
Ala Glu Ala Leu Gly Asn Gln Ser Ile Ser Ile Gly Thr Gly Asn
    3035                3040                3045

aaa gtc act ggt gat cat tcg ggt gcg att ggt gat cca act att 9189
Lys Val Thr Gly Asp His Ser Gly Ala Ile Gly Asp Pro Thr Ile
    3050                3055                3060

gta aat ggt gca aac agc tac tct gtg ggt aat aac aac caa gta 9234
Val Asn Gly Ala Asn Ser Tyr Ser Val Gly Asn Asn Asn Gln Val
    3065                3070                3075

ctt aca gat gac act ttc gta ctt gga aac aat gtc acc aaa act 9279
Leu Thr Asp Asp Thr Phe Val Leu Gly Asn Asn Val Thr Lys Thr
    3080                3085                3090

att gct ggt tca gta gta ttg ggt aac ggt tca gct gca acg aca 9324
Ile Ala Gly Ser Val Val Leu Gly Asn Gly Ser Ala Ala Thr Thr
    3095                3100                3105

ggt gct ggt gag gca ggc tat gcc tta tct gta gca aca aat gca 9369
Gly Ala Gly Glu Ala Gly Tyr Ala Leu Ser Val Ala Thr Asn Ala
    3110                3115                3120

gat aaa gcc gcg atc act aaa act acg tca agc act ggt gct gtt 9414
Asp Lys Ala Ala Ile Thr Lys Thr Thr Ser Ser Thr Gly Ala Val
    3125                3130                3135

gca gtt ggt gat gcg tcg agc ggt att tat cgt caa att acc ggt 9459
Ala Val Gly Asp Ala Ser Ser Gly Ile Tyr Arg Gln Ile Thr Gly
    3140                3145                3150

gtt gct gcg ggt agc gta gat tca gat gct gtg aac gtt gca cag 9504
Val Ala Ala Gly Ser Val Asp Ser Asp Ala Val Asn Val Ala Gln
    3155                3160                3165

tta aaa gcg gtg ggt aac caa gtt gta acg act caa act aca ttg 9549
Leu Lys Ala Val Gly Asn Gln Val Val Thr Thr Gln Thr Thr Leu
    3170                3175                3180

gtg aac agt ttg ggt ggt aac gct aaa gta aat gca gac ggt acg 9594
Val Asn Ser Leu Gly Gly Asn Ala Lys Val Asn Ala Asp Gly Thr
    3185                3190                3195

att aca gga cca act tat aat gtt gct caa ggt aat cag acc aat 9639
Ile Thr Gly Pro Thr Tyr Asn Val Ala Gln Gly Asn Gln Thr Asn
    3200                3205                3210

gtt ggt gat gca tta act gcg ctt gat aac gca att aat act gcg 9684
Val Gly Asp Ala Leu Thr Ala Leu Asp Asn Ala Ile Asn Thr Ala
    3215                3220                3225

gca aca aca tct aaa tcg act gtt tct aat ggt cag aat att gtt 9729
Ala Thr Thr Ser Lys Ser Thr Val Ser Asn Gly Gln Asn Ile Val
    3230                3235                3240

gtc agc aag agc aaa aat gca gat ggt tca gac aac tat gaa gta 9774
Val Ser Lys Ser Lys Asn Ala Asp Gly Ser Asp Asn Tyr Glu Val
    3245                3250                3255

tca aca gca aaa gac ttg aca gtt gat tct gtc aaa gcg ggt gat 9819
Ser Thr Ala Lys Asp Leu Thr Val Asp Ser Val Lys Ala Gly Asp
    3260                3265                3270

acg gtt ctg aat aat gca ggt att aca att ggc aat aac gca gtt 9864
Thr Val Leu Asn Asn Ala Gly Ile Thr Ile Gly Asn Asn Ala Val
    3275                3280                3285

gta ttg aac aac act gga tta acc att agt ggt gga cca agt gtt 9909
Val Leu Asn Asn Thr Gly Leu Thr Ile Ser Gly Gly Pro Ser Val
    3290                3295                3300

acc ttg gca ggc atc gat gca ggc aat aaa acc att caa aat gtt 9954
Thr Leu Ala Gly Ile Asp Ala Gly Asn Lys Thr Ile Gln Asn Val
    3305                3310                3315

gcg aat gca gta aat gca aca gat gca gtc aac aaa ggg caa ttg 9999
Ala Asn Ala Val Asn Ala Thr Asp Ala Val Asn Lys Gly Gln Leu
    3320                3325                3330

gac agc gca att aac aat gtg aat aac aat gta aat gag ctt gcc 10044
Asp Ser Ala Ile Asn Asn Val Asn Asn Asn Val Asn Glu Leu Ala
    3335                3340                3345

aac aac gct gtt aaa tat gac gat gca tca aaa gat aag atc aca 10089
Asn Asn Ala Val Lys Tyr Asp Asp Ala Ser Lys Asp Lys Ile Thr
    3350                3355                3360

ctt ggt ggt ggg gca act ggt aca aca atc acc aat gtg aaa gat 10134
Leu Gly Gly Gly Ala Thr Gly Thr Thr Ile Thr Asn Val Lys Asp

3365                    3370                    3375

ggt act gtt gcg caa ggt tct aaa gat gct gtg aat ggc ggt caa     10179
Gly Thr Val Ala Gln Gly Ser Lys Asp Ala Val Asn Gly Gly Gln
    3380                    3385                    3390

ttg tgg aat gtt caa caa caa gtt gat cag aac aca act gat att     10224
Leu Trp Asn Val Gln Gln Gln Val Asp Gln Asn Thr Thr Asp Ile
    3395                    3400                    3405

agc aat atc aaa aat gat att aac aac ggt act gtt ggt ttg gtt     10269
Ser Asn Ile Lys Asn Asp Ile Asn Asn Gly Thr Val Gly Leu Val
    3410                    3415                    3420

caa caa gca ggt aaa gat gca cca gtg acg gtt gca aaa gat act     10314
Gln Gln Ala Gly Lys Asp Ala Pro Val Thr Val Ala Lys Asp Thr
    3425                    3430                    3435

ggc ggt aca acg gtg aat gtc gct gga aca gat ggc aac cga gta     10359
Gly Gly Thr Thr Val Asn Val Ala Gly Thr Asp Gly Asn Arg Val
    3440                    3445                    3450

gtg aca ggt gtt aag gaa ggt gca gtg aat gca aca tct aaa gat     10404
Val Thr Gly Val Lys Glu Gly Ala Val Asn Ala Thr Ser Lys Asp
    3455                    3460                    3465

gct gtc aat ggt agt caa ttg aat aca acc aac caa gcg gta gtc     10449
Ala Val Asn Gly Ser Gln Leu Asn Thr Thr Asn Gln Ala Val Val
    3470                    3475                    3480

aat tat ctt ggt ggt ggg gca ggt tat gac aac att aca ggt agc     10494
Asn Tyr Leu Gly Gly Gly Ala Gly Tyr Asp Asn Ile Thr Gly Ser
    3485                    3490                    3495

ttc aca gcg cca agt tat acg gta ggt gac tcg aaa tac aac aat     10539
Phe Thr Ala Pro Ser Tyr Thr Val Gly Asp Ser Lys Tyr Asn Asn
    3500                    3505                    3510

gtt ggt ggc gca att gat gca ttg aat caa gca gat caa gca ttg     10584
Val Gly Gly Ala Ile Asp Ala Leu Asn Gln Ala Asp Gln Ala Leu
    3515                    3520                    3525

aat agc aaa att gac aat gtc agt aac aag ttg gat aac gca ttc     10629
Asn Ser Lys Ile Asp Asn Val Ser Asn Lys Leu Asp Asn Ala Phe
    3530                    3535                    3540

cgt att acc aac aac cgt att gat gat gta gag aaa aaa gcc aat     10674
Arg Ile Thr Asn Asn Arg Ile Asp Asp Val Glu Lys Lys Ala Asn
    3545                    3550                    3555

gct ggt att gcc gct gcg atg gct ctg gaa tca gca cca tat gtc     10719
Ala Gly Ile Ala Ala Ala Met Ala Leu Glu Ser Ala Pro Tyr Val
    3560                    3565                    3570

cca ggt aaa tat acc tat gca gca ggc gca gct tac cac ggt ggt     10764
Pro Gly Lys Tyr Thr Tyr Ala Ala Gly Ala Ala Tyr His Gly Gly
    3575                    3580                    3585

gaa aat gcg gta ggt gtg act tta cgt aaa act gca gac aat ggt     10809
Glu Asn Ala Val Gly Val Thr Leu Arg Lys Thr Ala Asp Asn Gly
    3590                    3595                    3600

cgt tgg tcg att aca ggc ggt gta gct gca gcg tct caa ggc gat     10854

Arg Trp Ser Ile Thr Gly Gly Val Ala Ala Ala Ser Gln Gly Asp
    3605                3610                3615

gca agt gtt cgt atc ggt atc agc ggt gtg att gac taa 10893
Ala Ser Val Arg Ile Gly Ile Ser Gly Val Ile Asp
    3620                3625                3630

<210> 4
<211> 3630
<212> PRT
<213> Acinetobacter sp

<400> 4

Met Asn Lys Ile Tyr Lys Val Ile Trp Asn Ala Thr Leu Leu Ala Trp
1               5                   10                  15

Val Ala Val Ser Glu Leu Ala Lys Gly Lys Thr Lys Ser Thr Thr Ser
            20                  25                  30

Lys Ser Lys Ala Lys Ser Leu Ser Ser Ser Val Ile Val Gly Gly Ile
        35                  40                  45

Ile Leu Thr Thr Pro Leu Ser Leu Ile Ala Ala Thr Val Gln Val Gly
    50                  55                  60

Gly Gly Thr Asn Ser Gly Thr Thr Ala Thr Ala Ser Thr Asn Cys Ala
65                  70                  75                  80

Asp Leu Tyr Asn Tyr Gln Asn Pro Glu Asn Ser Gly Ser Gly Ala Ala
                85                  90                  95

Gly Asn Tyr Asn Ala Gly Asn Pro Ser Val Cys Ser Ile Ala Ile Gly
            100                 105                 110

Glu Asn Ala Gln Gly Gly Thr Ser Gly Thr Gly Gly Ser Pro Gly Ile
        115                 120                 125

Ala Ile Gly Gly Asn Ser Lys Ala Thr Gly Gly Leu Ser Val Ala Ile
    130                 135                 140

Gly Gly Tyr Ala Gln Ala Thr Asn Val Gly Ser Ile Ala Leu Gly Thr
145                 150                 155                 160

Ala Ala Leu Ser Ser Gly Phe Asn Ser Leu Ala Ile Ser Arg Gln Ala
                165                 170                 175

Ala Ala Thr Asn Asn Tyr Ser Ile Ala Ile Gly Thr Thr Ser Val Ser
            180                 185                 190

Lys Gly Val Gly Ser Ile Ala Met Gly His Ser Thr Asn Ala Ser Gly
        195                 200                 205

Asp Gln Ser Ile Ala Ile Gly Ser Ser Asp Ala Val Asn Ser Ala Thr
    210                 215                 220

Ala Thr Thr Thr Tyr Asp Gly Thr Thr Asn Thr Gln Ala Ser Gly Ser
225                 230                 235                 240

Lys Ser Ile Ala Ile Gly Ala Ser Ala Lys Ala Ser Thr Asn Asn Ser
                245                 250                 255

Ile Ala Leu Gly Ala Gly Ser Val Thr Ser Ala Gln Ser Gly Asn Ser
            260                 265                 270

Tyr Leu Thr Gly Val Gly Ala Ser Ala Thr Asn Gly Val Val Ser Val
        275                 280                 285

Gly Thr Ser Thr Ala Thr Arg Arg Ile Gln Asn Val Ala Asp Gly Ser
    290                 295                 300

Ala Ala Ser Asp Ala Val Thr Val Ala Gln Leu Asp Lys Ala Tyr Asp
305                 310                 315                 320

Asp Thr Asn Gly Arg Leu Ala Ala Ala Leu Gly Thr Gly Ser Gly Ala
                325                 330                 335

Ala Tyr Asn Ala Ala Asn Asn Thr Tyr Thr Ala Pro Thr Asn Ile Gly
            340                 345                 350

Gly Thr Gly Lys Asn Thr Ile Asp Asp Ala Ile Lys Ala Thr Gln Arg
        355                 360                 365

Ser Val Val Ala Gly Ser Asn Ile Val Val Thr Pro Thr Thr Ala Ser
    370                 375                 380

Asp Gly Ser Ile Ser Tyr Ser Val Ala Thr Ser Ala Thr Pro Thr Phe
385                 390                 395                 400

Thr Ser Ile Thr Val Asn Asn Ala Pro Thr Ala Gly Thr Asp Ala Thr
                405                 410                 415

Asn Lys Thr Tyr Val Asp Ser Lys Ala Ala Ala Ser Arg Thr Glu Val
            420                 425                 430

Ala Ala Gly Ser Asn Val Ser Gly Val Val Lys Thr Thr Gly Ala Asn
        435                 440                 445

Gly Gln Asp Val Tyr Thr Val Asn Ala Asn Gly Thr Thr Ala Ser Ala
    450                 455                 460

Gly Ser Ser Ala Val Thr Val Thr Pro Gly Thr Lys Asp Ala Asn Asn
465                 470                 475                 480

Val Thr Asp Tyr Lys Val Asp Leu Ser Ala Thr Thr Lys Thr Asp Ile
                485                 490                 495

Gln Lys Gly Val Asp Ala Lys Asn Ala Val Asp Thr Ala Gly Leu Lys
            500                 505                 510

Phe Lys Gly Asp Thr Ala Thr Thr Ser Asn Thr Lys Lys Leu Gly Asp
        515                 520                 525

Thr Val Ser Ile Thr Gly Asp Thr Asn Ile Ser Thr Val Ala Thr Thr
    530                 535                 540

Asp Gly Val Gln Val Lys Leu Asn Pro Asn Leu Asp Leu Gly Ala Thr
545                 550                 555                 560

Gly Ser Val Lys Thr Gly Asn Thr Thr Ile Asn Asn Ala Gly Val Thr
                565                 570                 575

Ala Asp Gln Val Thr Val Gly Gly Val Val Ile Asn Asn Thr Ser Gly
            580                 585                 590

Ile Asn Ala Gly Gly Lys Ala Ile Thr Asn Val Ala Ala Pro Thr Asn
        595                 600                 605

Asn Thr Asp Ala Ala Asn Lys Lys Tyr Val Asp Asp Ala Gly Thr Ala
    610                 615                 620

Leu Thr Asn Leu Gly Phe Gly Leu Lys Ala Gln Asp Gly Thr Thr Val
625                 630                 635                 640

Asn Lys Lys Leu Gly Glu Ala Val Asp Ile Val Gly Ser Asn Ser Asn
                645                 650                 655

Ile Ser Thr Lys Val Asn Ala Gly Lys Val Glu Val Ala Leu Ser Asn
            660                 665                 670

Thr Leu Asp Leu Gly Thr Thr Gly Ser Val Thr Thr Gly Ser Thr Val
        675                 680                 685

Ile Asn Asn Ala Gly Val Thr Ala Thr Gln Val Thr Ala Asn Lys Val
    690                 695                 700

Thr Ile Asn Asn Ala Pro Thr Ala Gly Thr Asp Ala Thr Asn Lys Thr
705                 710                 715                 720

Tyr Val Asp Ser Lys Ala Ala Ala Ser Arg Thr Glu Val Ala Ala Gly
                725                 730                 735

Ser Asn Val Ser Gly Val Val Lys Thr Thr Gly Ala Asn Gly Gln Asp
            740                 745                 750

Ile Tyr Ala Val Asn Ala Asn Gly Thr Thr Ala Ser Ala Gly Ser Ser
        755                 760                 765

Ala Val Thr Val Thr Pro Gly Thr Lys Asp Ala Asn Asn Val Thr Asp
    770                 775                 780

Tyr Lys Val Asp Leu Ser Ala Thr Thr Lys Thr Asp Ile Gln Lys Gly
785                 790                 795                 800

Val Asp Ala Lys Asn Ala Val Asp Thr Ala Gly Leu Lys Phe Lys Gly
                805                 810                 815

Asp Thr Ala Thr Thr Ser Asn Thr Lys Lys Leu Gly Asp Thr Val Ser
            820                 825                 830

Ile Thr Gly Asp Thr Asn Ile Ser Thr Val Ala Thr Thr Asp Gly Val
        835                 840                 845

Gln Val Lys Leu Asn Pro Asn Leu Asp Leu Gly Ala Thr Gly Ser Val
    850                 855                 860

Lys Thr Gly Asn Thr Thr Ile Asn Asn Ala Gly Val Thr Ala Asp Gln
865                 870                 875                 880

Val Thr Val Gly Gly Val Val Ile Asn Asn Thr Ser Gly Ile Asn Ala
                885                 890                 895

Gly Gly Lys Ala Ile Thr Asn Val Ala Ala Pro Thr Asn Asn Thr Asp
            900                 905                 910

Ala Ala Asn Lys Lys Tyr Val Asp Asp Ala Gly Thr Ala Leu Thr Asn
        915                 920                 925

Leu Gly Phe Gly Leu Lys Ala Gln Asp Gly Thr Thr Val Asn Lys Lys
    930                 935                 940

Leu Gly Glu Ala Val Asp Ile Val Gly Ser Asn Ser Asn Ile Ser Thr

945                 950                 955                 960

Lys Val Asn Ala Gly Lys Val Glu Val Ala Leu Ser Asn Thr Leu Asp
                965                 970                 975

Leu Gly Thr Thr Gly Ser Val Thr Thr Gly Ser Thr Val Ile Asn Asn
            980                 985                 990

Ala Gly Val Thr Ala Thr Gln Val  Thr Ala Asn Lys Val  Thr Val Asn
        995                 1000                1005

Asn Ala  Pro Thr Ala Gly Thr  Asp Ala Thr Asn Lys  Thr Tyr Val
    1010                 1015                 1020

Asp Ser  Lys Ala Ala Ala Ser  Arg Thr Glu Val Ala  Ala Gly Ser
    1025                 1030                 1035

Asn Val  Ser Gly Val Val Lys  Thr Thr Gly Ala Asn  Gly Gln Asp
    1040                 1045                 1050

Val Tyr  Thr Val Asn Ala Asn  Gly Thr Thr Ala Ser  Ala Gly Ser
    1055                 1060                 1065

Ser Ala  Val Thr Val Thr Pro  Gly Thr Lys Asp Ala  Asn Asn Val
    1070                 1075                 1080

Thr Asp  Tyr Lys Val Asp Leu  Ser Ala Thr Thr Lys  Thr Asp Ile
    1085                 1090                 1095

Gln Lys  Gly Val Asp Ala Lys  Asn Ala Val Asp Thr  Ala Gly Leu
    1100                 1105                 1110

Lys Phe  Lys Gly Asp Thr Ala  Thr Thr Ser Asn Thr  Lys Lys Leu
    1115                 1120                 1125

Gly Asp  Thr Val Ser Ile Thr  Gly Asp Thr Asn Ile  Ser Thr Val
    1130                 1135                 1140

Ala Thr  Thr Asp Gly Val Gln  Val Lys Leu Asn Pro  Asn Leu Asp
    1145                 1150                 1155

Leu Gly  Ala Thr Gly Ser Val  Lys Thr Gly Asn Thr  Thr Ile Asn
    1160                 1165                 1170

Asn Ala  Gly Val Thr Ala Asp  Gln Val Thr Val Gly  Gly Val Val
    1175                 1180                 1185

Ile Asn Asn Thr Ser Gly Ile Asn Ala Gly Gly Lys Ala Ile Thr
    1190                1195                1200

Asn Val Ala Ala Pro Thr Asn Asn Thr Asp Ala Ala Asn Lys Lys
    1205                1210                1215

Tyr Val Asp Asp Ala Gly Thr Ala Leu Thr Asn Leu Gly Phe Gly
    1220                1225                1230

Leu Lys Ala Gln Asp Gly Thr Thr Val Asn Lys Lys Leu Gly Glu
    1235                1240                1245

Ala Val Glu Val Val Gly Ala Asp Ser Asn Ile Thr Thr Lys Val
    1250                1255                1260

Ala Gly Gly Gln Val Ala Ile Glu Leu Asn Lys Asn Leu Asn Asn
    1265                1270                1275

Leu Thr Gly Ile Thr Val Asn Asp Gly Thr Asn Gly Thr Asn Gly
    1280                1285                1290

Ser Thr Val Ile Gly Lys Asp Gly Ile Ser Val Lys Asp Gly Ser
    1295                1300                1305

Gly Asn Thr Ile Ala Gly Val Asp Asn Thr Ala Leu Thr Val Lys
    1310                1315                1320

Asp Gly Ser Gly Asn Thr Glu Thr Ser Ile Asn Gln Ala Ile Asn
    1325                1330                1335

Thr Leu Asn Ala Ala Gln Gly Glu Thr Asp Lys Phe Ala Val Lys
    1340                1345                1350

Tyr Asp Lys Asn Ala Asp Gly Ser Val Asn Tyr Asn Asn Ile Thr
    1355                1360                1365

Leu Ala Gly Thr Thr Ala Ser Ser Thr Gln Asp Ala Thr Thr Gly
    1370                1375                1380

Lys Ile Thr Thr Thr Gly Gly Thr Ser Leu Asn Asn Val Ala Ser
    1385                1390                1395

Ala Gly Asp Tyr Lys Asp Val Ala Asn Ala Ser Lys Gly Val Asn
    1400                1405                1410

Ala Gly Asp Leu Asn Asn Ala Val Val Asp Ala Thr Asn Ala Ala
    1415                1420                1425

Thr Ser Lys Gly Phe Ala Leu Gln Ala Ala Asp Gly Ala Lys Val
    1430                1435                1440

Gln Lys Asn Leu Gly Glu Ala Val Glu Val Val Gly Ala Asp Ser
    1445                1450                1455

Asn Ile Thr Thr Lys Val Ala Gly Gly Gln Val Ala Ile Glu Leu
    1460                1465                1470

Asn Lys Asn Leu Asn Asn Leu Thr Gly Ile Thr Val Asn Asp Gly
    1475                1480                1485

Thr Asn Gly Thr Asn Gly Ser Thr Val Ile Gly Lys Asp Gly Ile
    1490                1495                1500

Ser Val Lys Asp Gly Ser Gly Asn Thr Ile Ala Gly Val Asp Asn
    1505                1510                1515

Thr Ala Leu Thr Val Lys Asp Gly Ser Gly Asn Thr Glu Thr Ser
    1520                1525                1530

Ile Asn Gln Ala Ile Asn Thr Leu Asn Ala Ala Gln Gly Glu Thr
    1535                1540                1545

Asp Lys Phe Ala Val Lys Tyr Asp Lys Asn Thr Asp Gly Ser Thr
    1550                1555                1560

Asn Tyr Asn Ser Ile Thr Ala Gly Asn Gly Asn Gly Thr Ala Ala
    1565                1570                1575

Thr Ile Gly Thr Asp Thr Ala Gly Asn Ser Val Val Thr Ser Gly
    1580                1585                1590

Gly Thr Lys Ile Ser Asn Val Ala Asn Gly Val Asn Ala Ser Asp
    1595                1600                1605

Ala Val Asn Lys Gly Gln Leu Asp Ser Leu Ser Thr Gly Leu Thr
    1610                1615                1620

Asn Thr Gly Phe Gly Leu Lys Ala Ala Asp Gly Asn Thr Val Asn
    1625                1630                1635

Lys Lys Leu Gly Glu Ala Val Asp Val Val Gly Ala Asp Ser Asn
    1640                1645                1650

Ile Thr Thr Lys Val Ala Gly Gly Gln Val Ala Ile Glu Leu Asn
    1655                1660                1665

Lys Asn Leu Asn Asn Leu Thr Gly Ile Thr Val Asn Asp Gly Thr
    1670                1675                1680

Asn Gly Thr Asn Gly Ser Thr Val Ile Gly Lys Asp Gly Ile Ser
    1685                1690                1695

Ile Lys Asp Gly Ser Gly Asn Thr Ile Ala Gly Val Asp Asn Thr
    1700                1705                1710

Ala Leu Thr Val Lys Asp Gly Ser Gly Asn Thr Glu Thr Ser Ile
    1715                1720                1725

Asn Gln Ala Ile Asn Thr Leu Asn Ala Ala Gln Gly Glu Thr Asp
    1730                1735                1740

Lys Phe Ala Val Lys Tyr Asp Lys Asn Ala Asp Gly Ser Ala Asn
    1745                1750                1755

Tyr Asn Asn Ile Thr Leu Ala Gly Thr Thr Ala Ser Ser Thr Gln
    1760                1765                1770

Asp Ala Thr Thr Gly Lys Ile Thr Thr Thr Gly Gly Thr Ser Leu
    1775                1780                1785

Asn Asn Val Ala Ser Ala Gly Asp Tyr Lys Asp Val Ala Asn Ala
    1790                1795                1800

Ser Lys Gly Val Asn Ala Gly Asp Leu Asn Asn Ala Val Val Asp
    1805                1810                1815

Ala Thr Asn Ala Ala Thr Ser Lys Gly Phe Ala Leu Gln Ala Ala
    1820                1825                1830

Asp Gly Ala Lys Val Gln Lys Asn Leu Gly Glu Ala Val Glu Val
    1835                1840                1845

Val Gly Ala Asp Ser Asn Ile Thr Thr Lys Val Val Gly Gly Gln
    1850                1855                1860

Val Ala Ile Glu Leu Asn Lys Asn Leu Asn Asn Leu Thr Gly Ile
    1865                1870                1875

Thr Val Asn Asp Gly Thr Asn Gly Thr Asn Gly Ser Thr Val Ile
    1880                1885                1890

Gly Lys Asp Gly Ile Ser Val Lys Asp Gly Ser Gly Asn Thr Ile

1895                1900                1905

Ala Gly Val Asp Asn Thr Ala Leu Thr Val Lys Asp Gly Ser Gly
    1910                1915                1920

Asn Thr Glu Thr Ser Ile Asn Gln Ala Ile Asn Thr Leu Asn Ala
    1925                1930                1935

Ala Gln Gly Glu Thr Asp Lys Phe Ala Val Lys Tyr Asp Lys Asn
    1940                1945                1950

Ala Asp Gly Ser Val Asn Tyr Asn Asn Ile Thr Leu Ala Gly Thr
    1955                1960                1965

Thr Ala Ser Ser Thr Gln Asp Ala Thr Thr Gly Lys Ile Thr Thr
    1970                1975                1980

Thr Gly Gly Thr Ser Leu Asn Asn Val Ala Ser Ala Gly Asp Tyr
    1985                1990                1995

Lys Asp Val Ala Asn Ala Ser Lys Gly Val Asn Ala Gly Asp Leu
    2000                2005                2010

Asn Asn Ala Val Val Asp Ala Thr Asn Ala Ala Thr Ser Lys Gly
    2015                2020                2025

Phe Ala Leu Gln Ala Ala Asp Gly Ala Lys Val Gln Lys Asn Leu
    2030                2035                2040

Gly Glu Ala Val Glu Val Val Gly Ala Asp Ser Asn Ile Thr Thr
    2045                2050                2055

Lys Val Ala Gly Gly Gln Val Ala Ile Glu Leu Asn Lys Asn Leu
    2060                2065                2070

Asn Asn Leu Thr Gly Ile Thr Val Asn Asp Gly Thr Asn Gly Thr
    2075                2080                2085

Asn Gly Ser Thr Val Ile Gly Lys Asp Gly Ile Ser Val Lys Asp
    2090                2095                2100

Gly Ser Gly Asn Thr Ile Ala Gly Val Asp Asn Thr Ala Leu Thr
    2105                2110                2115

Val Lys Asp Gly Ser Gly Asn Thr Glu Thr Ser Ile Asn Gln Ala
    2120                2125                2130

Ile Asn Thr Leu Asn Ala Ala Gln Gly Glu Thr Asp Lys Phe Ala
    2135                2140                2145

Val Lys Tyr Asp Lys Asn Ala Asp Gly Ser Val Asn Tyr Asn Asn
    2150                2155                2160

Ile Thr Leu Ala Gly Thr Thr Ala Ser Ser Thr Gln Asp Ala Thr
    2165                2170                2175

Thr Gly Lys Ile Thr Thr Thr Gly Gly Thr Ser Leu Asn Asn Val
    2180                2185                2190

Ala Ser Ala Gly Asp Tyr Lys Asp Val Ala Asn Ala Ser Lys Gly
    2195                2200                2205

Val Asn Ala Gly Asp Leu Asn Asn Ala Val Val Asp Ala Thr Asn
    2210                2215                2220

Ala Ala Thr Ser Lys Gly Phe Ala Leu Gln Ala Ala Asp Gly Ala
    2225                2230                2235

Lys Val Gln Lys Asn Leu Gly Glu Ala Val Glu Val Val Gly Ala
    2240                2245                2250

Asp Ser Asn Ile Thr Thr Lys Val Ala Gly Gly Gln Val Ala Ile
    2255                2260                2265

Glu Leu Asn Lys Asn Leu Asn Asn Leu Thr Gly Ile Thr Val Asn
    2270                2275                2280

Asp Gly Thr Asn Gly Thr Asn Gly Ser Thr Val Ile Gly Lys Asp
    2285                2290                2295

Gly Ile Ser Val Lys Asp Gly Ser Gly Asn Thr Ile Ala Gly Val
    2300                2305                2310

Asp Asn Thr Ala Leu Thr Val Lys Asp Gly Ser Gly Asn Thr Glu
    2315                2320                2325

Thr Ser Ile Asn Gln Ala Ile Asn Thr Leu Asn Ala Ala Gln Gly
    2330                2335                2340

Glu Thr Asp Lys Phe Ala Val Lys Tyr Asp Lys Asn Ala Asp Gly
    2345                2350                2355

Ser Ala Asn Tyr Asn Asn Val Thr Leu Ala Gly Thr Asn Gly Thr
    2360                2365                2370

Ile Ile Ser Asn Val Lys Ala Gly Ala Val Thr Ser Thr Ser Thr
    2375                2380                2385

Asp Ala Ile Asn Gly Ser Gln Leu Tyr Gly Val Ala Asn Ser Val
    2390                2395                2400

Lys Asn Ala Ile Gly Gly Ser Thr Thr Ile Asp Ala Thr Thr Gly
    2405                2410                2415

Ala Ile Thr Thr Thr Asn Ile Gly Gly Thr Gly Ser Asn Thr Ile
    2420                2425                2430

Asp Gly Ala Ile Ser Ser Ile Lys Asp Ser Ala Thr Lys Ala Lys
    2435                2440                2445

Thr Thr Val Ser Ala Gly Asp Asn Val Val Val Thr Ser Gly Thr
    2450                2455                2460

Asn Ala Asp Gly Ser Thr Asn Tyr Glu Val Ala Thr Ala Lys Asp
    2465                2470                2475

Val Asn Phe Asp Lys Val Thr Val Gly Ser Val Val Val Asp Lys
    2480                2485                2490

Ser Ser Asn Thr Ile Lys Gly Leu Ser Asn Thr Thr Trp Asn Gly
    2495                2500                2505

Thr Ala Val Ser Gly Gln Ala Ala Thr Glu Asp Gln Leu Lys Thr
    2510                2515                2520

Val Ser Asp Ala Gln Gly Glu Thr Asp Lys Phe Ala Val Lys Tyr
    2525                2530                2535

Asp Lys Asn Ala Asp Gly Ser Ala Asn Tyr Asn Ser Ile Thr Ala
    2540                2545                2550

Gly Asn Gly Asn Gly Thr Ala Ala Thr Ile Gly Thr Asp Thr Ala
    2555                2560                2565

Gly Asn Ser Val Val Thr Ser Gly Gly Thr Lys Ile Ser Asn Val
    2570                2575                2580

Ala Asn Gly Val Asn Ala Ser Asp Ala Val Asn Lys Gly Gln Leu
    2585                2590                2595

Asp Ser Leu Ser Thr Gly Leu Thr Asn Thr Gly Phe Gly Leu Lys
    2600                2605                2610

Ala Ala Asp Gly Asn Thr Val Asn Lys Lys Leu Gly Glu Ala Val
    2615                2620                2625

Asp Val Val Gly Ala Asp Ser Asn Ile Thr Thr Lys Val Ala Gly
    2630                2635                2640

Gly Gln Val Ala Ile Glu Leu Asn Lys Asn Leu Asn Asn Leu Thr
    2645                2650                2655

Gly Ile Thr Val Asn Asp Gly Thr Asn Gly Thr Asn Gly Ser Thr
    2660                2665                2670

Val Ile Gly Lys Asp Gly Ile Ser Ile Lys Asp Gly Ser Gly Asn
    2675                2680                2685

Thr Ile Ala Gly Val Asp Asn Thr Ala Leu Thr Val Lys Asp Ser
    2690                2695                2700

Ser Gly Asn Thr Glu Thr Ser Ile Asn Gln Ala Ile Asn Thr Leu
    2705                2710                2715

Asn Ala Ala Gln Gly Glu Thr Asp Lys Phe Ala Val Lys Tyr Asp
    2720                2725                2730

Lys Asn Ala Asp Gly Ser Val Asn Tyr Asn Asn Val Thr Leu Ala
    2735                2740                2745

Gly Thr Asn Gly Thr Ile Ile Arg Asn Val Lys Ala Gly Ala Val
    2750                2755                2760

Thr Ser Thr Ser Thr Asp Ala Ile Asn Gly Ser Gln Leu Tyr Asp
    2765                2770                2775

Ile Ala Asn Ser Val Lys Asn Ala Ile Gly Gly Ser Thr Thr Arg
    2780                2785                2790

Asp Val Thr Thr Gly Ala Ile Thr Thr Thr Asn Ile Gly Gly Thr
    2795                2800                2805

Gly Ser Asn Thr Ile Asp Gly Ala Ile Ser Ser Ile Lys Asp Ser
    2810                2815                2820

Ala Thr Lys Ala Lys Thr Thr Ile Ser Ala Gly Asp Asn Val Val
    2825                2830                2835

Val Thr Ser Gly Thr Asn Ala Asp Gly Ser Thr Asn Tyr Glu Val

2840                2845                2850

Ala Thr Ala Lys Asp Val Asn Phe Asp Lys Val Thr Val Gly Asn
    2855                2860                2865

Val Val Val Asp Lys Ala Asn Asp Thr Ile Gln Gly Leu Ser Asn
    2870                2875                2880

Lys Asp Leu Asn Ser Thr Asp Phe Ala Thr Lys Gly Arg Ala Ala
    2885                2890                2895

Thr Glu Glu Gln Leu Lys Ala Val Ile Thr Ser Asn Ile Thr Glu
    2900                2905                2910

Val Val Asp Gly Asn Gly Asn Lys Val Asn Ile Ile Asp Gln Val
    2915                2920                2925

Val Asn Thr Lys Pro Asp Asn Lys Asn Gln Asp Ser Leu Phe Leu
    2930                2935                2940

Thr Tyr Asp Lys Gln Gly Gln Glu Thr Thr Asp Arg Leu Thr Ile
    2945                2950                2955

Gly Gln Thr Val Gln Lys Met Asn Thr Asp Gly Ile Lys Phe Phe
    2960                2965                2970

His Thr Asn Ala Asp Thr Ser Lys Gly Asp Leu Gly Thr Thr Asn
    2975                2980                2985

Asp Ser Ser Ala Gly Gly Leu Asn Ser Thr Ala Ile Gly Val Asn
    2990                2995                3000

Ala Ile Val Ala Asn Gly Ala Asp Ser Ser Val Ala Leu Gly His
    3005                3010                3015

Asn Thr Lys Val Asn Gly Lys Gln Ser Ile Ala Ile Gly Ser Gly
    3020                3025                3030

Ala Glu Ala Leu Gly Asn Gln Ser Ile Ser Ile Gly Thr Gly Asn
    3035                3040                3045

Lys Val Thr Gly Asp His Ser Gly Ala Ile Gly Asp Pro Thr Ile
    3050                3055                3060

Val Asn Gly Ala Asn Ser Tyr Ser Val Gly Asn Asn Asn Gln Val
    3065                3070                3075

Leu Thr Asp Asp Thr Phe Val Leu Gly Asn Asn Val Thr Lys Thr
    3080                3085                3090

Ile Ala Gly Ser Val Val Leu Gly Asn Gly Ser Ala Ala Thr Thr
    3095                3100                3105

Gly Ala Gly Glu Ala Gly Tyr Ala Leu Ser Val Ala Thr Asn Ala
    3110                3115                3120

Asp Lys Ala Ala Ile Thr Lys Thr Thr Ser Ser Thr Gly Ala Val
    3125                3130                3135

Ala Val Gly Asp Ala Ser Ser Gly Ile Tyr Arg Gln Ile Thr Gly
    3140                3145                3150

Val Ala Ala Gly Ser Val Asp Ser Asp Ala Val Asn Val Ala Gln
    3155                3160                3165

Leu Lys Ala Val Gly Asn Gln Val Val Thr Thr Gln Thr Thr Leu
    3170                3175                3180

Val Asn Ser Leu Gly Gly Asn Ala Lys Val Asn Ala Asp Gly Thr
    3185                3190                3195

Ile Thr Gly Pro Thr Tyr Asn Val Ala Gln Gly Asn Gln Thr Asn
    3200                3205                3210

Val Gly Asp Ala Leu Thr Ala Leu Asp Asn Ala Ile Asn Thr Ala
    3215                3220                3225

Ala Thr Thr Ser Lys Ser Thr Val Ser Asn Gly Gln Asn Ile Val
    3230                3235                3240

Val Ser Lys Ser Lys Asn Ala Asp Gly Ser Asp Asn Tyr Glu Val
    3245                3250                3255

Ser Thr Ala Lys Asp Leu Thr Val Asp Ser Val Lys Ala Gly Asp
    3260                3265                3270

Thr Val Leu Asn Asn Ala Gly Ile Thr Ile Gly Asn Asn Ala Val
    3275                3280                3285

Val Leu Asn Asn Thr Gly Leu Thr Ile Ser Gly Gly Pro Ser Val
    3290                3295                3300

Thr Leu Ala Gly Ile Asp Ala Gly Asn Lys Thr Ile Gln Asn Val
    3305                3310                3315

Ala Asn Ala Val Asn Ala Thr Asp Ala Val Asn Lys Gly Gln Leu
    3320                3325                3330

Asp Ser Ala Ile Asn Asn Val Asn Asn Asn Val Asn Glu Leu Ala
    3335                3340                3345

Asn Asn Ala Val Lys Tyr Asp Asp Ala Ser Lys Asp Lys Ile Thr
    3350                3355                3360

Leu Gly Gly Gly Ala Thr Gly Thr Thr Ile Thr Asn Val Lys Asp
    3365                3370                3375

Gly Thr Val Ala Gln Gly Ser Lys Asp Ala Val Asn Gly Gly Gln
    3380                3385                3390

Leu Trp Asn Val Gln Gln Gln Val Asp Gln Asn Thr Thr Asp Ile
    3395                3400                3405

Ser Asn Ile Lys Asn Asp Ile Asn Asn Gly Thr Val Gly Leu Val
    3410                3415                3420

Gln Gln Ala Gly Lys Asp Ala Pro Val Thr Val Ala Lys Asp Thr
    3425                3430                3435

Gly Gly Thr Thr Val Asn Val Ala Gly Thr Asp Gly Asn Arg Val
    3440                3445                3450

Val Thr Gly Val Lys Glu Gly Ala Val Asn Ala Thr Ser Lys Asp
    3455                3460                3465

Ala Val Asn Gly Ser Gln Leu Asn Thr Thr Asn Gln Ala Val Val
    3470                3475                3480

Asn Tyr Leu Gly Gly Gly Ala Gly Tyr Asp Asn Ile Thr Gly Ser
    3485                3490                3495

Phe Thr Ala Pro Ser Tyr Thr Val Gly Asp Ser Lys Tyr Asn Asn
    3500                3505                3510

Val Gly Gly Ala Ile Asp Ala Leu Asn Gln Ala Asp Gln Ala Leu
    3515                3520                3525

Asn Ser Lys Ile Asp Asn Val Ser Asn Lys Leu Asp Asn Ala Phe
    3530                3535                3540

Arg Ile Thr Asn Asn Arg Ile Asp Asp Val Glu Lys Lys Ala Asn
    3545                3550                3555

Ala Gly Ile Ala Ala Ala Met Ala Leu Glu Ser Ala Pro Tyr Val
    3560                3565                3570

Pro Gly Lys Tyr Thr Tyr Ala Ala Gly Ala Ala Tyr His Gly Gly
    3575                3580                3585

Glu Asn Ala Val Gly Val Thr Leu Arg Lys Thr Ala Asp Asn Gly
    3590                3595                3600

Arg Trp Ser Ile Thr Gly Gly Val Ala Ala Ala Ser Gln Gly Asp
    3605                3610                3615

Ala Ser Val Arg Ile Gly Ile Ser Gly Val Ile Asp
    3620                3625                3630

<210> 5
<211> 305
<212> PRT
<213> artificial sequence

<220>
<223> Nhead-GCN4pII-His

<400> 5

Met Ala Thr Val Gln Val Gly Gly Gly Thr Asn Ser Gly Thr Thr Ala
1               5                   10                  15

Thr Ala Ser Thr Asn Cys Ala Asp Leu Tyr Asn Tyr Gln Asn Pro Glu
            20                  25                  30

Asn Ser Gly Ser Gly Ala Ala Gly Asn Tyr Asn Ala Gly Asn Pro Ser
        35                  40                  45

Val Cys Ser Ile Ala Ile Gly Glu Asn Ala Gln Gly Gly Thr Ser Gly
    50                  55                  60

Thr Gly Gly Ser Pro Gly Ile Ala Ile Gly Gly Asn Ser Lys Ala Thr
65                  70                  75                  80

Gly Gly Leu Ser Val Ala Ile Gly Gly Tyr Ala Gln Ala Thr Asn Val
                85                  90                  95

Gly Ser Ile Ala Leu Gly Thr Ala Ala Leu Ser Ser Gly Phe Asn Ser
            100                 105                 110

Leu Ala Ile Ser Arg Gln Ala Ala Ala Thr Asn Asn Tyr Ser Ile Ala
        115                 120                 125

Ile Gly Thr Thr Ser Val Ser Lys Gly Val Gly Ser Ile Ala Met Gly
    130                 135                 140

His Ser Thr Asn Ala Ser Gly Asp Gln Ser Ile Ala Ile Gly Ser Ser
145                 150                 155                 160

Asp Ala Val Asn Ser Ala Thr Ala Thr Thr Thr Tyr Asp Gly Thr Thr
                165                 170                 175

Asn Thr Gln Ala Ser Gly Ser Lys Ser Ile Ala Ile Gly Ala Ser Ala
            180                 185                 190

Lys Ala Ser Thr Asn Asn Ser Ile Ala Leu Gly Ala Gly Ser Val Thr
        195                 200                 205

Ser Ala Gln Ser Gly Asn Ser Tyr Leu Thr Gly Val Gly Ala Ser Ala
    210                 215                 220

Thr Asn Gly Val Val Ser Val Gly Thr Ser Thr Ala Thr Arg Arg Ile
225                 230                 235                 240

Gln Asn Val Ala Asp Gly Ser Ala Ala Ser Asp Ala Val Thr Val Ala
                245                 250                 255

Gln Leu Asp Lys Ala Tyr Asp Asp Thr Asn Gly Arg Met Lys Gln Ile
            260                 265                 270

Glu Asp Lys Ile Glu Glu Ile Leu Ser Lys Ile Tyr His Ile Glu Asn
        275                 280                 285

Glu Ile Ala Arg Ile Lys Lys Leu Ile Lys Leu His His His His His
    290                 295                 300

His
305

<210>  6
<211>  7
<212>  PRT
<213>  artificial sequence

<220>
<223>  Synthetic Peptide

<400>  6

Gly Gly Ser Gly Gly Ser Lys
1               5

## Description

### [Technical Field]

The present disclosure relates to peptides that disintegrate the self-aggregation of AtaA polypeptides, which are adhesive polypeptides, or altered sequences thereof or fragments thereof having self-aggregation properties, and use thereof. The present disclosure further relates to peptides that separate the bond between AtaA polypeptides, or an AtaA polypeptide and another subject, for example, streptavidin or the like, and use thereof.

### [Background Art]

*Acinetobacter* sp. Tol5 (genus *Acinetobacter* bacteria Tol5 strain) are non-pathogenic gram-negative bacteria that have a high cell self-aggregation property and exhibits high adhesive properties to various material surfaces, from various hydrophobic plastic carriers to hydrophilic glass and even metal surfaces. The inventors have discovered a novel bacterial nanofiber present on the surface layer of a bacterial cell as a factor which brings about such adhesive properties that have not been reported in other microorganisms, and has further identified a new protein constituting the nanofiber. This protein belongs to the trimeric autotransporter adhesin (TAA) family of Gram-negative bacteria and is named AtaA by the inventors (Non-Patent Literature 1).

AtaA is extremely useful for immobilization of microorganisms and is expected to be applied to various bioprocesses (see, for example, Patent Literatures 1 and 2). Furthermore, in a previous patent application (Patent Literature 3), the inventors reported that various functional peptides and proteins can be introduced into AtaA and presented on the surface of a microorganism. Note that, in Patent Literature 1, AtaA and the gene encoding it (ataA gene) were referred to as AadA and aadA genes, respectively.

### [Citation List]

### [Patent Literature]

[PTL 1] WO 2009/104281
[PTL 2] WO 2014/156736
[PTL 3] WO 2015/030171

### [Non Patent Literature]

[NPL 1] Ishikawa, M.; Nakatani, H.; Hori, K., AtaA, a new member of the trimeric autotransporter adhesins from Acinetobacter sp. Tol 5 mediating high adhesiveness to various abiotic surfaces. PLoS One 2012, 7, (11), e48830.
[NPL 2] Linke, D.; Riess, T.; Autenrieth, I.B.; Lupas, A.; Kempf, V.A., Trimeric autotransporter adhesins: variable structure, common function. Trends Microbiol.2006, 14, (6), 264-270.

### [Summary of Invention]

### [Solution to Problem]

As a result of diligent studies, the inventors have found that a particular tripeptide sequence disintegrates the self-aggregation of the AtaA polypeptide or separates the bond between the AtaA polypeptide fragment and another subject.

Therefore, the present disclosure provides the following.
(1) A peptide comprising an amino acid sequence of AVL, SVL or ATL, or a functional equivalent sequence thereof.
(2) A peptide consisting of an amino acid sequence of AVL, SVL or ATL, or a functional equivalent sequence thereof.
(3) The peptide according to any one of the items above, which has a function of disintegrating the self-aggregation of a polypeptide having the amino acid sequence set forth in SEQ ID NO: 4 or an altered sequence and fragment thereof having a self-aggregation property.
(4) The peptide according to any one of the items above, which has a function of disintegrating the aggregation of cells expressing a polypeptide comprising the amino acid sequence set forth in SEQ ID NO: 4 or an altered sequence thereof or a fragment thereof.
(4B) An attaching and detaching method comprising the steps of: (1) bringing an autotransporter adhesin derived from *Acinetobacter* microorganisms, such as a microorganism to which a non-specific adhesion property is imparted or enhanced by introducing a DNA encoding the amino acid sequence set forth in SEQ ID NO: 4 or an altered sequence thereof, or a fragment thereof, into contact with a carrier to adhere the microorganism to the carrier; and (2) washing the carrier to which the microorganism is attached in the presence of the peptide according to any one of the items above to desorb the microorganism from the carrier.
(5) The peptide according to any one of the items above, which has a function of separating a bond between a polypeptide comprising the amino acid sequence set forth in SEQ ID NO: 1 or an altered sequence thereof or a fragment thereof, or a fusion or complex of the polypeptide with another amino acid sequence, and another subject.
(6) A composition comprising the peptide according to any one of the items above, for disintegrating self-aggregation of a polypeptide having the amino acid sequence set forth in SEQ ID NO: 4 or an altered sequence and fragment thereof having a self-aggregation property.
(7) A composition comprising the peptide according to any one of the items above or a similar peptide, for separating a bond between a polypeptide comprising the amino acid sequence set forth in SEQ ID NO: 1 or an altered sequence thereof or a fragment thereof, or a fusion or complex of the polypeptide with another amino acid sequence, and another subject.
(8) The composition according to any one of the items above, where the subject is a functionality-impairing entity.
(9) The composition according to any one of the items above, where the subject is streptavidin, neutral avidin or a variant of either thereof.
(10) The composition according to any one of the items above, where the functionality-impairing entity is a cell.
(11) A fusion or complex of the peptide according to any one of the items above and a functionality-impairing entity.
(12) Use of the peptide according to any one of the items above for disintegrating self-aggregation of a polypeptide having the amino acid sequence set forth in SEQ ID NO: 4 or an altered sequence and fragment thereof having a self-aggregation property.
(13) Use of the peptide according to any one of the items above or a similar peptide for separating a bond between a polypeptide comprising the amino acid sequence set forth in SEQ ID NO: 1 or an altered sequence thereof or a fragment thereof, or a fusion or complex of the polypeptide with another amino acid sequence, and another subject.
(14) The use according to any one of the items above, where the subject is a functionality-impairing entity.
(15) The use according to any one of the items above, where the subject is streptavidin, neutral avidin or a variant of either thereof.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

Using the present disclosure, it has become possible to disintegrate the self-aggregation of the AtaA polypeptides and fragments thereof. Therefore, the peptides of the present disclosure can inhibit laminated adhesion due to self-aggregation between AtaA polypeptides, and is thus useful, for example, in aspects where laminated adhesion is not preferable. Furthermore, the peptides of the present disclosure are capable of separating the bond of an AtaA polypeptide fragment to another subject (e.g., a functionality-imparting peptide such as streptavidin or neutral avidin) and is useful in controlling the on/off of processes in bioprocesses in technical fields such as fermentation industry, pharmaceutical industry, chemical product industry, regenerative medicine, cell engineering, medical engineering and biosensors as well as in material engineering processes such as surface modification and material design. Additionally, the peptides of the present disclosure are capable of separating cells (including microorganisms and biofilms) expressing AtaA polypeptides immobilized on the support, from the support, and adjustment of the thickness of the biofilm in the biofilm reactor and the separation from the adherent or fixed state allow cells and carriers to be reused. Furthermore, the peptides of the present disclosure can also be used as the means for isolating the Nhead domain present on the N-terminal side from the AtaA protein expressed on the cell membrane of *Acinetobacter.*

### [Brief Description of Drawings]

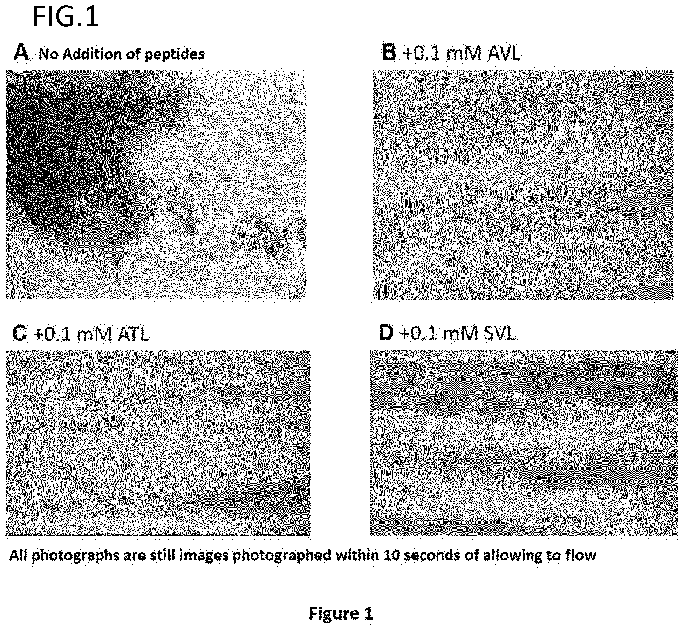

[Figure 1] Figure **1** shows a still image of the collapse of agglomerates of *Acinetobacter* sp. Tol5 strain when video shot with a microscope. **A** shows a microphotograph of the adherent aggregates of *Acinetobacter* sp. Tol5 strain, with a BS-N medium containing no peptide of the present disclosure delivered thereto. **B** shows a still image of the *Acinetobacter* sp. Tol5 strain with 0.1 mM AVL peptide added thereto. **C** shows a still image with the addition of 0.1 mM ATL peptide. **D** shows a still image with the addition of 0.1 mM SVL peptide. All photographs are still images photographed within 10 seconds after the medium containing the peptide (for A, the blank medium containing no peptide) was allowed to flow.

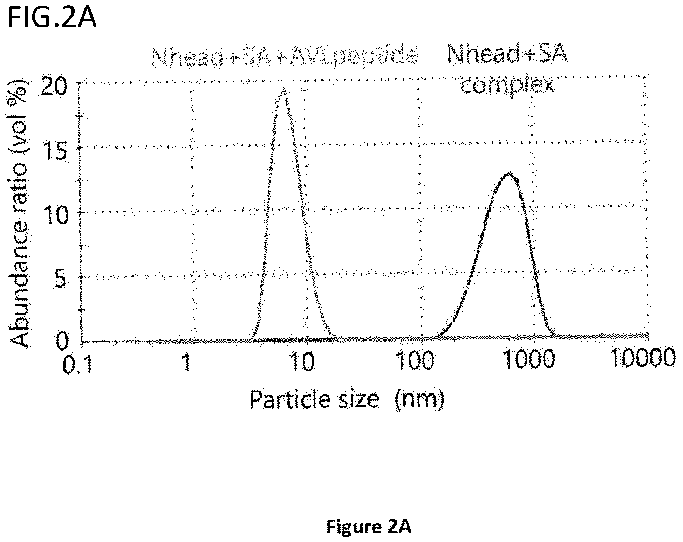

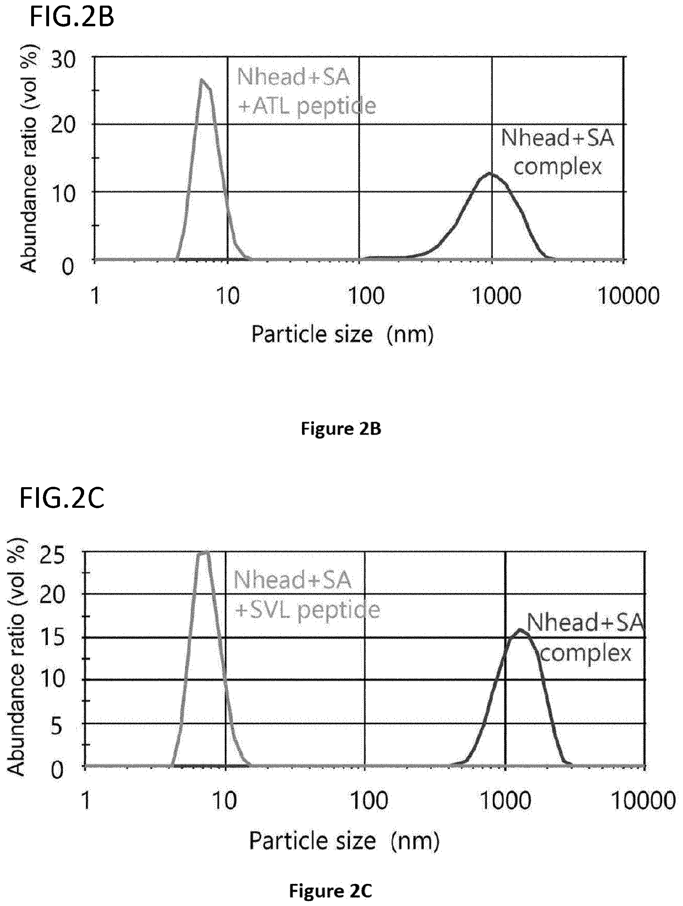

[Figure 2A] Figure **2** shows separation of the binding between E. coli recombinant Nhead and streptavidin by the peptides of the present disclosure. Figure **2A** shows results of AVL peptide addition. The vertical axis of the graph shows the abundance ratio (volume%) of each particle, while the horizontal axis thereof shows the particle size (nm) . Note that the peaks of Nhead and SA after the addition of the peptide overlap with each other.
[Figure 2B] Figure **2** shows separation of the binding between E. coli recombinant Nhead and streptavidin by the peptides of the present disclosure. Figure **2B** shows results of ATL peptide addition. The vertical axis of the graph shows the abundance ratio (volume%) of each particle, while the horizontal axis thereof shows the particle size (nm). Note that the peaks of Nhead and SA after the addition of the peptide overlap with each other.
[Figure 2C] Figure **2** shows separation of the binding between E. coli recombinant Nhead and streptavidin by the peptides of the present disclosure. Figure **2C** shows results of SVL peptide addition. The vertical axis of the graph shows the abundance ratio (volume%) of each particle, while the horizontal axis thereof shows the particle size (nm). Note that the peaks of Nhead and SA after the addition of the peptide overlap with each other.

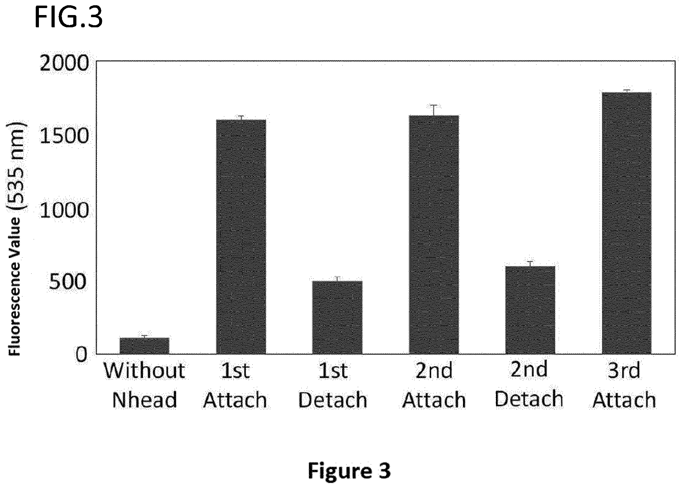

[Figure 3] Figure **3** shows the immobilization of biotinylated molecules using the bond between Nhead and streptavidin. The graph shows the detection result by fluorescence of the biotinylated molecules. The vertical axis of the graph shows the fluorescence intensity of 535 nm, while the horizontal axis shows the fluorescence intensity in order from the left, when Nhead is not immobilized, after the first binding reaction, after the first separation reaction, after the second binding reaction, after the second separation reaction, and after the third binding reaction.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or basic technical concepts that are particularly used herein are described hereinafter when appropriate.

### (Definitions of Terms)

As used herein, "adhere, adhesion" and "immobilize, immobilization" are used interchangeably and used in the sense commonly used in the art, and mean to make at least a portion of the subject of "adhesion" or "immobilization" at least relatively immobile from a certain position on the subject to be adhered or immobilized.

As used herein, "aggregate, aggregation" is used in the sense commonly used in the art and means that polypeptides, cells or the like, in their dispersed state, gather in a mass with the polypeptides themselves, with other polypeptides, with the cells or the like. Particularly when the same type of polypeptide chains aggregate together, it is referred to as "(self-aggregate, self-aggregation)". For example, as to AtaA, the self-aggregation of multiple AtaA or cells expressing AtaA causes the phenomenon of forming a mass of AtaA peptides or cells; thus, AtaA can be considered as having self-aggregating ability. It has been revealed in the present disclosure that the peptides of the present disclosure can disintegrate the self-aggregation between AtaAs or cells expressing AtaA. For example, the use of the peptides of the present disclosure prevents laminated adhesion from being performed, and thus, it is advantageous in the situation where the laminated adhesion is not preferable.

In addition, applications that maintain the adhesion of AtaA polypeptide but do not allow self-aggregation are useful in the following applications. Specifically, the peptides of the present disclosure can also disperse agglomerates of cells such as microorganisms by disintegrating the self-aggregation between AtaA. Accordingly, the peptides of the present disclosure can also be used in biofilm reactors for applications, such as stripping and cleaning biofilms, and is thus useful in the fermentation industry and biofuel production. Further, the peptides of the present disclosure control the on/off of the bond between the AtaA polypeptide fragment (particularly the fragment that has lost the aggregation property but maintains the adhesive property thereof) and the functionality-impairing entity so that the peptides are useful for controlling the function of the functionality-impairing entity in in bioprocesses in technical fields such as fermentation industry, pharmaceutical industry, chemical product industry, regenerative medicine, cell engineering, medical engineering and biosensors as well as in material engineering processes such as surface modification and material design.

As used herein, "bind, bound" is used in the sense commonly used in the art and means a physical or chemical association between two substances, or a combination thereof. In particular, in the present disclosure, the interaction between AtaA polypeptide and streptavidin, neutral avidin, variants thereof, or the like falls under the "bind" herein. As used herein, "binding" is also referred to as "specific binding" because it corresponds to a specific interaction. On the other hand, "adhesion" or "immobilization" is due to non-specific interactions.

As used herein, "separation, separate" is used in the sense commonly used in the art and means that in two or more substances that are in a "bound" state, at least a part of the "bound" state between the substances is released.

As used herein, "disintegration, disintegrate" is used in the sense commonly used in the art and means that in two or more substances in an "aggregated" state, at least a part of the "aggregated" state between the substances is released.

As used herein, "AtaA" is a protein belonging to the trimeric autotransporter adhesin (TAA) family possessed by Gram-negative bacteria. When "AtaA", "AtaA peptide", "a variant of AtaA peptide" and "AtaA polypeptide or a fragment thereof" are referred to herein, unless otherwise specified, they are to encompass not only the full-length AtaA polypeptide, but also variants and fragments of the AtaA polypeptide that have either or both the self-aggregation property and the binding ability to other subjects. AtaA usually has adhesive ability. AtaA forms a homotrimer and has a domain group structure with a signal peptide-Nhead-Nstalk-Chead-Cstalk-Membrane anchor arranged therein in this order from the amino terminal to the carboxy terminal. Respective domain groups are linked by a structure such as a neck or a coiled coil. In addition, Nhead, Nstalk, Chead, and Cstalk include various domains such as Ylhead, Trp-ring, GIN and FGG, and also include some domains repeatedly. Respective domains are linked by a structure such as a neck or a coiled coil. The full-length amino acid sequence and nucleic acid sequence of AtaA are shown in SEQ ID NOs: 4 and 3, respectively. The amino acid sequence and nucleic acid sequence of the fragment sequence of AtaA that does not include a signal peptide at the N-terminal but includes methionine at the beginning are shown in SEQ ID NOs: 1 and 2, respectively. In SEQ ID NOs: 1 and 2, Nhead (including Neck) corresponds to amino acid positions 2 to 258 and nucleic acid positions 4 to 774, Nstalk corresponds to amino acid positions 259 to 2846 and nucleic acid positions 775 to 8538, Chead (not including Neck) corresponds to amino acid positions 2855 to 3093 and nucleic acid positions 8563 to 9279, and Cstalk (including β-barrel inclusions) corresponds to amino acid positions 3094 to 3518 and nucleic acid positions 9280 to 10554. In addition, in the Nhead domain, there are two loop structures that are related to adhesive property and aggregation property and are necessary for binding to the functionality-impairing entity, where these loop structures correspond to amino acid positions 22-50 and 161-175, and nucleic acid positions 64-150 and 481-525, respectively.

As used herein, "*Acinetobacter*" means the genus *Acinetobacter* in the taxonomy. The genus *Acinetobacter* typically includes the *Acinetobacter* sp. Tol5 strain. The *Acinetobacter* sp. Tol5 strain is a strain with toluene resolution ability, isolated from the exhaust gas treatment reactor and is available from Katsutoshi Hori Laboratory, Nagoya University. The AtaA peptide or fragments thereof in the present disclosure include, but are not limited to, those produced with the *Acinetobacter* sp. Tol5 strain belonging to the genus *Acinetobacter.*

As used herein, the "functionality-impairing entity" is used in the sense commonly used in the art and means those that have any functions or actions. The functionality-impairing entity is bound by the AtaA polypeptide or fragment thereof in the present disclosure. The functions or actions are typically various functions or actions useful in bioprocesses in technical fields such as pharmaceutical industry, chemical product industry, regenerative medicine, cell engineering, medical engineering and biosensors, in materials engineering processes and the like, and include, but are not limited to, functions such as labeling. In one embodiment, the functionality-impairing entity is a protein and/or peptide, or a cell including them, a portion thereof, a biological tissue or organ, or a portion thereof. In a specific embodiment, the functionality-impairing entity is streptavidin, neutral avidin or a variant thereof.

As used herein, "streptavidin" is used in the sense commonly used in the art and means streptavidin produced by the genus Streptomyces, or variants, derivatives or equivalents thereof. Examples of the amino acid sequence thereof include, but are not limited to, P22629-1, and examples of the nucleic acid sequence thereof include, but are not limited to, those specified by X03591. The "streptavidin" herein typically binds to biotin with high affinity. As used herein, "neutral avidin" is used interchangeably with "NeutrAvidin" and the like in the same sense, and means a variant of avidin (particularly, a sugar chain-removed substance) which has a pI value near neutrality and in which non-specific binding is suppressed. "Neutral avidin" also typically binds to biotin with high affinity. "Streptavidin" and "neutral avidin" have the property of specifically binding to the AtaA polypeptide or fragments thereof in the present disclosure.

As used herein, a "corresponding" amino acid or nucleic acid or moiety refer to: an amino acid or nucleotide that has or is expected to have a similar action as a given amino acid or nucleotide or moiety in the polypeptide or polynucleotide to be used as a reference for comparison, in a certain polypeptide or polynucleotide molecule (e.g., AtaA and the like); and for adhesive polypeptides in particular, an amino acid that is present at similar positions in the site required for adhesion or self-aggregation and makes a similar contribution to adhesive property. For example, as for an antisense molecule, it can be a similar part in the ortholog corresponding to a particular part of the antisense molecule. The corresponding amino acid can be, for example, a specific amino acid that is cysteinylated, glutathioneized, S-S bond formed, oxidized (e.g., methionine side chain oxidation), formylated, acetylated, phosphorylated, glycosylated, myristilized, and the like. Alternatively, the corresponding amino acid can be the amino acid responsible for trimerization. Such a "corresponding" amino acid or nucleic acid may be a region or domain over a certain range. Therefore, in such cases, they are referred to herein as a "corresponding" region or domain.

As used herein, "fusion" means that two or more molecules (e.g., polypeptides or nucleic acids) or parts thereof are linked by covalent binding. When two or more moieties are polypeptides and they are covalently bound, they are fusions or fusion products produced by "fusion", or fusion proteins or fusion polypeptides, which can also be referred to as chimeric polypeptides. Regularly, in the case of a polypeptide fusion, a fusion of nucleic acids is prepared by linking the base sequences of nucleic acids encoding two types of polypeptides, and the fusion is used as a design drawing to produce a fusion polypeptide as a recombinant protein.

As used herein, "complex" is used in the sense commonly used in the art and means any construct including two or more moieties. For example, if one of the moieties is a polypeptide, the other of the moieties may be a polypeptide or any other substances (e.g., sugars, lipids, nucleic acids and other hydrocarbons). Two or more moieties that constitute the complex herein can be bound in a noncovalent manner (e.g., hydrogen bond, ionic bond, hydrophobic interaction, van der Waals force, and the like). Therefore, as used herein, the "complex" refers to molecules formed by binding a plurality of types of molecules, such as polypeptides, polynucleotides, lipids, sugars, and small molecules, by methods other than covalent binding.

In the present disclosure, "functional equivalent sequence" is used for peptides and the like of the present disclosure and means an amino acid sequence or nucleic acid sequence having a function equivalent to the original function. Typical functional equivalent sequences can include, but are not limited to, sequences including conservative substitution amino acids.

In the present disclosure, "conservative substitution" means a substitution in which the hydrophilicity index and/or the hydrophobicity index of the substituting amino acid is similar to that of the original amino acid as described above. Examples of the conservative substitution are well known to those of skill in the art and include, but are not limited to, for example, substitutions within each of the following groups: arginine and lysine; glutamic acid and aspartic acid; serine and threonine; glutamine and asparagine; and valine, leucine, isoleucine, and the like. It is understood that certain sequences of the tripeptides of the present disclosure may typically be conservatively substituted in part or in whole.

The "variant", "derivative", "analog" or "mutant" (of proteins, peptides, and the like) as used herein are used interchangeably, and the above terms preferably include molecules including regions that are substantially homologous to the protein or peptide of interest, although not intended to be limiting; and in the case of amino acid sequence, nucleic acid sequence (nucleotide sequence, base sequence), such sequences are referred to as "altered sequence", "derived sequence", "analogous sequence", and "mutated sequence". Such molecules, in various embodiments, are at least 30%, 40%, 50%, 60%, 70%, 80%, 90%, 95%, or 99% identical, throughout amino acid sequences of the same size, or in comparison with sequences to be aligned by conducting alignment with a computer homology program that is known in the art; or nucleic acids encoding such molecules can hybridize to sequences encoding component proteins under (highly) stringent, moderately stringent, or non-stringent conditions. This means that each is a product of protein alteration by amino acid substitutions, deletions and additions, in which the variant or derivative thereof still exhibits the biological function of the original protein, but not necessarily to the same degree. The variant may have a sequence that is preferably 70% or more identical, more preferably 80% or more identical, even more preferably 90% or more identical, still even more preferably 95% or more identical, 98% or more identical, 99% or more identical, to the original sequence. For example, it is also possible to investigate the biological function of such a protein by appropriate and available *in vitro* assays described herein or known in the art. "Functionally active" as used herein refers to a polypeptide, i.e., a fragment or derivative, having a structural, regulatory, or biochemical function of protein, such as biological activity, according to aspects to which the polypeptide, i.e., the fragment or derivative, of the present disclosure is associated herein. Accordingly, in one embodiment, the variant of the present disclosure refers to a functional equivalent.

As used herein, "protein", "polypeptide" and "peptide" are used with the same meaning herein and refer to a polymer of amino acids of any length. As used herein, an amino acid polymer with the amino acids in length of 3, 4, 5, 6, 7, 8, 9 or 10 amino acids is specifically referred to as an "oligopeptide". The length of the oligopeptide is preferably 3 to 10 amino acids, more preferably 3 to 6 amino acids, and even more preferably 3 to 5 amino acids. The polymer may be linear, branched or cyclic. The amino acids may be natural or non-natural, or may be altered amino acids. The term may also include those assembled into a complex of multiple polypeptide chains. The term also includes naturally or artificially altered amino acid polymers. Such alterations include, for example, disulfide bond formation, glycosylation, lipidation, acetylation, phosphorylation, methylation, trimethylation or any other manipulation or alteration (e.g., conjugation with a labeling component) This definition also includes, for example, polypeptides including one or more analogs of amino acids (including, for example, unnatural amino acids), peptide-like compounds (e.g., peptoids) and other alterations known in the art. As used herein, "amino acid" is a general term for organic compounds having an amino group and a carboxyl group. When the polypeptide according to the embodiments of the present disclosure includes a "specific amino acid sequence", any amino acid in the amino acid sequence may be chemically modified. In addition, any amino acid in the amino acid sequence may form a salt or a solvate. Furthermore, any amino acid in the amino acid sequence may be L-type or D-type. Even in such cases, the protein according to the embodiments of the present disclosure can be said to include the above "specific amino acid sequence". As to the chemical modification that an amino acid included in a protein undergoes *in vivo,* knowns are, for example, N-terminal modification (e.g., acetylation, myristoylation, etc.), C-terminal modification (e.g., amidation, glycosylphosphatidylinositol addition, etc.), side chain modification (e.g., phosphorylation, sugar chain addition, etc.) or the like. The amino acids may be any natural or non-natural amino acids that satisfy the objectives of the present disclosure.

As used herein, peptides having the ability to disintegrate self-aggregation are sometimes referred to as "disintegration peptides", and peptides that have the ability to separate bonds are referred to as "separated peptides". While not wishing to be bound by theory, it is understood that even if the peptides have the ability to separate, the peptides may not be able to detach adhesion.

As used herein, "polynucleotide", "oligonucleotide" and "nucleic acid" are used with the same meaning herein and refer to a polymer of nucleotide of any length. The term also includes an "oligonucleotide derivative" or "polynucleotide derivative". The "oligonucleotide derivative" or "polynucleotide derivative" refers to an oligonucleotide or polynucleotide that includes a derivative of a nucleotide or has an unusual bond between nucleotides and is used interchangeably. Specific examples of such oligonucleotides include, for example 2'-O-methyl-ribonucleotide, an oligonucleotide derivative in which the phosphate diester bond in the oligonucleotide is converted to a phosphorothioate bond, an oligonucleotide derivative in which the phosphate diester bond in the oligonucleotide is converted to an N3'-P5'phosphodiester bond, an oligonucleotide derivative in which a ribose and a phosphate diester bond in the oligonucleotide are converted into a peptide nucleic acid bond, an oligonucleotide derivative in which uracil in the oligonucleotide is substituted by C-5 propynyl uracil, an oligonucleotide derivative in which uracil in the oligonucleotide is substituted by C-5 thiazole uracil, an oligonucleotide derivative in which cytosine in the oligonucleotide is substituted by C-5 propynylcytosine, an oligonucleotide derivative in which cytosine in the oligonucleotide is substituted by phenoxazine-modified cytosine, an oligonucleotide derivative in which ribose in DNA is substituted by 2'-O-propylribose, and an oligonucleotide derivative in which ribose in the oligonucleotide is substituted by 2'-methoxyethoxyribose. Unless otherwise indicated, the specific nucleic acid sequence is also intended to include sequences that are explicitly shown, as well as the conservatively altered variants (e.g., a degenerate codon substitution) and complementary sequences thereof. Specifically, the degenerate codon substitution can be achieved by creating a sequence in which the third position of one or more selected (or all) codons is substituted by a mixed base and/or deoxyinosine residue (Batzer et al., Nucleic Acid Res.19:5081(1991); Ohtsuka et al., J. Biol. Chem. 260: 2605-2608(1985); Rossolini et al., Mol. Cell. Probes 8:91-98(1994)). As used herein, "nucleic acid" is also used interchangeably with genes, cDNAs, mRNAs, oligonucleotides, and polynucleotides. As used herein, "nucleotide" may be natural or non-natural.

As used herein, "gene" means a factor that defines a genetic trait, and "gene" may also refer to "polynucleotide", "oligonucleotide" and "nucleic acid".

As used herein, "homology" of a gene means the degree of identity of two or more gene sequences to each other, and generally, having "homology" means a high degree of identity or similarity. Thus, as the homology of two certain genes increases, the identity or similarity of the sequences thereof increases. Whether or not two types of genes are homologous can be determined by direct comparison of the sequences, or in the case of nucleic acids, can be examined by hybridization methods under stringent conditions. In direct comparison of two gene sequences, the genes thereof are homologous if the DNA sequences are typically at least 50% identical, preferably at least 70% identical, more preferably at least 80%, 90%, 95%, 96%, 97%, 98% or 99% identical, between the gene sequences. Similarly, the concept of "homology" also applies to amino acid sequences, in which case the numerical values of identity etc. can be interpreted by replacing them with amino acid sequences. Accordingly, as used herein, "homolog" or "homologous gene product" means a protein in another species, preferably a microorganism, more preferably a bacterium, which exerts the same biological function as the protein component of the complex to be further described herein. Such a homolog is also sometimes referred to as an "ortholog gene product". It is understood that such a homolog, homologous gene product, ortholog gene product, etc. can also be used as long as they meet the objectives of the present disclosure.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.7.1 (published on October 19, 2017) of the NCBI. Herein, values for "identity" generally refer to a value obtained when aligned under the default conditions using BLAST. However, when a higher value is obtained by changing a parameter, the highest value is considered the value of identity. When identity is evaluated in a plurality of regions, the highest value thereamong is considered the value of identity. "Similarity" is a value calculated by taking into consideration a similar amino acid in addition to identity.

In one embodiment of the present disclosure, the "several" may be, for example, 10, 8, 6, 5, 4, 3, or 2, or less than or equal to any of these values. It is known that a polypeptide with one or several amino acid residue deletions, additions, insertions or substitutions with other amino acids, still maintains its biological activity (Mark et al., Proc Natl Acad Sci USA.1984 Sep; 81(18): 5662-5666., Zoller et al., Nucleic Acids Res. 1982 Oct 25; 10(20): 6487-6500., Wang et al., Science.1984 Jun 29; 224(4656): 1431-1433.). Peptides with deletions and the like can be prepared, for example, by a site-specific mutagenesis method, a random mutagenesis method, or the like. As a site-specific mutagenesis method, for example, KOD-Plus-Mutagenesis Kit (TOYOBO CO., LTD.) can be used. It is possible to select a polypeptide having the same activity as the wild-type from the mutant-type polypeptides in which a deletion or the like has been introduced, by performing various characterizations such as FACS analysis and ELISA.

In one embodiment of the present disclosure, the numerical value of identity or the like, i.e., "70% or greater", can be, for example, 70% or greater, 75% or greater, 80% or greater, 85% or greater, 90% or greater, 95% or greater, 96% or greater, 97% or greater, 98% or greater, 99% or greater, or 100% or greater, or within a range between any two of numerical values of such starting points. The above "identity" is computed by computing the ratio of the number of amino acids homologous in two or more amino acid sequences in accordance with a known method described above. Specifically, amino acid sequences in a group of amino acid sequences to be compared are aligned before computing the ratio, and a space is introduced in a part of the amino acid sequences if needed to maximize the ratio of identical amino acids. A method for alignment, ratio computation method, comparison method, and computer program associated therewith are conventional and well known in the art (e.g., aforementioned BLAST or the like). As used herein, "identity" and "similarity" can be represented by a value measured by NCBI's BLAST, unless specifically noted otherwise. Blastp can be used with the default settings as the algorithm for comparing amino acid or base sequences with BLAST. Measurement results are quantified as Positives or Identities.

As used herein, a "polynucleotide that hybridizes under stringent conditions" refers to well-known conditions commonly used in the art. Such a polynucleotide can be obtained by using a polynucleotide selected from the polynucleotides of the present disclosure as a probe and using a colony hybridization method, a plaque hybridization method or a southern blot hybridization method. Specifically, the above means such a polynucleotide that can be identified by performing hybridization at 65°C in the presence of 0.7 to 1.0 M NaCl using a filter with immobilized DNA from a colony or plaque, followed by washing the filter under 65°C conditions with a 0.1 to 2-fold concentration SSC (saline-sodium citrate) solution (where the composition of the 1-fold concentration SSC solution is 150 mM sodium chloride and 15 mM sodium citrate). For the "stringent conditions", the conditions below, for example, can be adopted: (1) use of low ionic strength and high temperature for washing (e.g., at 50°C, 0.015M sodium chloride/0.0015M sodium citrate/0.1% sodium dodecyl sulfate); (2) use of a denaturing agent such as formamide during hybridization (e.g., at 42°C, 50% (v/v) formamide and 0.1% bovine serum albumin/0.1% ficoll/0.1% polyvinylpyrrolidone/50 mM and pH 6.5 sodium phosphate buffer, and 750 mM sodium chloride, 75 mM citrate sodium), or (3) incubation overnight at 37°C in a solution containing 20% formamide, 5 × SSC, 50 mM sodium phosphate (pH 7.6), 5 × denhard solution, 10% dextran sulfate, and 20 mg/ml denatured sheared salmon sperm DNA, followed by washing the filter with 1 × SSC at about 37 to 50°C. Note that the formamide concentration may be 50% or higher. Washing time may be 5, 15, 30, 60, or 120 minutes, or longer. Multiple factors such as temperature and salt concentration can be considered as factors that affect the stringency of the hybridization reaction, and for details, Ausubel et al., Current Protocols in Molecular Biology, Wiley Interscience Publishers,(1995) may be referenced. Examples of "highly stringent conditions" are 0.0015M sodium chloride, 0.0015M sodium citrate, 65 to 68°C, or 0.015M sodium chloride, 0.0015M sodium citrate, and 50% sodium citrate, at 42°C. Hybridization can be performed in accordance with the method described in experimental publications such as Molecular Cloning 2nd ed., Current Protocols in Molecular Biology, Supplement 1-38, DNA Cloning 1: Core Techniques, A Practical Approach, Second Edition, Oxford University Press (1995). In this regard, sequences containing only the A sequence or only the T sequence are preferably excluded from the sequences to be hybridized under stringent conditions. Moderate stringent conditions can be easily determined by one of ordinary skill in the art on the basis of, for example, the length of the DNA, shown in Sambrook et al., Molecular Cloning: A Laboratory Manual, No. 3, Vol.1, 7. 42-7. 45 Cold Spring Harbor Laboratory Press, 2001; and as to nitrocellulose filters, included are the use of: hybridization conditions with a pre-cleaning solution of 5 × SSC, 0.5% SDS, and 1.0 mM EDTA (pH 8.0), and at about 40 to 50°C, about 50% formamide, 2 × SSC-6 × SSC (or other similar hybridization solution, such as Stark's solution, in about 50% formamide at about 42°C); and washing conditions of about 60°C, 0.5 × SSC, 0.1% SDS. Accordingly, the peptides used in this disclosure also include polypeptides coded by nucleic acid molecules that hybridize under highly or moderately stringent conditions to nucleic acid molecules that code the polypeptides specifically described in the present disclosure.

The peptides of the present disclosure may preferably be "purified" or "isolated". A "purified" substance or biological factor (e.g., nucleic acid or protein) as used herein refers to one in which at least some of the factors naturally associated with the substance or biological factor have been removed. Thus, the purity of the biological factor in the purified biological factor is usually higher (i.e., enriched) than the purity of the biological factor in the state in which the biological factor is normally present. The term, "purified", as used herein means that there is preferably at least 75% by weight, more preferably at least 85% by weight, even more preferably at least 95% by weight, and most preferably at least 98% by weight, of the same type of biological factor present. The substance or biological factor used in the present disclosure is preferably a "purified" substance. An "isolated" substance or biological factor (e.g., nucleic acid or protein) as used herein refers to one in which the factors naturally associated with the substance or biological factor have been substantially removed. The term, "isolated", as used herein does not necessarily have to be expressed in purity as it varies in accordance with the objective thereof, but if necessary, means that there is preferably at least 75% by weight, more preferably at least 85% by weight, even more preferably at least 95% by weight, and most preferably at least 98% by weight, of the same type of biological factor present. The substance used in the present disclosure is preferably an "isolated" substance or biological factor.

As used herein, "fragment" refers to a polypeptide or polynucleotide with a sequence length of 1 to n-1 with respect to a full-length polypeptide or polynucleotide (with the length of n) . The length of the fragment can be varied as appropriate according to the objective thereof, and the lower limit of the length thereof, in the case of polypeptides, includes, for example, 3, 4, 5, 6, 7, 8, 9, 10, 15, 20, 25, 30, 40, 50 and more amino acids; and lengths represented by integers not specifically listed here (e.g., 11) can also be appropriate as lower limits. Further, in the case of polynucleotides, included are 5, 6, 7, 8, 9, 10, 15, 20, 25, 30, 40, 50, 75, 100 and more nucleotides; and lengths represented by integers not specifically listed here (e.g., 11) can also be appropriate as lower limits. As used herein, it is understood that such a fragment falls within the scope of the present disclosure, for example, if the full length one functions as a separable active molecule, as long as the fragment itself also functions as a peptide.

As used herein, "biological function" refers to a specific function that a certain gene or a nucleic acid molecule or polypeptide associated therewith may have *in vivo* or *in vitro* in the context of the gene, nucleic acid molecule or polypeptide, the examples of which include, but are not limited to, for example, a disintegration function, a separation function and the like. As used herein, the biological function can be exerted by the corresponding "biological activity". As used herein, "biological activity" refers to the activity that a factor (e.g., polynucleotide, protein, etc.) may have, and includes activities that exert various functions (e.g., a disintegration function and a separation function). The "biological activity" may be an activity exerted *in vivo* or an activity exerted *in vitro* by secretion or the like. For example, if a certain factor is an enzyme, the biological activity thereof includes the enzyme activity thereof. Such biological activity can be measured by techniques well known in the art. Thus, "activity" refers to various measurable indicators that indicate or reveal binding (either direct or indirect) or various measurable indicators that affect the response (i.e., have a measurable effect in response to some exposure or stimulus), which may also include, for example, affinities for compounds that directly bind to the polypeptides or polynucleotides of the present disclosure, or for example, the amount of upstream or downstream protein after some stimulus or event, or other similar scales of function.

As used herein, "expression" of a gene, polynucleotide, polypeptide or the like refers to the gene or the like to undergo a certain action *in vivo* thus turning into a different form. This preferably refers to a gene, a polynucleotide or the like to be transcribed and translated into the form of a polypeptide; however, it is also an aspect of the expression that they are transcribed to produce mRNA. Accordingly, the "expression product" as used herein includes such a polypeptide or protein, or mRNA. More preferably, the form of such a polypeptide may be the one that is post-translationally processed. For example, the expression level of a peptide can be determined by any method. Specifically, assessing the amount of mRNA encoding the peptide, the amount of the peptide, and the biological activity of the peptide allows finding out of the expression level of the peptide. The amount of mRNA encoding the peptide, polypeptide or protein can be determined by methods as detailed elsewhere herein or by other methods known in the art.

As used herein, "similarity", "functional equivalence", or "functional equivalent" means any entity that has the same objective function but a different structure with respect to the original entity of interest. Thus, it is understood that the functional equivalent of the peptide of the present disclosure is not the peptide of the present disclosure itself, but includes a mutant or variant thereof (e.g., an amino acid sequence variant or the like), which has a biochemical action of the peptide, such as the function to separate bonds or the function to disintegrate self-aggregation as well as such a functional equivalent (including, for example, a nucleic acid encoding the mutant or variant, a vector or a cell including the nucleic acid, and the like) which, at the time of action, can be transformed into a mutant or variant that has the biochemical action of the peptide. In the present disclosure, it is understood that the functional equivalent of the peptide can be used in the same manner as the original peptide, without special mention. Functional equivalents can be found by searching databases and the like. As used herein, "searching" refers to finding another nucleic acid base sequence with a particular function and/or property by utilizing a certain nucleic acid base sequence electronically, biologically or otherwise. Examples of electronic searching include, but are not limited to, BLAST (Altschul et al., J. Mol. Biol. 215: 403-410(1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)). Examples of biological searching include, but are not limited to, stringent hybridization, microarrays with genomic DNA attached to nylon membranes, etc. or microarrays attached to glass plates (microarray assays), PCR and *in situ* hybridization. Herein, it is intended that the genes used in the present disclosure should also include the corresponding genes identified by such electronic and biological searching.

As for the functional equivalent of the present disclosure (polypeptide, etc.), it is possible to use amino acid sequences in which one or more amino acids are inserted, substituted or deleted, or added to one or both ends. As used herein, "one or more amino acids are inserted, substituted or deleted, or added to one or both ends" means that alterations have been made with the substitution of a plurality of amino acids that may occur naturally, by well-known technical methods such as site-specific mutagenesis methods, or by natural mutations. An altered amino acid sequence can have, for example, 1 to 30, preferably 1 to 20, more preferably 1 to 9, still more preferably 1 to 5, and particularly preferably 1 to 2 amino acids added to one or both ends thereof. The altered amino acid sequence may be preferably such an amino acid sequence that has one or more (preferably one or several, or 1, 2 or 3) conservative substitutions in the amino acid sequence of AVL, ATL or SVL. "Conservative substitution" herein means a substitution of one or more amino acid residues with other chemically similar amino acid residues so as not to substantially alter the function of the peptide. Examples thereof include substitutions of a hydrophobic residue with another hydrophobic residue, substitutions of a polar residue with another polar residue having the same charge, and the like. Functionally similar amino acids that can be substituted in this manner are known in the art for each amino acid. Specific examples include alanine, valine, isoleucine, leucine, proline, tryptophan, phenylalanine, methionine, and the like for nonpolar (hydrophobic) amino acids, and glycine, serine, threonine, tyrosine, glutamine, asparagine, cysteine, and the like for polar (neutral) amino acids. Examples of positively charged (basic) amino acids include arginine, histidine, lysine, and the like. Further, examples of a negatively-charged (acidic) amino acid include aspartic acid, glutamic acid, and the like.

As used herein, "support" refers to a substance capable of carrying a polypeptide, protein, polynucleotide, cell, bacterium, or virus. The material for use as a support can be any material that can form a solid surface, and examples thereof include, but are not limited to, a lipid double structure (e.g., liposome), glass, silica, silicon, ceramic, silicon dioxide, plastic, metal (including alloy), titanium, acrylic, natural and synthetic polymer (e.g., polystyrene, polypropylene, polyethylene, polyvinyl chloride, polyurethane, cellulose, chitosan, dextran, nylon, polycarbonate, polylactic acid, and polytetrafluoroethylene (PTFE)) .

As used herein, "kit" refers to a unit providing parts to be provided (e.g., an enzyme, buffer, user manual, and the like) which are generally separated into two or more compartments. Such a kit form is preferred when providing a composition, which should not be provided in a mixed state for stability or the like and is preferably used by mixing immediately prior to use. Such a kit advantageously comprises an instruction or user manual describing how the provided parts (e.g., peptides) are used or how a reagent or waste fluid after use should be processed. When a kit is used as a reagent kit herein, the kit generally comprises an instruction or the like describing the method of use of the enzyme, or the like.

As used herein, "instruction" is a document with an explanation of the method of use of the present disclosure for users. The instruction provides description for instructing the method of use of the present disclosure. If required, the instruction is prepared in accordance with a format specified by a regulatory authority of the country in which the present invention is practiced (e.g., Ministry of Health, Labour and Welfare, Ministry of Agriculture, Forestry and Fisheries, or the like in Japan, Food and Drug Administration (FDA) or Department of Agriculture (USDA) in the U.S., or the like), with an explicit description showing approval by the regulatory authority. An instruction can be provided in, but not limited to, paper media. An instruction can also be provided in a form such as electronic media (e.g., websites provided on the Internet or emails).

### (Preferred embodiments)

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

### (Peptide with disintegration or separation function)

In one aspect, the present disclosure provides peptides comprising the amino acid sequences of AVL, SVL or ATL or altered sequences thereof (particularly functional equivalent sequences). Herein, it is understood that the "functional equivalent sequence" includes a peptide having any sequence with the ability to disintegrate the self-aggregation of AtaA, or the ability to separate the binding of an AtaA fragment to another subject (e.g., functionality-impairing molecules, such as streptavidin and neutral avidin), of AVL, SVL or ATL peptides. Preferably, the peptides of the present disclosure include an amino acid sequence of AVL, SVL or ATL. More preferably, the peptides of the present disclosure consist of an amino acid sequence of AVL, SVL or ATL.

The peptides of the present disclosure may be in the form of oligopeptides including an amino acid sequence of AVL, SVL or ATL or altered sequences thereof. The above oligopeptides may be such oligopeptides in which any 1, 2, 3, 4, 5, 6, 7 or more amino acids are added to the N-terminus, C-terminus, or both of the amino acid sequences of AVL, SVL or ATL or altered sequences thereof. Preferably, amino acids added to the N-terminus, C-terminus, or both are amino acids with short side chains (e.g., Gly, Ala, Val, Leu and Ile, etc.) or amino acids with no side chain charge (amino acids other than Glu, Asp, Lys, His and Arg).

In a preferred embodiment, this peptide consists of an amino acid sequence of AVL, SVL or ATL or a functional equivalent sequence thereof. In such a preferred embodiment, a short peptide referred to as a tripeptide surprisingly exerts the ability to separate the binding of AtaA to other molecules (e.g., streptavidin and neutral avidin).

In one aspect, the present disclosure provides a composition for separating the binding of AtaA or a variant thereof to another molecule. This composition contains a peptide comprising the amino acid sequence of AVL, SVL or ATL or a functional equivalent sequence thereof. Preferably, the peptides of the present disclosure include the amino acid sequence of AVL, SVL or ATL. More preferably, the peptides contained in this composition are an AVL, SVL or ATL tripeptide.

In one preferred embodiment, the AtaA or a variant thereof or fragment thereof, which is the subject of disintegration of self-aggregation or separation of binding by the peptides of the present disclosure, may be any embodiment of the "AtaA polypeptide" described herein. In one embodiment, the peptides of the present disclosure are used to disintegrate an agglomerate of AtaA or a variant thereof. Disintegration of the agglomerate facilitates the isolation, purification, etc., of AtaA or a variant thereof. In addition, the peptides of the present disclosure are used to disintegrate the aggregation of cells expressing a polypeptide including the amino acid sequence set forth in SEQ ID NO: 4 or an altered sequence thereof or a fragment thereof. Thus, the peptides of the present disclosure can disintegrate microbial agglomerates and biofilms formed by AtaA or a variant thereof. In another embodiment, the peptides of the present disclosure are used to separate the binding of the AtaA fragment or a variant thereof set forth in SEQ ID NO: 1 to another subject.

In one embodiment, the present disclosure provides a method for attaching and detaching microorganisms, the method including the steps of: (1) bringing an autotransporter adhesin derived from *Acinetobacter* microorganisms, such as a microorganism to which a non-specific adhesion property is imparted or enhanced by introducing a DNA encoding the amino acid sequence set forth in SEQ ID NO: 4 or an altered sequence thereof, or a fragment thereof, into contact with a carrier to adhere the microorganism to the carrier; and (2) washing the carrier to which the microorganism is attached in the presence of the peptide of the present disclosure (e.g., the peptide according to the item (1) or (2) of the present disclosure) to desorb the microorganism from the carrier. As for the attaching and detaching method, for example, approaches described in Japanese Laid-Open Publication No. 2017-55676 and the like can be utilized. As used herein, "attaching and detaching (method)" refers to a method including the step (operation) of attaching a microorganism to a carrier and the step (operation) of desorbing the microorganism attached to the carrier.

In the above step (1), i.e., the attaching step, prepared first are microorganisms (also referred to herein as "adhesive property-imparting microorganisms") in which a non-specific adhesive property has been imparted or enhanced by the introduction of DNA (e.g., a sequence encoding the amino acid sequence set forth in SEQ ID NO: 4 or an altered sequence thereof, or a fragment thereof; also referred to as "adhesive property-imparting DNA" herein) encoding AtaA derived from *Acinetobacter* microorganisms. Examples of the adhesive property-imparting DNA are, preferably, sequences encoding the amino acid sequence set forth in SEQ ID NO: 4 or an altered sequence thereof, or a fragment thereof, which is any AtaA polypeptide described in detail in other parts of the present specification, isolated or identified from *Acinetobacter* sp. Tol5 strains.

Various microorganisms can be used as the microorganisms into which adhesive property-imparting DNA is introduced (target microorganisms). The target microorganisms are not particularly limited, and examples thereof include microorganisms having no or weak non-specific adhesive property. The target microorganisms may be any of a wild strain, a mutant strain, and a recombinant strain. Appropriate microorganism are selected in accordance with objectives of the present invention. Examples of microorganisms that can be used as the target microorganisms include: bacteria of the genus *Escherichia*, such as *Escherichia coli*; bacteria of the genus *Acinetobacter,* such as *Acinetobacter calcoaceticus*; bacteria of the genus *Ralstonia,* such as *Ralstonia eutropha;* bacteria of the genus *Pseudomonas,* such as *Pseudomonas putida* or *Pseudomonas fluorescens;* bacteria of the genus *Aeromonas,* such as *Aeromonas caviae;* bacteria of the genus *Alcaligenes,* such as *Alcaligenes latus;* bacteria of the genus *Xanthomonas,* such as *Xanthomonas campestris;* bacteria of the genus *Desulfomonile,* such as *Desulfomonile tiedjei;* bacteria of the genus *Desulfuromonas,* such as *Desulfuromonas chloroethenica;* bacteria of the genus *Chromobacterium,* such as *Chromobacterium chocolatum;* bacteria of the genus *Burkholderia,* such as *Burkholderia arboris;* bacteria of the genus *Rhodobacter;* bacteria of the genus *Acidovorax,* such as *Acidovorax facilis;* and bacteria of the genus *Zymomonas,* such as *Zymomonas mobilis.* Introduction of the adhesive property-imparting DNA into the target microorganisms and transformation thereof allows obtaining of microorganisms with the non-specific adhesive property imparted thereto or enhanced therein. Typically, ligating the adhesive property-imparting DNA to a suitable vector and transforming the target microorganisms (host microorganisms) with the vector allows obtaining of microorganisms with the non-specific adhesive property imparted thereto or enhanced therein. Specifically, the vector was prepared by ligating the DNA under the control of a constitutively expressed promoter or by ligating the DNA under the control of an inducible expression system promoter. Transforming the host microorganisms with this vector allows obtaining of microorganisms with the non-specific adhesive property imparted thereto or enhanced therein.

In the step (1), the prepared adhesive property-imparting microorganisms are brought into contact with a carrier. The contact here is carried out under conditions where the adhesive property-imparting microorganisms can adhere to the carrier. The adhesive property-imparting microorganisms have high adhesion and exhibit adhesive properties to various carriers under normal culture conditions. Therefore, except in special cases (typically, when the desorption treatment liquid adopted in the step (2) described below is used as a medium, or under low ionic strength (see International Publication No. WO 2014/156736 Pamphlet)), the adhesive property-imparting microorganisms adhere to the carrier by contact.

The adhesive property-imparting microorganisms adhered to the carrier in the step (1) are usually subjected to one or more treatments or reactions (for details of the treatments or reactions, see Japanese Laid-Open Publication No. 2017-55676 for example). In the present disclosure, the step (2), that is, the desorption step is subsequently performed. In the step (2), the adhesive property-imparting microorganisms adhered to the carrier are treated with a solution containing the separated peptide according to the present disclosure that meets predetermined conditions so that the adhesive property-imparting microorganisms can be desorbed from the carrier.

In one embodiment, other molecules are streptavidin, neutral avidin or variants thereof. They are useful because the separation and binding can be freely manipulated in various test systems using streptavidin, neutral avidin or variants thereof.

In one typical embodiment, the present disclosure provides a composition for disintegrating self-aggregation of polypeptides having the amino acid sequence set forth in SEQ ID NO: 4 or an altered sequence and fragment thereof having a self-aggregation property.

In one embodiment, the other molecules mentioned above are functionality-impairing entities. Examples of the functionality-impairing entities include, but are not limited to, such entities having functions useful in biosensors, separation and labeling of biomolecules, regenerative medicine, production of bio, organic or inorganic hybrid materials, surface modification and functionalization, fermentation industry, pharmaceutical industry, chemical product industry, and the like. In a specific embodiment, functionality-impairing entities are proteins and/or peptides. In a more specific embodiment, functionality-impairing entities are streptavidin, neutral avidin, derivatives thereof, enzymes, antibodies, fluorescent molecules, receptors, functional peptides (antibacterial, fluorescent and signaling factors, inducing factors, etc.), nucleic acid aptamers, serum components, or cells. In a specific embodiment, the peptides of the present disclosure are provided as a complex or fusion with the above functional molecules.

### (AtaA polypeptides)

The peptides of the present disclosure are useful for disintegrating the self-aggregation of a full-length AtaA polypeptide consisting of the amino acid sequence set forth in SEQ ID NO: 4, or a polypeptide having an altered sequence and fragment thereof having a self-aggregation property.

The peptides of the present disclosure are also useful for disintegrating the aggregation of cells expressing a full-length AtaA polypeptide consisting of the amino acid sequence set forth in SEQ ID NO: 4 or a polypeptide including an altered sequence or fragment thereof having a self-aggregation property. The cells expressing the AtaA polypeptide or an altered sequence or fragment thereof may be bacterial cells expressing AtaA polypeptides by means, such as vectors, or other bacterial cells as well as the cells that naturally express AtaA such as the *Acinetobacter* sp. Tol5 strain.

### (Production of proteins or peptides)

The proteins and peptides of the present disclosure can be produced by expression systems using microorganisms, or chemical synthesis.

Needless to say, however, in the case of short peptides such as tripeptides, these peptides can also be synthesized by peptide synthesis methods well known in the art.

### (Use of peptides)

In one aspect, the peptides of the present disclosure are useful in materials engineering processes. In another aspect, the disintegration peptides and separated peptides of the present disclosure are useful in industries that use bioprocesses. In a certain embodiment, the disintegration peptides and separated peptides of the present disclosure disintegrate the self-aggregation of the AtaA polypeptides to densely consolidate and immobilize the proteins, peptides or microorganisms, thus enabling material engineering processes such as surface modification and material design as well as processes in bioprocesses in technical fields such as fermentation industry, pharmaceutical industry, chemical product industry, regenerative medicine, cell engineering, medical engineering and biosensors, to be carried out efficiently. Additionally, the peptides of the present disclosure are capable of separating cells (including microorganisms and biofilms) expressing AtaA polypeptides immobilized on the support, from the support, where adjustment of the thickness of the biofilm in the biofilm reactor and the separation from the adherent or fixed state allow cells and carriers to be reused.

### (Production of AtaA polypeptides)

In one embodiment, the adhesive polypeptides and fusion polypeptides of the present disclosure are produced by expression systems using microorganisms. The adhesive polypeptides or fusion polypeptides thereof of the present disclosure, can be produced in *E. coli* as recombinant proteins in the manner commonly used in laboratories. That is, a gene encoding a fragment of AtaA or a fusion peptide obtained by fusing a peptide to the fragment is inserted at an appropriate position of an appropriate *E. coli* expression vector, such as the pET system, to transform *E*. *coli,* followed by adding an inducer to induce gene expression or to constitutively express by constitutive expression promotion. The protein is often produced within the cytoplasm; however, the protein can also be secreted and produced in the periplasm. In the latter case, the gene sequence encoding the signal peptide originally possessed by the AtaA protein or the signal peptide integrated into the *E. coli* expression vector is coupled to the 5'end side encoding the protein so that the protein can be produced in the form of a signal peptide added to the amino-terminal side of the protein. Note that the signal peptide is cleaved and removed when the produced protein is secreted into the periplasm; thus, the resulting AtaA fragment or fusion protein thereof does not contain a signal peptide. The protein produced within cells is usually separated and purified from the solubilizing component, but may be obtained as an active protein by unwinding after its separation from the inclusion body. Note that the produced protein can be separated and purified by a commonly used affinity purification method using a His tag or Strep tag fused to the protein in advance. Furthermore, when a SNAP tag or the like is fused thereto, a molecule having a benzylguanyl group may be used for separation and purification. To further increase the degree of purification, ion exchange chromatography or gel filtration may be used in combination. In the production method of adhesive polypeptides using this microorganism, detailed conditions, such as the method for expressing adhesive polypeptides, the host for expressing adhesive polypeptides and the method for purifying adhesive polypeptides secreted from the host, are to be appropriately adjusted by those skilled in the art. Furthermore, the adhesive polypeptides of the present disclosure can also be expressed using various expression systems such as, secretory production systems and cell-free expression systems, in addition to the intrabacterial expression systems of microorganisms.

The method for causing the above-mentioned microorganisms to produce the polypeptides of interest including the adhesive polypeptides of the present disclosure is merely exemplary, and those skilled in the art can prepare synthetic genes using a method commonly used in the art, while referring to the nucleic acid sequences (base sequences) or amino acid sequences of the genes of the present disclosure, thereby readily performing the design and construct of a DNA fragment to be inserted into the expression vector.

### (General technology)

The molecular biological methodology, biochemical methodology, and microbiological methodology used herein are well known and commonly used in the art, which are described, for example, in Sambrook J. et al. (1989). Molecular Cloning: A Laboratory Manual, Cold Spring Harbor and its 4th Ed. (2012), Masato Okada, Kaori Miyazaki, 2011, "Protein Experiment Notes, Revised 4th Edition", First and Second Volumes, Yodosha Co., Ltd., or the like. Relevant portions thereof (which may be the entire document) are incorporated herein by reference.

### (Note)

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When required, organisms used in the following Examples were handled in compliance with the guidelines stipulated by the Nagoya University, regulatory agency, or the Cartagena Protocol. For reagents, the specific products described in the Examples were used. However, the reagents can be substituted with an equivalent product from another manufacturer (Sigma-Aldrich, Fuji Film, Wako Pure Chemical, Nacalai Tesque, R & D Systems, USCN Life Science INC, Thermo Fisher Scientific, Kanto Chemical, Funakoshi, Tokyo Chemical Industry, Merck, or the like).

The Nhead recombinant protein used in the following examples was prepared as follows.

### (Manufacture Examples)

The construct design of the Nhead recombinant protein is shown in SEQ ID NO: 5. The Nhead recombinant protein designed in this way expresses a recombinant polypeptide corresponding to the amino acid fragment at positions 59-325 of the AtaA sequence: the amino acid fragment at positions 2-268 of SEQ ID NO: 1. To prepare this construct, a gene fragment encoding amino acid 1-268 positions of SEQ ID NO: 1 was inserted into the restriction enzyme XbaI/BsaI digestion fragment of the pIBA-GCN4tri-His vector, thus preparing pIBA :: Nhead recombinant DNA (pIBA-GCN4tri-His : : ataA₅₉-₃₂₅).

Cultures obtained by culturing BL21 star (DE3; pIBA-GCN4tri-His :: ataA₅₉-₃₂₅) overnight were diluted 1: 100 in a LB medium and incubated at 37°C for 3 hours. After the incubation, 0.2 µg/ml anhydrous tetracycline (AHTC) was added to the medium, followed by further incubation at 28°C for 6 hours. The cells were harvested by centrifugation at 5, 000×g for 15 minutes at 4°C and resuspended in a lysis buffer (20 mM Tris-HCl, 150 mM NaCl and 20 mM imidazole, pH 9.0), followed by dissolving at 1,000 bar for 10 minutes using a high-pressure homogenizer (LAB 2000, SMT Co., Ltd.). After centrifugation at 10,000×g for 15 minutes at 4°C, the supernatant was loaded onto a Ni-NTA Superflow column (Qiagen NV) and washed twice with lysis buffer to remove unbound protein. The bound protein was eluted with an elution buffer (20 mM Tris-HCl, 150 mM NaCl and 400 mM imidazole, pH 9.0). The eluted protein was loaded onto an anion exchange column (HiTrap Q HP, GE Healthcare) and the flow-through fraction containing the recombinant protein was dialyzed against 50 mM phosphate buffer (pH 6.0). The recombinant protein was then purified using a cation exchange column (HiTrap SP HP, GE Healthcare) in a phosphate buffer (pH 6.0) with a linear concentration gradient of 0 to 1,000 mM NaCl. The peak fraction containing the recombinant protein was concentrated by ultrafiltration using a centrifugal filter device (Amicon ultra, EMD Millipore), loaded onto a gel filtration column (HiLoad 26/60 Superdex 200 pg, GE Healthcare) equilibrated with 25 mM Tris-HCl (pH 9.0), and purified by gel filtration. Those prepared by the above procedure were used in the following examples.

### (Example 1: Characteristics of agglutinates of Acinetobacter sp. Tol5 strain)

In this example, microscopic observation of agglutinates of *Acinetobacter* sp. Tol 5 strain was performed.

### (Method)

### (Preparation of Flow Cell)

The basic structure of the flow cell system constructed in the present disclosure is a silicone tube, for delivering a cell suspension or fluid, to which a glass tube is connected for observing cells. To construct a rectangular parallelepiped flow cell system, a silicone tube with a length of 300 mm, an inner diameter of 1 mm, and an outer diameter of 2 mm was connected to either end of rectangular parallelepiped glass with a length of 50 mm and each inner diameter of 1 mm (Vitrocom), where the connections were sealed with paraffin film. The silicone tube at the entrance was connected to a syringe pump (Legato 200, KD Scientific) via a three-way stopcock (Terumo Corporation), and the liquid was sent to the cell observation part (flow cell) of the rectangular parallelepiped glass tube.

*Acinetobacter* sp. Tol5 cell suspension suspended in BS-N medium was flowed through the above flow cell at a rate of 64 µl/min for 30 minutes to attach Tol5 cell aggregates to the glass flow cell. Subsequently, the BS-N medium containing 0.1 mM AVL peptide, 0.1 mM ATL peptide or 0.1 mM SVL peptide was flowed at the same rate, and the behavior of the attached Tol5 cell agglomerates was continuously observed with a digital microscope (VHX-200 manufactured by KEYENCE Corporation). As a control, a BS-N medium free of the peptides was delivered at the same rate.

The composition of the BS-N medium used in Example 1 is described in the paper, (S. Ishii, J. Koki, H. Unno, K. Hori; Two Morphological Types of Cell Appendages on a Strongly Adhesive Bacterium, Acinetobacter sp. Strain Tol5, Appl. Environ. Microbiol. 70, (2004) 5026-5029) and the paper, (H. Watanabe, Y. Tanji, H. Unno, K. Hori; Rapid Conversion of Toluene by an Acinetobacter sp. Tol5 Mutant Showing Monolayer Adsorption to Water-Oil Interface, J. Biosci. Bioeng. 106 (2008) 226-230).

### (Results and Discussion)

The results are shown in Figure **1****.** As to the *Acinetobacter* sp. Tol5 strain, there was no change in the aggregates of the bacterial cells when the control, or the medium containing no peptides, was flowed, whereas when AVL peptide, ATL peptide or SVL peptide was added, rapid (within 10 seconds) disintegration of the bacterial cell aggregation of the *Acinetobacter* sp. Tol5 strain was observed. From this, it was confirmed that the AVL peptide, ATL peptide or SVL peptide is a factor that disintegrates the self-aggregation between AtaA proteins.

### (Example 2: Separation activity of AVL, ATL and SVL Peptides)

This example shows that the binding between *E. coli* recombinant Nhead and streptavidin is separated by AVL, ATL and SVL peptides.

### (Methods)

*E. coli* recombinant Nhead (fragment of AtaA polypeptide) was prepared according to the method described in (Manufacture Examples). An equal amount of 0.2 mg/ml of recombinant protein of Nhead (SEQ ID NO: 5) and 0.2 mg/ml of streptavidin were mixed and allowed to stand for 5 minutes. 40 µL of the above mixture was placed in a quartz cell, and the grain shape was measured with a DLS measuring device equipped with a He-Ne laser (wavelength 633 nm) (Zetasizer Nano ZSP, Malvern Instruments, UK), thus confirming that the recombinant protein of Nhead and streptavidin were bound to form a complex. Subsequently, AVL peptide was added to the mixture so as to reach 0.2 µg/ml, followed by mixing and allowing to stand for 5 minutes, and the DLS measurement was performed again. The DLS measurement was performed under the conditions of a temperature of 25°C, an equilibrium time of 120 seconds, and a scattering angle of 173°.

### (Results and Discussion)

The results are shown in Figures **2A-2C****.** In the complex of Nhead and streptavidin, a diameter peak was observed at a particle size of about 1000 nm, whereas when AVL peptide (Figure **2A**), ATL peptide (Figure **2B**) and SVL peptide (Figure **2C**) were added to the complex of Nhead and streptavidin, a diameter peak was observed in the particle size of about 5 to 10 nm. From this result, it was considered that the binding between Nhead and streptavidin was separated by the addition of AVL peptide, ATL peptide and SVL peptide.

### (Example 3: Repeated adhesion/separation operations for biotinylated molecules)

This example shows the reversible immobilization of biotinylated molecules using the binding between Nhead and streptavidin.

### (Methods)

Three solutions were prepared by individually dissolving *E. coli* recombinant Nhead protein, streptavidin, and biotinylated fluorescent peptide (in which the N-terminal of the peptide GGSGGSK (SEQ ID NO: 6) was modified with fluorescein and the C-terminal was modified with biotin) in PBS buffer to a concentration of 1 µM.

First, 100 µl of PBS buffer was applied to a black 96-well polystyrene plate for fluorescence measurement. Subsequently, the operation (1) below was performed to measure the negative control value (blank fluorescence value due to streptavidin adsorbed on the plate) prior to the immobilizing of the Nhead protein. Next, 100 µl of Nhead protein solution was applied. The Nhead protein was adhered to the plate by allowing it to stand at 37°C for 1 hour. Thereafter, the following operations were repeated in the order of (1) → (2) → (1) → (2) → (1).
(1) Streptavidin binding operation
(1-1). The solution was removed, and 100 µl of streptavidin solution was applied.
(1-2). Streptavidin was caused to bind to the Nhead protein immobilized on the plate by allowing it to stand at 28°C for 30 minutes.
(1-3). The solution was removed and 100 µl of biotinylated fluorescent peptide was applied.
(1-4). The biotinylated fluorescent peptide was caused to bind to streptavidin by allowing it to stand at 28°C for 30 minutes.
(1-5). The above was washed 5 times with 200 µl PBS buffer.
(1-6). A 100 µl PBS buffer was applied and fluorescence measurement (excitation wavelength: 485 nm, fluorescence wavelength: 535 nm) was performed with a plate reader (ARVO X3 manufactured by PerkinElmer).
(1-7). The buffer was removed.
(2) Streptavidin separation operation
(2-1). The above was washed twice with 200 µl of 100 µM AVL peptide solution and once with 200 µl of PBS buffer (applied and removed as is).
(2-2). Residual fluorescence was determined by applying 100 µl of PBS buffer and re-measuring the fluorescence value with a plate reader.

### (Results and Discussion)

The results are shown in Figure **3****.** In the fluorescence measurement performed after the first binding reaction of the biotinylated fluorescent peptide, an increase in the fluorescence value was observed. This result demonstrated that the biotinylated fluorescent peptide was bound to the complex of Nhead and streptavidin adhered to the polystyrene plate. In the fluorescence measurement performed after the addition of the AVL peptide, a decrease in the fluorescence value was observed, indicating that the binding between Nhead and streptavidin was separated. Similar results were observed after the second binding reaction and separation reaction, and after the third binding reaction, indicating that the binding between Nhead and streptavidin was reversibly bound and separated.

### (Note)

As described above, the present disclosure is exemplified by the use of its preferred embodiments. However, the above descriptions and examples are provided for purposes of illustration only and not for the purpose of limiting the present disclosure. Accordingly, it is understood that the present invention is not limited to the embodiments or examples specifically described herein and the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2019-132490 filed on July 18, 2019 with the Japan Patent Office. It is understood that the entire content thereof is incorporated herein by reference.

### [Industrial Applicability]

The present disclosure has utility in industries that use bioprocesses.

### [Sequence Listing Free Text]

SEQ ID NO: 1: amino acid sequence of AtaA fragment including methionine at the starting position, excluding the signal peptide
SEQ ID NO: 2: nucleic acid sequence of AtaA fragment including methionine at the starting position, excluding the signal peptide
SEQ ID NO: 3: full-length nucleic acid sequence of AtaA
SEQ ID NO: 4: full-length amino acid sequence of AtaA
SEQ ID NO: 5: amino acid sequence of recombinant protein in Nhead domain of AtaA
SEQ ID NO: 6: amino acid sequence of artificial peptide

## Claims

1. A peptide comprising an amino acid sequence of AVL, SVL or ATL, or a functional equivalent sequence thereof.

2. A peptide consisting of an amino acid sequence of AVL, SVL or ATL, or a functional equivalent sequence thereof.

3. The peptide according to claim 1, which has a function of disintegrating the self-aggregation of a polypeptide having the amino acid sequence set forth in SEQ ID NO: 4 or an altered sequence and fragment thereof having a self-aggregation property.

4. The peptide according to claim 1 or 2, which has a function of disintegrating the aggregation of cells expressing a polypeptide comprising an amino acid sequence set forth in SEQ ID NO: 4 or an altered sequence thereof or a fragment thereof.

5. The peptide according to claim 1, which has a function of separating a bond between a polypeptide comprising an amino acid sequence set forth in SEQ ID NO: 1 or an altered sequence thereof or a fragment thereof, or a fusion or complex of the polypeptide with another amino acid sequence, and another subject.

6. A composition comprising the peptide according to any one of claims 1 to 5, for disintegrating self-aggregation of a polypeptide having the amino acid sequence set forth in SEQ ID NO: 4 or an altered sequence and fragment thereof having a self-aggregation property.

7. A composition comprising the peptide according to any one of claims 1 to 5 or a similar peptide, for separating a bond between a polypeptide comprising the amino acid sequence set forth in SEQ ID NO: 1 or an altered sequence thereof or a fragment thereof, or a fusion or complex of the polypeptide with another amino acid sequence, and another subject.

8. The composition according to claim 7, wherein the subject is a functionality-impairing entity.

9. The composition according to claim 7, wherein the subject is streptavidin, neutral avidin or a variant of either thereof.

10. A fusion or complex of the peptide according to any one of claims 1 to 5 and a functionality-impairing entity.

11. Use of the peptide according to any one of claims 1 to 5 for disintegrating the self-aggregation of a polypeptide having the amino acid sequence set forth in SEQ ID NO: 4 or an altered sequence and fragment thereof having a self-aggregation property.

12. Use of the peptide according to any one of claims 1 to 5 or a similar peptide for separating a bond between a polypeptide comprising the amino acid sequence set forth in SEQ ID NO: 1 or an altered sequence thereof or a fragment thereof, or a fusion or complex of the polypeptide with another amino acid sequence, and another subject.

13. The use according to claim 12, wherein the subject is a functionality-impairing entity.

14. The use according to claim 12, wherein the subject is streptavidin, neutral avidin or a variant of either thereof.
